# EUROPEAN PATENT APPLICATION

(11) **EP 4 438 597 A1**
(43) Date of publication of application: **02.10.2024**
(21) Application number: 23164460.0
(22) Date of filing: 27.03.2023
(51) Int. Cl.: C07D 277/64, A61P 25/28, A61K 31/454

(54) **2-STYRYLBENZOTHIAZOLE DERIVATIVES AS A-SYNUCLEIN BINDING COMPOUNDS**

(71) Applicant: Eberhard Karls Universität Tübingen Medizinische Fakultät, 72074 Tübingen (DE)
(72) Inventor: Di Nanni, Adriana, 72070 Tübingen (DE); Sing, Saw Ran, 72074 Tübingen (DE); Maurer, Andreas, 72074 Tübingen (DE); Battisti, Umberto Maria, 2100 Copenhagen (DK); Bowden, Gregory David, 72074 Tübingen (DE); Herfert, Kristina, 72074 Tübengen (DE); Herth, Matthias Manfred, 21121 Malmö (SE); Pichler, Bernd, 85298 Fernhag (DE); Boeckermann, Adam, 72076 Tübingen (DE)
(74) Representative: Witte, Weller & Partner Patentanwälte mbB

(57) **Abstract**

The present invention relates to α-synuclein binding compounds, a method for diagnosis, use of the α-synuclein binding compound in such diagnosis, as well as a diagnostic composition comprising such α-synuclein binding compound.

## Description

The present invention relates to α-synuclein binding compounds, a method for diagnosis, use of the α-synuclein binding compound in such diagnosis, as well as a diagnostic composition comprising such α-synuclein binding compound.

### FIELD OF THE INVENTION

The present invention relates to the field of visualization of spatial distribution of internal structures of a mammal's body, especially to the development of small molecules for visualization of brain structures for diagnostic and research purposes, more specifically to compounds for diagnosis and research on synucleinopathies.

### BACKGROUND

Synucleinopathies are a diverse group of neurodegenerative diseases that are characterized by the abnormal accumulation of aggregates of alpha-synuclein (α-synuclein or αSYN) protein in neurons, nerve fibers or glial cells. Such aggregates form intracellular αSYN fibrils. Prominent examples of synucleinopathies are Parkinson's disease (PD), multiple system atrophy (MSA) and dementia with Lewy bodies (DLB). The pathogenesis of synucleinopathies is still not fully understood and a diagnosis is challenging due to the diverse set of symptoms caused by the different synucleinopathies and other neurodegenerative diseases, as for example Alzheimer's disease (AD), which is characterized by β-amyloid (Aβ) accumulation, or tauopathies, characterized by accumulation of highly phosphorylated Tau protein.

An important premise for treatment as well as research on synucleinopathies is the ability to diagnose such diseases as early as possible or differently speaking, reliable techniques for in vivo detection of αSYN for clinical purposes as well as in vitro or ex vivo detection for research purposes.

Detection of αSYN is challenging since Aβ fibrils or Tau fibrils are structurally similar to αSYN fibrils, making a discrimination between them difficult. Furthermore, αSYN shows an at least 10-times lower abundance than Aβ in human brain. Thus, the detection has to be highly sensitive and highly selective for αSYN. Another challenge is the intracellular location of the αSYN accumulation, which makes αSYN a difficult target to be accessed in vivo by drugs, detecting agents or αSYN binding compounds, that may bind to or otherwise interact with αSYN. Apart from this, a drug or detecting agent needs to cross the blood-brain-barrier (BBB), since the αSYN fibrils are located in the brain.

Such drugs or detecting agents are currently not available.

Kaide, S. et al., Synthesis and Evaluation of (18)F-Labeled Chalcone Analogue for Detection of alpha-Synuclein Aggregates in the Brain Using the Mouse Model; ACS Chem Neurosci 2022, 13 (20), 2982-2990, discloses 4-nitrophenyl chalcone-derivatives as αSYN binding compounds. However, clearance from brain was unsatisfactory. Furthermore, the options for structural optimization, as reducing lipophilicity, to improve pharmacokinetic characteristics of those 4-nitrophenyl chalcone-derivatives is very limited.

Gaur, P. et al., Fluorescent Probe for Selective Imaging of α-Synuclein Fibrils in Living Cells; ACS Chemical Neuroscience 2021, 12 (8), 1293-1298, discloses a 2-styrylbenzothiazol derivative, called RB1, as a αSYN binding compound. RB1 is structurally derived from thioflavin T, which is known to bind to Aβ fibrils and is used for its detection. RB1 shows a limited degree of selectivity towards αSYN over Aβ as compared to thioflavin T, which does not bind to αSYN. It is assumed that RB1 cannot provide good signals in the brain because of its structural properties, which do not allow its accumulation in the brain.

At the International Symposium on Radiopharmaceutical Sciences (iSRS) in Nantes, France, from May 2022, derivatives of the RB1 structure were disclosed. However, the disclosed structures are incomplete, not fully defined and possible applicability of those compounds in diagnosis of synucleinopathies or even binding αSYN is not shown. The applicant found that those compounds do not accumulate in the brain of mammals.

It is an object of the invention to overcome the disadvantages and drawbacks of the prior art. Especially, it is an object of the present invention to provide for reliable αSYN binding compounds for in vivo, in vitro and ex vivo detection of αSYN for diagnostic and research purposes.

This object is fully solved by the present invention.

### SUMMARY OF THE INVENTION

In an aspect of the invention, the above-identified disadvantages are overcome by providing an α-synuclein binding compound having the structure 1, 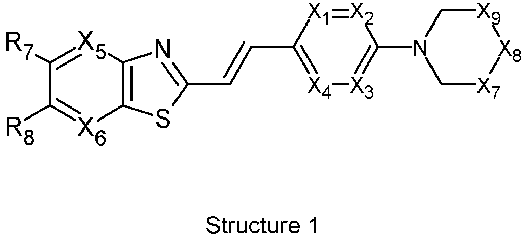
- wherein X₁-X₆ are independently selected from the group consisting of: C-Rₓ, N, wherein the x in each Rₓ is equal to the index of the X to which the Rₓ is bound, with the provisor that within the group consisting of: X₁, X₂ and within the group consisting of: X₃, X₄, no more than one X is N; and
- wherein R₇, R₈ and Rₓ are independently selected from the group consisting of: H, F, Cl, Br, OH, C₁-C₃ alkyl ether-group, fluorinated C₁-C₃ alkyl-group, fluorinated C₁-C₃ alkyl ether-group and are preferably selected from the group consisting of: H, O-CH₃, F; and
- wherein X₇-X₉ are independently selected from the group consisting of: CH₂, O, NH, S and are preferably selected from the group consisting of: CH₂, O, with the provisor that no heteroatom is directly bound to a heteroatom.

Hereinafter, any R or X, as well as any R or X comprising any index as for example R₁, Rₓ, or X₁, is to be understood as a placeholder, which by itself does not refer to any atom or atom-group. Within the accompanying text, such placeholders are defined regarding possible atoms or atom-groups that can be represented by them.

When referring to αSYN, αSYN binding, or affinity towards αSYN, this is to be understood as to include αSYN fibrils, binding to αSYN fibrils, or affinity towards αSYN fibrils, respectively, wherein the term "αSYN fibrils" can refer to αSYN fibrils or other types of unphysiological αSYN accumulations that may form in a mammal's body.

Surprisingly, the affinity towards αSYN and more importantly the selectivity of compounds having a structure represented by structure 1 towards αSYN over Aβ exceeds compounds of prior art. The affinity and selectivity of compounds having a structure represented by structure 1 has turned out to be strongly triggered by structural modifications at the placeholder positions of structure 1.

Selecting R₅ (bound to Xs), R₆ (bound to X₆), R₇ and R₈ independently from the group consisting of: H, F, Cl, Br, OH, C₁-C₃ alkyl ether-group, fluorinated C₁-C₃ alkyl-group, and fluorinated C₁-C₃ alkyl ether-group advantageously does improve especially the affinity of the compound towards αSYN. However, also other features of the compound are positively influenced by such selection.

A C₁-C₃ alkyl ether-group is preferably a methyl ether-group (O-CH₃). A fluorinated C₁-C₃ alkyl-group is preferably a 2-fluoro ethyl-group (CH₂-CH₂-F). A fluorinated C₁-C₃ alkyl ether-group is preferably a 2-fluoro ethyl ether-group (O-CH₂-CH₂-F).

Selecting X₇, X₈ and X₉ independently from the group consisting of: CH₂, O, NH, and S, advantageously does improve especially the pharmacokinetics by reducing the lipophilicity of the compound. However, also other features of the compound are positively influenced by such selection. Avoiding two heteroatoms in direct vicinity ensures that the reactivity of the compound remains limited to a suitable level.

Selecting X₁ to X₆ independently from the group consisting of: C-Rₓ, N, wherein the x in each Rₓ is equal to the index of the X to which the Rₓ is bound, allows the following advantages: By selecting an X to be N, further reduction of the compound's lipophilicity for further improvement of pharmacokinetics can be achieved. By selecting an X to be C-Rₓ, especially the affinity of the compound towards αSYN, but also other features of the compound can be improved. However, also other features of the compound are positively influenced by such selection. Again, avoiding two heteroatoms in direct vicinity ensures that the reactivity of the compound remains limited to a suitable level.

A compound of structure 1 advantageously can pass the BBB easily. Without being bound to this theory, it is assumed that this is caused at least partially by the absence of a positively charged quaternary nitrogen atom (N) at position 3 of the benzothiazole cycle. This is an advantage over benzothiazole derivatives known from prior art [e.g., Gaur et al. (op. cit.)], which do contain a quaternary nitrogen, and thus a permanent positive charge, which could partially cause a deficiency in passing the BBB for brain-uptake. In contrast, ability to pass the BBB advantageously allows the compound according to the invention to enter the brain, and thus a tissue that is known to contain αSYN, and a tissue the accumulation of αSYN is known to cause diseases.

A compound of structure 1 displays a high affinity for binding to αSYN fibrils, when compared to compounds provided by the prior art. This advantageously allows a reduction of the amount of the compound for targeting αSYN. Hereinafter, "targeting αSYN" is to be understood as bringing the αSYN binding compound into contact with or administering the αSYN binding compound to a substance, material, living being, or animal suspected of containing αSYN, wherein the substance, material, living being, or animal is preferably selected from the group consisting of: a mammal, a tissue derived from a mammal, a sample derived from a mammal, a sample derived from a tissue of a mammal, and an artificially generated sample. Preferably the mammal is a human, more preferably a human suspected of being affected by a synucleinopathy.

Additionally, a compound of structure 1 displays a high selectivity towards αSYN over Aβ. This advantageously allows to specifically target αSYN in brain tissue, where the abundance of Aβ is much higher than the one of αSYN, by binding of the αSYN binding compound to αSYN and not to Aβ. This allows to discriminate between αSYN and Aβ as being bound or not bound to the compound.

Further advantageously, a compound of structure 1 is highly suitable for reduction of lipophilicity for further improvement of pharmacokinetics, since multiple sites of the structure allow for the introduction of hydrophilic structures or substituents, grossly without strongly affecting the αSYN affinity and selectivity.

In another embodiment, X₁ to X₆ are C-Rₓ.

Selecting X₁ to X₆ are to be not N, but C-Rₓ advantageously can favour affinity or selectivity of the compound towards αSYN over an attempt to reduce lipophilicity.

In another aspect of this embodiment R₁ to R₄ are H.

Selecting R₁ to R₄ to be H, advantageously restricts the position of -I-Effect introducing substituents at the aromatic system selectable for any R, as F, Cl, Br, OH, a C₁-C₃ alkyl ether-group, and fluorinated C₁-C₃ alkyl ether-group, to the benzothiazole heterocycle, which has turned out to improve especially the affinity and selectivity of the compound towards αSYN. Also, the position of a fluorine comprising substituent generally, as for example a fluorinated C₁-C₃ alkyl-group, a fluorinated C₁-C₃ alkyl ether-group or F, is restricted this way to the benzothiazole heterocycle, which is a position proven to be advantageous for affinity of the αSYN binding compound towards αSYN and its selectivity towards αSYN over Aβ.

In another aspect of this embodiment, R₅, R₆ and R₇ are H.

Selecting R₅, R₆ and R₇ to be H, advantageously restricts the position of -I-Effect introducing substituents selectable for any R, as F, Cl, Br, OH, a C₁-C₃ alkyl ether-group, fluorinated C₁-C₃ alkyl-group, and fluorinated C₁-C₃ alkyl ether-group, to the position of R₈ at the benzothiazole heterocycle, which has turned out to be the most favourable position, especially regarding the affinity and selectivity of the compound towards αSYN.

In another aspect of this embodiment, R₈ is F.

Among the selectable -I-Effect introducing substituents of any R, F has turned out to be the substituent providing the highest affinity and selectivity of the compound towards αSYN.

In another embodiment, X₇ to X₉ are selected with the provisor that no more than one of X₇ to X₉ is not CH₂.

Advantageously, selecting only one of X₇ to X₉ to be CH₂, O, NH or S, can favour affinity or selectivity of the compound towards αSYN over reducing lipophilicity.

In another embodiment, the αSYN binding compound has the structure 2 or the structure 3.

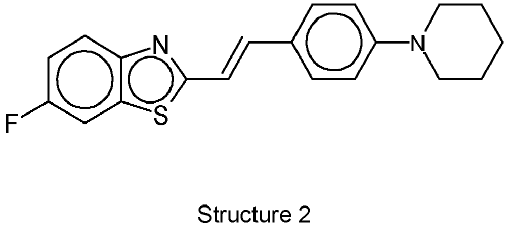

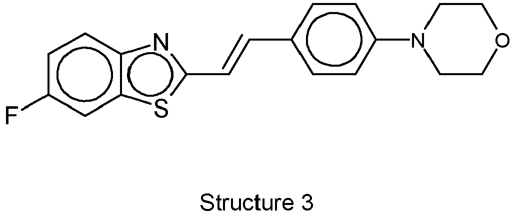

An αSYN binding compound having the structure 2 or the structure 3 advantageously showed the highest affinity and selectivity towards αSYN. Furthermore, such αSYN binding compounds display favorable pharmacokinetics in that, after intravenous injection into a mammal's circulation, they are distributed to the site of action, which is primarily the brain of the mammal, and subsequently, those αSYN binding compounds are metabolized, thus cleared, which reduces a burden for the respective mammal.

In one aspect of this embodiment, the compound is fluorescent.

A fluorescence of the αSYN binding compound advantageously allows a detection of the compound by detecting the fluorescence, for example when targeting αSYN.

"Detection" is to be understood as any type of qualitative or quantitative proof of presence of the αSYN binding compound, wherein the presence of the αSYN binding compound can be for example visualized or localized in a relative or absolute way.

Detection of the compound's fluorescence is advantageously facilitating especially in vitro or ex vivo detection, visualization, or localization of αSYN in a mammal derived tissue, any sample generated from mammal derived tissue or artificially generated samples suspected to contain αSYN, since in such cases the emitted fluorescence is less covered, shielded or absorbed by tissue surrounding the site of binding between αSYN binding compound and αSYN. Furthermore, fluorescence detection is a common technique in science and medicine. The fluorescence does not only facilitate the detection itself but also makes a conjugation of the αSYN binding compound with another fluorescent compound not necessary. Such conjugation, which is a common method to fluorescently mark compounds or antibodies for example, might disadvantageously interfere with the affinity and/or selectivity of the αSYN binding compound towards αSYN.

In another aspect of this embodiment, at least one atom of the compound is a radionuclide.

Presence of a radionuclide as an atom of the αSYN binding compound advantageously allows detecting the radiation, for example when targeting αSYN.

Detection of the compound's radiation is advantageously facilitating especially in vivo or ex vivo detection, visualization or localization of αSYN in a mammal derived tissue, any sample generated from mammal derived tissue or artificially generated samples suspected to contain αSYN, wherein the site of binding between αSYN binding compound and αSYN is covered by surrounding tissue or any other material shielding or absorbing fluorescence. Furthermore, the use of a radionuclide has the advantage that due to suitable selection of the radionuclide, both the radiation duration (half-life), range, strength and type can be adjusted and adapted to the desired purposes. The radioactivity is able to overcome covering, shielding or absorption by the tissue or other material surrounding the site of binding between αSYN binding compound and αSYN. Furthermore, radiation detection is a common technique in science and medicine.

In this embodiment, the radionuclide containing αSYN binding compound is preferably used as a so called "tracer" in positron-emission-tomography (PET) or single photon emission computed tomography (SPECT). A tracer is a molecule being incorporated into a target cell or target structure in a mammal's body or binding to a cell or target structure in a mammal's body, wherein the molecule can be "traced" or detected by a method, which is advantageously a non-invasive method as positron emission tomography or single photon emission computed tomography.

In another aspect of this embodiment, at least one C-atom is a ¹¹C radionuclide. ¹¹C is a positron emitting radionuclide and thus advantageously allows application of the αSYN binding compound in PET. Moreover, ¹¹C due to its short half-live of about 20 min is a short-lived radionuclide which advantageously minimizes the exposition of the living being to radiation. Furthermore, ¹¹C is widely used for PET imaging with small molecules so that empirical values already exist with this compound and the laboratories and clinics have established suitable workflows. Apart from this, any αSYN binding compound disclosed herein contains at least one C atom and thus, this embodiment can be combined with any of those.

In another aspect of this embodiment, at least one F atom is a ¹⁸F radionuclide. ¹⁸F is a positron emitting radionuclide and thus advantageously allows application of the αSYN binding compound in PET. Moreover, ¹⁸F due to its short half-live of about 110 min is a short-lived radionuclide which advantageously minimizes the exposition of the living being to radiation. Furthermore, ¹⁸F is widely used for PET imaging with small molecules so that empirical values already exist with this compound and the laboratories and clinics have established suitable workflows. The use of ¹⁸F can be preferred over the use of ¹¹C when a transportation or otherwise prolonged handling of the αSYN binding compound is necessary, since the half-live of ¹⁸F is longer than the one of ¹¹C.

¹⁸F surprisingly also lead to an enhanced affinity of the αSYN compound towards αSYN, especially with respect to the αSYN compound of structure 2 and 3.

In another aspect, the invention relates to the αSYN binding compound for use in diagnosis of a disease, wherein the disease is preferably a neurodegenerative disease, more preferably a neurodegenerative disease associated with unphysiological α-synuclein presence in a tissue of a mammal.

Herein, the term "disease" is to be understood as generally in medicine as any kind of unphysiological condition of the respective living being, such as a mammal, which may be caused for example genetically, oncologically, microbially, bacterially, virally, or psychologically. The term disease may also refer to any kind of unphysiological condition, which may be caused for example by physical damage of the living being's body, or aging.

Advantageously, the αSYN binding compound for use in diagnosis of a disease allows to diagnose any disease as not being a disease associated with unphysiological α-synuclein presence in a tissue of a mammal. This is especially advantageous with regard to neurogenerative diseases not associated with unphysiological α-synuclein presence in a tissue of a mammal.

The αSYN binding compound has a high affinity towards αSYN and a high selectivity for αSYN over Aβ. Thus, use of the αSYN binding compound in diagnosis of a neurodegenerative disease advantageously allows to discriminate between different types of neurogenerative diseases, which are otherwise often difficult to discriminate due to overlapping pathogenesis and/or symptoms, by proving or disproving that an accumulation of αSYN is present in the brain of a mammal. This advantageously can lead to an early diagnosis of a neurogenerative disease and its type, thus allowing an earlier onset of and a correct or precisely adjusted therapy or treatment.

In this embodiment, use of the αSYN binding compound in diagnosis of a neurodegenerative disease associated with unphysiological α-synuclein presence in a tissue of a living being, such as a mammal, can allow a reliable positive diagnosis of this type of neurogenerative diseases by reducing the probability of false negative diagnosis. To date, no other compound displaying a reliable selectivity for αSYN over Aβ is available, which can lead for example to false negative diagnosis of a neurogenerative disease associated with enhanced αSYN accumulation as being associated with enhanced Aβ accumulation.

An unphysiological αSYN presence in a tissue of a living being, such as a mammal, can be any enhanced or reduced concentration in, spatial distribution in, or presence at any site of the tissue, which can cause an acute or chronic disease or may be expected to cause in future any type of disease.

In embodiments, in which an atom of the αSYN binding compound is a radionuclide, the high affinity of the αSYN binding compound towards αSYN allows to reduce the amount of the αSYN binding compound when targeting αSYN. This advantageously reduces the exposition of a mammal, when using the αSYN binding compound in diagnosis of a disease by targeting αSYN in a living being, such as a mammal, or a user of the αSYN binding compound, when using the αSYN binding compound in diagnosis of a disease by targeting αSYN, to potentially disease-inducing or otherwise damaging radiation.

In another embodiment, the αSYN binding compound can be used in the diagnosis of a synucleinopathy in a living being, wherein the synucleinopathy is preferably selected from the group consisting of: Parkinson's disease, multiple system atrophy, and dementia with Lewy bodies.

Synucleinopathies are neurodegenerative diseases known to be associated with unphysiological α-synuclein presence in a tissue of a living being, such as a mammal. Thus, the aforementioned advantages in use of the compound in diagnosis of a neurodegenerative disease associated with unphysiological α-synuclein presence in a tissue of a mammal apply to synucleinopathies.

In another aspect, the present invention relates to a diagnostic composition containing the α-synuclein binding compound according to the invention and a pharmaceutically acceptable carrier.

A suitable carrier for the specific diagnosis, will be clear to the skilled person. Additional disclosure concerning pharmaceutically acceptable carriers can be found for example in Trucillo, P. Drug Carriers: Classification, Administration, Release Profiles, and Industrial Approach. Processes 2021, 9, 470, which is incorporated herein in its entirety by reference.

The diagnostic composition can be administered to any material or substance, preferably to a material or substance, regarding which targeting αSYN and detecting the αSYN binding compound can yield information based on which a diagnosis of a disease can be given, for example by a physician.

In one embodiment, the diagnostic composition is configured for administration to a living being, wherein the living being is preferably a mammal.

An administration of the diagnostic composition to a living being advantageously allows early diagnosis as well as tracking of progression of a disease and thus advantageously allows an early or prophylactic therapy or treatment in case of a positive diagnosis of a disease. Also, an adjustment of the therapy according to the progression of the disease is possible.

In another aspect, the invention relates to a method for diagnosis of a disease of a living being, wherein the disease is preferably a neurodegenerative disease, more preferably a neurodegenerative disease associated with unphysiological α-synuclein presence in a tissue of a mammal, comprising the targeting of αSYN and the detection of the αSYN binding compound.

The targeting can comprise a systemic administration or local administration of the αSYN binding compound or the diagnostic compound comprising the αSYN binding compound to a living being, such as a mammal, preferably a systemic administration by intravenous injection. The living being is preferably a mammal. The mammal is preferably a human, more preferably a human suspected of being affected by a synucleinopathy.

It is understood that the features mentioned above and those yet to be explained below can be used not only in the particular combination given, but also in other combinations or in isolation, without departing from the scope of the present invention.

The invention is now described and explained in further detail by referring to the following non-limiting examples and drawings.

### BRIEF DESCRIPTION OF THE FIGURES

Fig. 1: Development of a library of 2-styrylbenzothiazoles and structure of RB1 and RB2 fluorescent probes.
Fig. 2: General synthetic pathway for N-methylated and non-methylated 2-styrylbenzothiazoles: a) DMSO, NaOH aq. 18M, r.t., 2h to 24h; b) MeCN, Mel or MeNs, 80°C, overnight; c) EtOH, 80°C, overnight.
Fig. 3: Synthetic pathway for analogs with altered π-system lenght: a) Pd(PPh₃)₄, Cs₂CO₃, piperidine, toluene, 110°C, 5h; b) MeNs, chlorobenzene, 80°C, overnight; c) H₂SO₄ conc., acetaldehyde, 0°C, 1h; d) DMSO, NaOH aq. 18M, r.t., 3h; e) EtOH, 80°C, overnight.
Fig. 4: a) Structure of compounds combining promising features and their αSYN binding affinity (mean Kᵢ ± SEM, three data points) determined by [³H]PiB competition assays; b) competition binding assays on Aβ fibrils for compounds 14a, 17a, 43, 44.
Fig. 5: 2D ¹H NOESY NMR spectra of a) PFSB (9a) and b) 9b. In the spectrum (a), a weak interaction between H₈ and H_{2b}H_{6b} shows the sample is a mixture of (E)-9a and (Z)-9a. Such interaction is not detected in the spectrum (b), highlighting (Z)-9b as the preferential configuration for the *N*-methylated compound.
Fig. 6: a) Development of the less lipophilic analog MFSB; b) binding affinity of PFSB, MFSB and 15a to αSYN and Aβ fibrils determined by [³H]PiB competition assays (three repetitions each, ^{a} = two repetitions), c) competition of PFSB and MFSB with [³H]MODAG-001 for αSYN binding.
Fig. 7: General synthetic pathway for the synthesis of BPin precursors and fluorine-18 labeling: a) B₂Pin₂, KOAc, Pd(dppf)Cl₂, DMF, 100°C, 45 min; b) Cu(Otf)₂, pyridine, n-BuOH, [¹⁸F]TBAF, DMA, 120°C, 20 min.
Fig. 8: Example of analytical QC HPLC of [¹⁸F]PFSB and its non-radioactive reference compound using a Luna column 5 µm C18 (2) 100 Å 250 x 4.6 mm, isocratic method: 88% MeCN in 25 mM ammonium formate at pH 8, 1 mL/min. The ratio between the two peaks (retention time ≈ 10.1 and 10.5 min) is dependent on the E/Z isomer ratio.
Fig. 9: Example of radioTLC of [¹⁸F]PFSB, eluent: PE/EtOAc 2:1.
Fig. 10: Example of analytical QC HPLC of [¹⁸F]MFSB and its non-radioactive reference compound using a Luna column 5 µm C18 (2) 100 Å 250 x 4.6 mm, isocratic method: 72% MeCN in 25 mM ammonium formate at pH 8, 1 mL/min. The ratio between the two peaks (retention time ≈ 9.1 and 9.7 min) is dependent on the E/Z isomer ratio.
Fig. 11: Example of radioTLC of [¹⁸F]MFSB, eluent: PE/EtOAc 1:1.
Fig. 12: In vitro autoradiography of [¹⁸F]PFSB and [¹⁸F]MFSB on human brain slices and correspondent IHC with αSYN-staining (MSA, PD and Ctrl tissues) and Aβ-staining (AD tissue). The scale bar represents a 0.5 cm size.
Fig. 13: In vivo evaluation of [¹⁸F]MFSB pharmacokinetic profile: a) whole-body PET/MR sagittal images at different time points ; b) time-activity curves in brain regions; c) time-activity curves of whole brain, kidney, lung and liver.

### EXAMPLES

### 1. Materials and methods

### 1.1 Synthesis of compounds

All chemicals were purchased from Sigma Aldrich (St. Louis, Missouri, USA), abcr GmbH (Karlsruhe, Germany), or Carl Roth (Karlsruhe, Germany) and used without any further purification.

Reaction progress was monitored by thin-layer chromatography (TLC) on 0.20 mm Polygram SIL G/UV254 (silica gel 60) TLC plates (Macherey-Nagel, Düren, Germany) with the chosen eluent mixture and/or analytical HPLC-MS (ESI detector, Agilent, Santa Clara, California, USA) equipped with a Luna 5 µm C18 (2) 100 Å 50 x 2 mm column (Phenomenex, Torrance, California, USA) [gradient: 0 - 7.60 min (0% to 100% B), 7.60 - 8.80 (100% B), 8.80 - 9.30 min (100% to 0% B), 9.30 - 13.0 min (0% B); solvent A: 0.1% formic acid in H₂O; solvent B: acetonitrile; 0.4 mL/min] or with a Zorbax Eclipse XBD-C18 50 x 4.6 mm column (Agilent, Santa Clara, California, USA) [gradient: 0-6 min (0% to 100% B); solvent A: H₂O:MeCN:formic acid 95:5:0.1 v/v%; solvent B: 0.1% formic acid in MeCN; 1 mL/min].

Purification was performed through automated flash chromatography on an Isolera 4 system (Biotage, Uppsala, Sweden) or a CombiFlash NextGen 300+ (Teledyne ISCO, Lincoln, NE, USA).

¹H, ¹³C and ¹⁹F NMR spectra were acquired on an Avance III AV 600 (1H: 600.13 MHz; 13C: 150.61 MHz) or an Avance II AV 400 (1H: 400 MHz; 13C: 101 MHz; 19F: 376 MHz) spectrometer (Bruker, Billerica, Massachusetts, USA). All chemical shift (δ) are reported as parts per million (ppm) and referenced to residual solvent peaks (CDCl₃: δ_{H} = 7.26, δ_{C} = 77.16; DMSO-*d₆*: δ_{H} = 2.50, δ_{C} = 39.52).

### 1.1.1. General procedure A

To a solution of the selected 2-methylbenzothiazole (6.70 mmol) and the selected 4-aminobenzaldehyde or 4-aminocinnamaldehyde (7.37 mmol) in DMSO (6.50 mL), NaOH aq. 18M (6.50 mL) was slowly added. The mixture was stirred at room temperature for 2h to 24h. A yellow precipitate was formed. The mixture was diluted with water and complete precipitation was allowed. The precipitate was filtered under vacuum to afford a yellow solid which was recrystallized from EtOAc.

### 1.1.2. General procedure B

According to the procedure disclosed in Gaur, P. et al., Fluorescent Probe for Selective Imaging of α-Synuclein Fibrils in Living Cells; ACS Chemical Neuroscience 2021, 12 (8), 1293-1298, a solution of the selected 2,3-dimethylbenzothiazolium salt (0.86 mmol) and the selected 4-aminobenzaldehyde or 4-aminocinnamaldehyde (1.83 mmol) in EtOH (7.00 mL) was refluxed overnight. Color turned to red/purple. The precipitate was filtered under vacuum and washed with EtOAc to afford a dark solid which was recrystallized from EtOH and/or Et₂O.

### 1.1.3. Individual compounds

### 4-methyl-2-(4-(piperidin-1-yl)styryl)benzo[d]thiazole (1a)

The synthesis was carried out according to general procedure A (46 mg, 60%). R*_{f}*: 0.48 (Hept/EtOAc 3:1). ¹H NMR (600 MHz, CDCl₃) δ 7.69 - 7.63 (m, 1H), 7.47 (d, J = 8.8 Hz, 2H), 7.39 (d, J = 16.1 Hz, 1H), 7.30 (d, J = 16.1 Hz, 1H), 7.25 - 7.20 (m, 2H), 6.91 (d, J = 8.7 Hz, 2H), 3.28 (t, J = 5.6 Hz, 4H), 2.75 (s, 3H), 1.70 (p, J = 5.6 Hz, 4H), 1.66 - 1.60 (m, 2H). ¹³C NMR (151 MHz, CDCl₃) δ 167.1, 153.5, 152.6, 137.9, 134.1, 132.7, 128.8, 126.8, 125.6, 125.0, 119.0, 118.9, 115.5, 49.6, 25.7, 24.5, 18.7.

### 3,4-dimethyl-2-(4-(piperidin-1-yl)styryl)benzo[d]thiazol-3-ium 4-nitrobenzenesulfonate (1b)

The synthesis was carried out according to general procedure B (119 mg, 59%). R*_{f}*: 0.19 (DCM/MeOH 5%). ¹H NMR (400 MHz, DMSO-*d₆*) δ 8.19 (d, J = 8.3 Hz, 2H), 8.12 (br s, 1H), 8.01 (d, J = 15.3 Hz, 1H), 7.89 (d, J = 8.5 Hz, 2H), 7.83 (d, J = 8.2 Hz, 2H), 7.66 (d, J = 15.3 Hz, 1H), 7.54 (s, 2H), 7.04 (d, J = 8.7 Hz, 2H), 4.43 (s, 3H), 3.49 (s, 4H), 2.91 (s, 3H), 1.61 (s, 6H). ¹³C NMR (101 MHz, DMSO-*d₆*) δ 171.5, 154.4, 153.5, 149.6, 147.2, 140.9, 132.8, 132.7, 127.7, 127.7, 127.3, 126.9, 123.3, 122.3, 121.7, 113.5, 107.0, 47.6, 40.0, 25.0, 23.9, 20.9.

### 4-methoxy-2-(4-(piperidin-1-yl)styryl)benzo[d]thiazole (2a)

The synthesis was carried out according to general procedure A (10 mg, 5%). R*_{f}*: 0.18 (Hept/EtOAc 3:1). ¹H NMR (600 MHz, CDCl₃) δ 7.45 (dt, J = 8.7, 2.9 Hz, 2H), 7.41 (dd, J = 8.0, 0.9 Hz, 1H), 7.38 (d, J = 16.1 Hz, 1H), 7.31 (d, J = 16.1 Hz, 1H), 7.28 (t, J = 8.0 Hz, 1H), 6.91 (dd, J = 8.8, 2.7 Hz, 2H), 6.88 (dd, J = 8.0, 0.9 Hz, 1H), 4.05 (s, 3H), 3.27 (t, J = 5.6 Hz, 4H), 1.70 (p, J = 5.6 Hz, 4H), 1.65 - 1.60 (m, 2H). ¹³C NMR (151 MHz, CDCl₃) δ 166.9, 153.3, 152.6, 144.2, 137.7, 135.8, 128.8, 126.0, 125.6, 119.0, 115.5, 113.6, 106.7, 56.0, 49.6, 25.7, 24.5.

### 4-methoxy-3-methyl-2-(4-(piperidin-1-yl)styryl)benzo[d]thiazol-3-ium iodide (2b)

The synthesis was carried out according to general procedure B (46 mg, quant.). R*_{f}*: 0.24 (DCM/MeOH 5%). ¹H NMR (600 MHz, DMSO-*d₆*) δ 7.97 (d, J = 15.3 Hz, 1H), 7.87 (d, J = 8.6 Hz, 2H), 7.83 (d, J = 8.1 Hz, 1H), 7.66 - 7.52 (m, 2H), 7.37 (d, J = 8.2 Hz, 1H), 7.04 (d, J = 8.6 Hz, 2H), 4.43 (s, 3H), 4.04 (s, 3H), 3.49 (t, J = 5.3 Hz, 4H), 1.77 - 1.51 (m, 6H). ¹³C NMR (151 MHz, DMSO-*d₆*) δ 170.7, 153.5, 149.7, 149.3, 132.7, 131.1, 128.7, 128.6, 122.3, 115.5, 113.5, 111.7, 106.9, 56.9, 47.5, 39.3, 25.1, 23.9.

### 4-fluoro-2-(4-(piperidin-1-yl)styryl)benzo[d]thiazole (3a)

The synthesis was carried out according to general procedure A (187 mg, 56%). R*_{f}*: 0.43 (Hept/EtOAc 3:1). ¹H NMR (400 MHz, CDCl₃) δ 7.58 (dd, J = 8.0, 1.0 Hz, 1H), 7.51 - 7.40 (m, 3H), 7.30 - 7.24 (m, 2H), 7.14 (ddd, J = 10.5, 8.2, 1.0 Hz, 1H), 6.91 (dt, J = 8.8, 8.8, 2.7 Hz, 1H), 3.34 - 3.22 (m, 4H), 1.76 - 1.66 (m, 4H), 1.66 - 1.59 (m, 2H). ¹³C NMR (101 MHz, CDCl₃) δ 168.7, 155.6 (d, J = 255.6 Hz), 152.7, 143.0 (d, J = 13.4 Hz), 139.1, 136.9 (d, J = 3.6 Hz), 129.0, 125.6 (d, J = 7.0 Hz), 125.1, 118.1, 117.2 (d, J = 4.3 Hz), 115.3, 112.0 (d, J = 18.0 Hz), 49.4, 25.6, 24.5. ¹⁹F NMR (376 MHz, CDCl₃) δ - 122.8.

### 4-fluoro-3-methyl-2-(4-(piperidin-1-yl)styryl)benzo[d]thiazol-3-ium 4-nitrobenzenesulfonate (3b)

The synthesis was carried out according to general procedure B (165 mg, 76%). R*_{f}*: 0.12 (DCM/MeOH 5%). ¹H NMR (400 MHz, DMSO-*d₆*) δ 8.19 (d, J = 8.3 Hz, 2H), 8.14 - 8.09 (m, 1H), 8.06 (d, J = 15.3 Hz, 1H), 7.91 (d, J = 8.6 Hz, 2H), 7.83 (d, J = 8.4 Hz, 2H), 7.73 - 7.57 (m, 3H), 7.04 (d, J = 8.7 Hz, 2H), 4.33 (d, J = 2.3 Hz, 3H), 3.52 (t, J = 5.1 Hz, 4H), 1.79-1.49 (m, 6H). ¹³C NMR (101 MHz, DMSO-*d₆*) δ 172.1, 154.4, 153.7, 151.0, 150.5 (d, J = 252.0 Hz), 147.2, 133.3, 130.5 (d, J = 9.8 Hz), 129.3, 128.3 (d, J = 8.0 Hz), 126.9, 123.3, 122.2, 120.1 (d, J = 4.0 Hz), 115.9 (d, J = 20.2 Hz), 113.4, 106.0, 47.5, 38.3 (d, J = 11.1 Hz), 25.1, 23.9. ¹⁹F NMR (376 MHz, DMSO-*d₆*) δ -125.2.

### 5-methyl-2-(4-(piperidin-1-yl)styryl)benzo[d]thiazole (4a)

The synthesis was carried out according to general procedure A (100 mg, 43%). R*_{f}*: 0.44 (Hept/EtOAc 3:1). ¹H NMR (400 MHz, CDCl₃) δ 7.75 (s, 1H), 7.69 (d, J = 8.1 Hz, 1H), 7.46 (d, J = 8.6 Hz, 2H), 7.42 (d, J = 16.1 Hz, 1H), 7.21 (d, J = 16.1 Hz, 1H), 7.16 (dd, J = 8.1, 1.6 Hz, 1H), 6.91 (d, J = 8.6 Hz, 2H), 3.28 (t, J = 5.5 Hz, 4H), 2.49 (s, 3H), 1.70 (p, J = 5.5 Hz, 4H), 1.64 (d, J = 5.8 Hz, 4H). ¹³C NMR (101 MHz, CDCl₃) δ 168.3, 154.5, 152.6, 137.8, 136.3, 131.2, 128.9, 126.6, 125.5, 122.8, 121.0, 118.6, 115.5, 49.6, 25.7, 24.5, 21.6.

### 3,5-dimethyl-2-(4-(piperidin-1-yl)styryl)benzo[d]thiazol-3-ium iodide (4b)

The synthesis was carried out according to general procedure B (278 mg, 99%). R*_{f}*: 0.21 (DCM/MeOH 5%). ¹H NMR (400 MHz, DMSO-*d₆*) δ 8.17 (d, J = 8.3 Hz, 1H), 8.01 (d, J = 15.4 Hz, 1H), 7.96 (br s, 1H), 7.88 (d, J = 8.8 Hz, 2H), 7.63 (d, J = 15.4 Hz, 1H), 7.53 (dd, J = 8.3, 1.5 Hz, 1H), 7.04 (d, J = 8.8 Hz, 2H), 4.21 (s, 3H), 3.49 (t, J = 5.2 Hz, 4H), 2.54 (s, 3H), 1.71 - 1.50 (m, 6H). ¹³C NMR (101 MHz, DMSO-*d₆*) δ 171.3, 153.5, 149.2, 142.2, 139.3, 132.7, 128.8, 123.9, 123.3, 122.3, 115.9, 113.4, 107.0, 47.5, 35.6, 25.1, 23.9, 21.1.

### 5-methoxy-2-(4-(piperidin-1-yl)styryl)benzo[d]thiazole (5a)

The synthesis was carried out according to general procedure A (170 mg, 58%). R*_{f}*: 0.29 (Hept/EtOAc 3:1). ¹H NMR (400 MHz, CDCl₃) δ 7.67 (d, J = 8.7 Hz, 1H), 7.51 - 7.40 (m, 4H), 7.18 (d, J = 16.1 Hz, 1H), 6.98 (dd, J = 8.7, 2.5 Hz, 1H), 6.91 (dt, J = 8.8, 2.9 Hz, 2H), 3.89 (s, 3H), 3.35 - 3.21 (m, 4H), 1.77 - 1.65 (m, 4H), 1.62 (ddd, J = 7.7, 5.9, 3.4 Hz, 2H). ¹³C NMR (101 MHz, CDCl₃) δ 169.3, 159.2, 155.4, 152.6, 137.6, 128.9, 126.2, 125.5, 121.7, 118.3, 115.4, 115.0, 105.3, 55.7, 49.5, 25.7, 24.5.

### 5-methoxy-3-methyl-2-(4-(piperidin-1-yl)styryl)benzo[d]thiazol-3-ium iodide (5b)

The synthesis was carried out according to general procedure B (222 mg, 97%). R*_{f}*: 0.19 (DCM/MeOH 5%). ¹H NMR (400 MHz, DMSO-*d₆*) δ 8.18 (d, J = 8.9 Hz, 1H), 7.99 (d, J = 15.4 Hz, 1H), 7.87 (d, J = 8.8 Hz, 2H), 7.63 (d, J = 2.3 Hz, 1H), 7.62 (d, J = 15.4 Hz, 1H), 7.32 (dd, J = 8.9, 2.3 Hz, 1H), 7.04 (d, J = 8.8 Hz, 2H), 4.22 (s, 3H), 3.94 (s, 3H), 3.48 (t, J = 5.2 Hz, 4H), 1.73- 1.51 (m, 6H). ¹³C NMR (101 MHz, DMSO-*d₆*) δ 171.9, 160.5, 153.4, 148.6, 143.4, 132.6, 124.5, 122.3, 118.5, 116.6, 113.5, 107.2, 99.9, 56.3, 47.5, 35.7, 25.0, 23.9.

### 5-fluoro-2-(4-(piperidin-1-yl)styryl)benzo[d]thiazole (6a)

The synthesis was carried out according to general procedure A (402 mg, 99%). R*_{f}*: 0.45 (Hept/EtOAc 3:1). ¹H NMR (400 MHz, CDCl₃) δ 7.73 (dd, J = 8.8, 5.1 Hz, 1H), 7.62 (dd, J = 9.6, 2.5 Hz, 1H), 7.51 - 7.41 (m, 3H), 7.19 (d, J = 16.1 Hz, 1H), 7.09 (td, J = 8.8, 2.5 Hz, 1H), 6.91 (dt, J = 8.9, 3.0 Hz, 2H), 3.35 - 3.23 (m, 4H), 1.78 - 1.66 (m, 4H), 1.66-1.58 (m, 2H). ¹³C NMR (101 MHz, CDCl₃) δ 170.6, 162.1 (d, J = 242.8 Hz), 155.1 (d, J = 12.1 Hz), 152.7, 138.6, 129.7 (d, J = 1.9 Hz), 129.1, 125.1, 122.1 (d, J = 9.9 Hz), 118.0, 115.3, 113.4 (d, J = 25.0 Hz), 108.8 (d, J = 23.6 Hz), 49.4, 25.7, 24.5. ¹⁹F NMR (376 MHz, CDCl₃) δ -116.3.

### 5-fluoro-3-methyl-2-(4-(piperidin-1-yl)styryl)benzo[d]thiazol-3-ium iodide (6b)

The synthesis was carried out according to general procedure B (175 mg, quant.). R*_{f}*: 0.19 (DCM/MeOH 5%). ¹H NMR (400 MHz, DMSO-*d₆*) δ 8.34 (dd, J = 9.0, 5.1 Hz, 1H), 8.12 (dd, J = 9.7, 2.4 Hz, 1H), 8.06 (d, J = 15.3 Hz, 1H), 7.89 (d, J = 8.9 Hz, 2H), 7.64 - 7.55 (m, 2H), 7.04 (d, J = 8.9 Hz, 2H), 4.19 (s, 3H), 3.51 (t, J = 5.2 Hz, 4H), 1.71 - 1.54 (m, 6H). ¹³C NMR (101 MHz, DMSO-*d₆*) δ 173.7, 162.8 (d, J = 245.4 Hz), 154.2, 150.5, 143.7 (d, J = 12.6 Hz), 133.6, 126.1 (d, J = 10.0 Hz), 123.1 (d, J = 2.1 Hz), 122.7, 116.1 (d, J = 24.6 Hz), 113.9, 107.2, 104.0 (d, J = 28.9 Hz), 48.0, 36.3, 25.6, 24.4. ¹⁹F NMR (376 MHz, DMSO-*d₆*) δ -111.1.

### 6-methyl-2-(4-(piperidin-1-yl)styryl)benzo[d]thiazole (7a)

The synthesis was carried out according to general procedure A (132 mg, 26%). R*_{f}*: 0.31 (Hept/EtOAc 3:1). ¹H NMR (400 MHz, CDCl₃) δ 7.89 (d, J = 8.3 Hz, 1H), 7.69 (d, J = 1.7 Hz, 1H), 7.53 (dt, J = 8.8, 2.6 Hz, 1H), 7.47 (d, J = 16.1 Hz, 1H), 7.35 - 7.28 (m, 2H), 6.99 (d, J = 8.4 Hz, 2H), 3.47 - 3.23 (m, 4H), 2.55 (s, 3H), 1.78 (p, J = 5.7 Hz, 4H), 1.73-1.62 (m, 3H). ¹³C NMR (151 MHz, CDCl₃) δ 167.1, 152.6, 152.2, 137.6, 135.2, 134.5, 128.8, 127.8, 125.6, 122.2, 121.3, 118.6, 115.5, 49.6, 25.7, 24.5, 21.7.

### 3,6-dimethyl-2-(4-(piperidin-1-yl)styryl)benzo[d]thiazol-3-ium iodide (7b)

The synthesis was carried out according to general procedure B (273 mg, 97%). R*_{f}*: 0.27 (DCM/MeOH 5%). ¹H NMR (600 MHz, DMSO-*d₆*) δ 8.10 (s, 1H), 8.01 (d, J = 15.3 Hz, 2H), 8.00 (d, J = 8.6 Hz, 1H), 7.87 (d, J = 8.8 Hz, 2H), 7.62 (d, J = 15.3 Hz, 1H), 7.61 (dd, J = 8.6, 1.6 Hz, 1H), 7.04 (d, J = 8.8 Hz, 2H), 4.22 (s, 3H), 3.49 (t, J = 5.2 Hz, 4H), 2.51 (s, 1H), 1.70 - 1.62 (m, 2H), 1.62 - 1.56 (m, 4H). ¹³C NMR (151 MHz, DMSO-*d₆*) δ 170.5, 153.5, 149.1, 140.1, 137.9, 132.7, 130.1, 126.9, 123.3, 122.3, 115.7, 113.5, 107.0, 47.5, 35.6, 25.1, 23.9, 20.9.

### 6-methoxy-2-(4-(piperidin-1-yl)styryl)benzo[d]thiazole (8a)

The synthesis was carried out according to general procedure A (95 mg, 19%). R*_{f}*: 0.22 (Hept/EtOAc 3:1). ¹H NMR (400 MHz, CDCl₃) δ 7.83 (d, J = 8.9 Hz, 1H), 7.45 (d, J = 8.4 Hz, 2H), 7.34 (d, J = 16.1 Hz, 1H), 7.29 (d, J = 2.6 Hz, 1H), 7.19 (d, J = 16.1 Hz, 1H), 7.04 (dd, J = 8.9, 2.6 Hz, 1H), 6.91 (d, J = 8.4 Hz, 2H), 3.88 (s, 3H), 3.27 (t, J = 5.4 Hz, 4H), 1.70 (p, J = 5.6 Hz, 4H), 1.62 (q, J = 6.5 Hz, 2H). ¹³C NMR (151 MHz, CDCl₃) δ 165.8, 157.8, 152.5, 148.7, 137.1, 135.7, 128.7, 125.6, 123.2, 118.6, 115.5, 115.4, 104.4, 56.0, 49.6, 25.7, 24.5.

### 6-methoxy-3-methyl-2-(4-(piperidin-1-yl)styryl)benzo[d]thiazol-3-ium iodide (8b)

The synthesis was carried out according to general procedure B (89 mg, 32%). R*_{f}*: 0.18 (DCM/MeOH 5%). ¹H NMR (400 MHz, DMSO-*d₆*) δ 8.02 (d, J = 9.2 Hz, 1H), 7.99 - 7.91 (m, 2H), 7.86 (d, J = 8.5 Hz, 2H), 7.60 (d, J = 15.4 Hz, 1H), 7.38 (d, J = 9.2 Hz, 1H), 7.03 (d, J = 8.6 Hz, 2H), 4.21 (s, 3H), 3.90 (s, 3H), 3.57 - 3.44 (m, 4H), 1.82 - 1.47 (m, 6H). ¹³C NMR (101 MHz, DMSO-*d₆*) δ 169.2, 158.9, 153.4, 148.3, 136.1, 132.4, 128.7, 122.4, 117.6, 117.0, 113.5, 107.3, 106.7, 56.2, 47.5, 35.8, 25.0, 23.9.

### 6-fluoro-2-(4-(piperidin-1-yl)styryl)benzo[d]thiazole (9a - PFSB)

The synthesis was carried out according to general procedure A (34 mg, 24%). R*_{f}*: 0.38 (Hept/EtOAc 3:1). ¹H NMR (400 MHz, CDCl₃) δ 7.87 (dd, J = 8.9, 4.8 Hz, 1H), 7.50 (dd, J = 8.2, 2.6 Hz, 1H), 7.46 (d, J = 8.4 Hz, 2H), 7.39 (d, J = 16.1 Hz, 1H), 7.23 - 7.12 (m, 2H), 6.91 (d, J = 8.6 Hz, 2H), 3.28 (t, J = 5.3 Hz, 4H), 1.86 - 1.66 (m, 4H), 1.67 - 1.57 (m, 2H). ¹³C NMR (101 MHz, CDCl₃) δ 167.9 (d, J = 3.3 Hz), 160.5 (d, J = 245.1 Hz), 152.7, 150.8 (d, J = 1.7 Hz), 138.3, 135.3 (d, J = 11.0 Hz), 128.9, 125.2, 123.4 (d, J = 9.4 Hz), 118.1, 115.4, 114.7 (d, J = 24.6 Hz), 107.8 (d, J = 26.8 Hz), 49.5, 25.7, 24.5. ¹⁹F NMR (376 MHz, CDCl₃) δ -116.4.

### 6-fluoro-3-methyl-2-(4-(piperidin-1-yl)styryl)benzo[d]thiazol-3-ium iodide (9b)

The synthesis was carried out according to general procedure B (96 mg, 88%). R*_{f}*: 0.16 (DCM/MeOH 5%). ¹H NMR (400 MHz, DMSO-*d₆*) δ 8.25 (dd, J = 8.3, 2.7 Hz, 1H), 8.15 (dd, J = 9.1, 4.3 Hz, 1H), 8.04 (d, J = 15.3 Hz, 1H), 7.89 (d, J = 8.8 Hz, 2H), 7.70 (td, J = 9.1, 2.7 Hz, 1H), 7.62 (d, J = 15.3 Hz, 1H), 7.04 (d, J = 8.8 Hz, 2H), 4.22 (s, 3H), 3.50 (t, J = 5.2 Hz, 4H), 1.70 - 1.51 (m, 6H). ¹³C NMR (101 MHz, DMSO-*d₆*) δ 172.1 (d, J = 1.6 Hz), 161.0 (d, J = 246.6 Hz), 154.1, 150.4, 139.3, 133.4, 129.0 (d, J = 12.0 Hz), 122.7, 118.2 (d, J = 9.5 Hz), 117.6 (d, J = 25.6 Hz), 113.9, 111.1 (d, J = 28.8 Hz), 107.3, 48.0, 36.4, 25.6, 24.4. ¹⁹F NMR (376 MHz, DMSO-*d₆*) δ -112.6.

### 7-methyl-2-(4-(piperidin-1-yl)styryl)benzo[d]thiazole (10a)

The synthesis was carried out according to general procedure A (28 mg, 43%). R*_{f}*: 0.40 (Hept/EtOAc 3:1). ¹H NMR (400 MHz, CDCl₃) δ 7.79 (d, J = 8.1 Hz, 1H), 7.54 - 7.40 (m, 3H), 7.36 (t, J = 7.7 Hz, 1H), 7.23 (d, J = 16.1 Hz, 1H), 7.13 (d, J = 7.3 Hz, 1H), 6.91 (d, J = 8.4 Hz, 2H), 3.28 (t, J = 5.3 Hz, 4H), 2.56 (s, 3H), 1.70 (p, J = 5.4 Hz, 4H), 1.65 - 1.62 (m, 2H). ¹³C NMR (101 MHz, CDCl₃) δ 167.7, 154.0, 152.5, 138.0, 134.7, 131.6, 128.9, 126.4, 125.6, 125.3, 120.1, 118.6, 115.6, 49.7, 25.6, 24.5, 21.6.

### 3,7-dimethyl-2-(4-(piperidin-1-yl)styryl)benzo[d]thiazol-3-ium iodide (10b)

The synthesis was carried out according to general procedure B (11 mg, 64%). R*_{f}*: 0.19 (DCM/MeOH 5%). ¹H NMR (600 MHz, DMSO-*d₆*) δ 8.13 (d, J = 15.3 Hz, 1H), 7.95 (d, J = 8.4 Hz, 1H), 7.88 (d, J = 8.8 Hz, 2H), 7.72 (dd, J = 8.4, 7.4 Hz, 1H), 7.66 (d, J = 15.3 Hz, 1H), 7.54 (d, J = 7.4 Hz, 1H), 7.05 (d, J = 8.8 Hz, 2H), 4.24 (s, 3H), 3.51 (t, J = 5.4 Hz, 4H), 2.60 (s, 3H), 1.70-1.62 (m, 2H), 1.62-1.55 (m, 4H). ¹³C NMR (151 MHz, DMSO-*d₆*) δ 170.7, 153.6, 150.1, 141.8, 132.9, 132.7, 129.3, 127.9, 126.6, 122.3, 113.7, 113.5, 106.8, 47.5, 35.9, 25.1, 23.9, 19.3.

### 7-methoxy-2-(4-(piperidin-1-yl)styryl)benzo[d]thiazole (11a)

The synthesis was carried out according to general procedure A and purified by flash chromatography (PE/EtOAc 1% to 20% B) to afford the product as a yellow solid (25 mg, 13%). R*_{f}*: 0.41 (PE/EtOAc 4:1). ¹H NMR (600 MHz, CDCl₃) δ 7.60 (d, J = 8.1 Hz, 1H), 7.52 - 7.45 (m, 3H), 7.39 (t, J = 8.0 Hz, 1H), 7.24 (d, J = 15.8 Hz, 1H), 7.03 (s, 2H), 6.80 (d, J = 8.0 Hz, 1H), 3.99 (s, 3H), 3.30 (t, J = 5.5 Hz, 4H), 1.89 - 1.70 (m, 4H), 1.68-1.62 (m, 2H). ¹³C NMR (151 MHz, CDCl₃) δ 168.4, 155.6, 154.4, 154.3, 137.6, 129.0, 127.2, 126.8, 122.8, 119.2, 116.0, 115.5, 105.3, 56.1, 50.0, 25.3, 24.0.

### 7-methoxy-3-methyl-2-(4-(piperidin-1-yl)styryl)benzo[d]thiazol-3-ium iodide (11b)

The synthesis was carried out according to general procedure B (21 mg, 46%). R*_{f}*: 0.21 (DCM/MeOH 5%). ¹H NMR (600 MHz, DMSO-*d₆*) δ 8.12 (d, J = 15.3 Hz, 1H), 7.87 (d, J = 8.6 Hz, 2H), 7.75 (t, J = 8.2 Hz, 1H), 7.69 (d, J = 8.5 Hz, 1H), 7.63 (d, J = 15.3 Hz, 1H), 7.31 (d, J = 8.1 Hz, 1H), 7.05 (d, J = 8.6 Hz, 2H), 4.21 (s, 3H), 4.06 (s, 3H), 3.46 - 3.42 (m, 4H), 1.69 - 1.54 (m, 6H). ¹³C NMR (151 MHz, DMSO-*d₆*) δ 171.4, 153.6, 153.6, 150.2, 143.2, 133.0, 130.8, 122.2, 114.7, 113.4, 108.7, 108.4, 106.7, 56.9, 47.5, 36.0, 25.1, 23.9.

### 7-fluoro-2-(4-(piperidin-1-yl)styryl)benzo[d]thiazole (12a)

The synthesis was carried out according to general procedure A and purified by flash chromatography (PE/EtOAc 1% to 20% B) to afford the product as a yellow solid (119 mg, 72%). R*_{f}*: 0.49 (PE/EtOAc 4:1). ¹H NMR (600 MHz, CDCl₃) δ 7.75 (d, J = 8.1 Hz, 1H), 7.50 - 7.45 (m, 3H), 7.39 (td, J = 8.1, 5.4 Hz, 1H), 7.21 (d, J = 16.1 Hz, 1H), 7.05 (t, J = 8.6 Hz, 1H), 6.98 - 6.90 (m, 2H), 3.29 (t, J = 5.5 Hz, 4H), 1.71 (br s, 4H), 1.65 - 1.60 (m, 2H). ¹³C NMR (151 MHz, CDCl₃) δ 169.0, 157.1 (d, J = 248.7 Hz), 157.0 (d, J = 2.7 Hz), 152.5, 138.9, 129.1, 127.1 (d, J = 7.3 Hz), 125.3, 121.3 (d, J = 16.6 Hz), 118.4 (d, J = 3.5 Hz), 117.8, 115.6, 110.5 (d, J = 18.9 Hz), 49.7, 25.5, 24.3.

### 7-fluoro-3-methyl-2-(4-(piperidin-1-yl)styryl)benzo[d]thiazol-3-ium iodide (12b)

The synthesis was carried out according to general procedure B (42 mg, 69%). R*_{f}*: 0.19 (DCM/MeOH 5%). ¹H NMR (600 MHz, DMSO-*d₆*) δ 8.18 (d, J = 15.1 Hz, 1H), 7.96 (d, J = 8.4 Hz, 1H), 7.90 (d, J = 8.6 Hz, 2H), 7.83 (q, J = 8.0 Hz, 1H), 7.69 - 7.57 (m, 2H), 7.06 (d, J = 8.6 Hz, 2H), 4.22 (s, 3H), 3.54 (t, J = 5.4 Hz, 4H), 1.65 (q, J = 5.6 Hz, 2H), 1.63 - 1.53 (m, 4H). ¹³C NMR (151 MHz, DMSO-*d₆*) δ 172.2, 156.3 (d, J = 247.7 Hz), 154.4, 151.8, 144.9 (d, J = 15.5 Hz), 134.1, 131.4 (d, J = 7.5 Hz), 122.6, 114.4 (d, J = 23.1 Hz), 113.9, 113.0, 109.5, 106.5, 48.0, 36.7, 25.7, 24.4.

### 2-(4-(piperidin-1-yl)phenyl)benzo[d]thiazole (13a)

To a solution of 41 (140 mg, 0.48 mmol) in toluene (4.00 mL), Pd(PPh₃)₄ (28.0 mg, 0.02 mmol) and Cs₂CO₃ (140 mg, 0.72 mmol) were added under argon atmosphere. Piperidine (0.12 mL, 1.21 mmol) was diluted with toluene (1.00 mL) and slowly added. The mixture was refluxed for 5h. It was diluted with water and extracted with EtOAc. The organic phase was dried over MgSO₄, evaporated under reduced pressure, and purified by flash chromatography (PE/EtOAc 1% to 10% B) to afford the product (14.0 mg, 10%). R*_{f}*: 0.50 (PE/EtOAc 5:1). ¹H NMR (600 MHz, CDCl₃) δ 8.01 (d, J = 8.1 Hz, 1H), 7.98 (d, J = 8.5 Hz, 2H), 7.85 (d, J = 7.9 Hz, 1H), 7.47 - 7.42 (m, 1H), 7.35 - 7.29 (m, 1H), 7.02 (br s, 2H), 3.34 (t, J = 5.5 Hz, 4H), 1.77 - 1.74 (m, 4H), 1.65 (p, J = 5.7 Hz, 2H). ¹³C NMR (151 MHz, CDCl₃) δ 166.2, 154.2, 151.7, 134.7, 129.0, 128.3, 126.3, 124.7, 122.7, 121.7, 121.6, 52.7, 29.8, 25.3.

### 3-methyl-2-(4-(piperidin-1-yl)phenyl)benzo[d]thiazol-3-ium (13b)

To a solution of 13a (16.0 mg, 0.05 mmol) in chlorobenzene (1.50 mL) was added methyl nosylate (MeNs) (14.0 mg, 0.06 mmol). The mixture was stirred overnight at 80°C. The precipitate was filtered under vacuum and triturated in Et₂O. The yellow solid was further purified by semipreparative HPLC (0.1% TFA in H₂O/MeCN 20% to 60% B in 15 min) to isolate the product from the aniline N-methylated byproduct (10.0 mg, 59%). R*_{f}*: 0.13 (DCM/MeOH 5%). ¹H NMR (600 MHz, DMSO-*d₆*) δ 8.40 (dd, J = 8.1, 1.2 Hz, 1H), 8.25 (d, J = 8.4 Hz, 1H), 7.89 (ddd, J = 8.5, 7.2, 1.2 Hz, 1H), 7.81 (dt, J = 9.1, 3.2 Hz, 2H), 7.78 (ddd, J = 8.2, 7.2, 1.0 Hz, 1H), 7.19 (dt, J = 9.1, 3.2 Hz, 2H), 4.25 (s, 3H), 3.55 - 3.53 (m, 4H), 1.72 - 1.64 (m, 2H), 1.64 - 1.57 (m, 4H). ¹³C NMR (151 MHz, DMSO-*d₆*) δ 173.4, 153.8, 142.7, 132.5, 129.3, 128.1, 127.7, 123.9, 116.9, 113.5, 111.6, 47.4, 38.1, 24.9, 23.8.

### 2-(4-(4-(piperidin-1-yl)phenyl)buta-1,3-dien-1-yl)benzo[d]thiazole (14a)

The synthesis was carried out according to general procedure A (32 mg, 17%). R*_{f}*: 0.48 (PE/EtOAc4:1). ¹H NMR (600 MHz, CDCl₃) δ 7.90 (d, J = 8.1 Hz, 1H), 7.78 (d, J = 8.0 Hz, 1H), 7.39 (t, J = 7.7 Hz, 1H), 7.33 (d, J = 8.3 Hz, 2H), 7.29 (t, J = 7.5 Hz, 2H), 7.23 (d, J = 16.1 Hz, 1H), 6.84 (d, J = 7.7 Hz, 2H), 6.83 - 6.66 (m, 3H), 3.20 (t, J = 5.4 Hz, 4H), 1.64 (q, J = 5.8 Hz, 4H), 1.59 - 1.53 (m, 2H). ¹³C NMR (151 MHz, CDCl₃) δ 167.6, 154.1, 152.2, 139.3, 138.3, 134.4, 128.4, 126.8, 126.3, 125.1, 124.1, 123.4, 122.7, 121.5, 115.6, 49.7, 25.7, 24.4.

### 3-methyl-2-(4-(4-(piperidin-1-yl)phenyl)buta-1,3-dien-1-yl)benzo[d]thiazol-3-ium iodide (14b)

The synthesis was carried out according to general procedure B (109 mg, 87%). R*_{f}*: 0.16 (DCM/MeOH 5%). ¹H NMR (600 MHz, DMSO-*d₆*) δ 8.33 (d, J = 8.1 Hz, 1H), 8.15 (d, J = 8.4 Hz, 1H), 7.99 (dd, J = 14.6, 10.9 Hz, 1H), 7.81 (ddd, J = 8.5, 7.2, 1.2 Hz, 1H), 7.71 (ddd, J = 8.1, 7.3, 1.0 Hz, 1H), 7.53 (d, J = 8.8 Hz, 2H), 7.43 (d, J = 15.1 Hz, 1H), 7.35 (d, J = 14.6 Hz, 1H), 7.21 (dd, J = 15.1, 10.9 Hz, 1H), 7.00 (d, J = 8.8 Hz, 2H), 4.16 (s, 3H), 3.39 (t, J = 5.1 Hz, 4H), 1.63-1.56 (m, 6H). ¹³C NMR (151 MHz, DMSO-*d₆*) δ 170.6, 154.5, 152.4, 150.9, 147.4, 141.9, 130.4, 129.1, 127.8, 127.3, 124.0, 122.9, 116.3, 114.3, 112.9, 48.0, 35.7, 25.0, 24.0.

### 4-(4-(2-(benzo[d]thiazol-2-yl)vinyl)phenyl)morpholine (15a)

The synthesis was carried out according to general procedure A (47 mg, 59%). R*_{f}*: 0.21 (Hept/EtOAc 3:1). ¹H NMR (600 MHz, CDCl₃) δ 7.96 (d, J = 8.1 Hz, 1H), 7.84 (d, J = 8.0 Hz, 1H), 7.51 (d, J = 8.5 Hz, 2H), 7.46 (d, J = 16.1 Hz, 1H), 7.45 (t, J = 7.4 Hz, 1H), 7.35 (t, J = 7.6 Hz, 1H), 7.26 (d, J = 16.1 Hz, 1H), 6.91 (d, J = 8.5 Hz, 2H), 3.87 (t, J = 4.9 Hz, 4H), 3.25 (t, J = 4.8 Hz, 4H).¹³C NMR (151 MHz, CDCl₃) δ 167.8, 154.1, 152.1, 137.7, 134.4, 128.9, 126.8, 126.3, 125.1, 122.8, 121.6, 119.2, 115.2, 66.9, 48.5.

### 3-methyl-2-(4-morpholinostyryl)benzo[d]thiazol-3-ium iodide (15b)

The synthesis was carried out according to general procedure B (52 mg, 69 %). R*_{f}*: 0.13 (DCM/MeOH 5%). ¹H NMR (600 MHz, DMSO-*d₆*) δ 8.34 (dd, J = 8.2, 1.2 Hz, 1H), 8.14 (d, J = 8.3 Hz, 1H), 8.10 (d, J = 15.4 Hz, 1H), 7.95 (dt, J = 9.0, 2.9 Hz, 2H), 7.81 (ddd, J = 8.5, 7.2, 1.2 Hz, 1H), 7.74 (d, J = 15.4 Hz, 1H), 7.72 (ddd, J = 8.2, 7.2, 1.0 Hz, 1H), 7.09 (dt, J = 9.0, 3.1 Hz, 2H), 4.27 (s, 3H), 3.80 - 3.70 (m, 4H), 3.42 (dd, J = 5.7, 4.1 Hz, 4H). ¹³C NMR (151 MHz, DMSO-*d₆*) δ 171.7, 153.8, 149.5, 142.0, 132.4, 129.0, 127.7, 127.1, 123.9, 123.5, 116.2, 113.6, 108.2, 65.8, 46.5, 35.8.

### 2-(4-thiomorpholinostyryl)benzo[d]thiazole (16a)

The synthesis was carried out according to general procedure A (6 mg, 11%). R*_{f}*: 0.39 (Hept/EtOAc 3:1). ¹H NMR (600 MHz, CDCl₃) δ 7.96 (d, J = 8.1 Hz, 1H), 7.84 (d, J = 7.9 Hz, 1H), 7.49 (d, J = 8.5 Hz, 2H), 7.47 - 7.42 (m, 2H), 7.35 (td, J = 8.0, 1.1 Hz, 1H), 7.25 (d, J = 16.1 Hz, 1H), 6.88 (d, J = 8.3 Hz, 2H), 3.77 - 3.67 (m, 4H), 2.74 (t, J = 5.1 Hz, 4H). ¹³C NMR (151 MHz, CDCl₃) δ 167.9, 154.0, 151.2, 137.8, 134.3, 129.1, 126.4, 126.1, 125.1, 122.7, 121.6, 119.0, 116.0, 51.2, 26.3.

### 3-methyl-2-(4-thiomorpholinostyryl)benzo[d]thiazol-3-ium iodide (16b)

The synthesis was carried out according to general procedure B (33 mg, 65%). R*_{f}*: 0.13 (DCM/MeOH 5%). ¹H NMR (600 MHz, DMSO-*d₆*) δ 8.33 (dd, J = 8.1, 1.2 Hz, 1H), 8.13 (d, J = 8.4 Hz, 1H), 8.08 (d, J = 15.4 Hz, 1H), 7.97 - 7.89 (m, 2H), 7.81 (ddd, J = 8.5, 7.2, 1.2 Hz, 1H), 7.74 - 7.66 (m, 2H), 7.07 (d, J = 9.0 Hz, 2H), 4.26 (s, 3H), 3.94 - 3.84 (m, 4H), 2.72 - 2.63 (m, 4H). ¹³C NMR (151 MHz, DMSO-*d₆*) δ 171.6, 152.3, 149.5, 142.0, 132.8, 128.9, 127.6, 127.0, 123.9, 122.7, 116.1, 113.9, 107.6, 49.5, 35.7, 25.2.

### 2-(4-(pyrrolidin-1-yl)styryl)benzo[d]thiazole (17a)

The synthesis was carried out according to general procedure A (15 mg, 15%). R*_{f}*: 0.50 (Hept/EtOAc 3:1). ¹H NMR (600 MHz, CDCl₃) δ 7.93 (d, J = 8.1 Hz, 1H), 7.82 (d, J = 7.9 Hz, 1H), 7.55 - 7.38 (m, 4H), 7.31 (t, J = 7.5 Hz, 1H), 7.18 (d, J = 16.1 Hz, 1H), 6.57 (d, J = 8.3 Hz, 2H), 3.35 (t, J = 6.1 Hz, 4H), 2.03 (hept, J = 3.4 Hz, 4H). ¹³C NMR (151 MHz, CDCl₃) δ 168.6, 154.2, 148.9, 138.8, 134.2, 129.2, 126.2, 124.8, 122.8, 122.5, 121.5, 116.7, 112.0, 47.7, 25.6.

### 3-methyl-2-(4-(pyrrolidin-1-yl)styryl)benzo[d]thiazol-3-ium iodide (17b)

The synthesis was carried out according to general procedure B (118 mg, 96%). R*_{f}*: 0.16 (DCM/MeOH 5%). ¹H NMR (400 MHz, DMSO-*d₆*) δ 8.28 (d, J = 8.0 Hz, 1H), 8.12 - 7.99 (m, 2H), 7.90 (d, J = 8.6 Hz, 2H), 7.77 (t, J = 7.6 Hz, 1H), 7.67 (t, J = 7.6 Hz, 1H), 7.58 (d, J = 15.2 Hz, 1H), 6.69 (d, J = 8.6 Hz, 2H), 4.21 (s, 3H), 3.43 - 3.35 (m, 4H), 2.00 (p, J = 3.2 Hz, 4H). ¹³C NMR (101 MHz, DMSO-*d₆*) δ 171.1, 151.0, 150.3, 141.9, 133.1, 128.8, 127.3, 126.7, 123.7, 121.3, 115.8, 112.4, 105.6, 47.6, 35.4, 24.8.

### 2-(4-(4-fluoropiperidin-1-yl)styryl)benzo[d]thiazole (18a)

The synthesis was carried out according to general procedure A (84 mg, 57%). R*_{f}*: 0.23 (Hept/EtOAc 3:1). ¹H NMR (400 MHz, DMSO-*d₆*) δ 8.04 (dd, J = 7.9, 1.1 Hz, 1H), 7.91 (d, J = 8.1 Hz, 1H), 7.61 (d, J = 8.6 Hz, 2H), 7.54 (d, J = 16.1 Hz, 1H), 7.48 (td, J = 7.2, 1.0 Hz, 1H), 7.39 (td, J = 8.2, 0.9 Hz, 1H), 7.36 (d, J = 16.1 Hz, 1H), 7.00 (d, J = 8.4 Hz, 2H), 4.87 (dtt, J = 49.0, 7.3, 3.6 Hz, 1H), 3.58 - 3.42 (m, 2H), 3.27 (ddd, J = 12.6, 7.7, 3.8 Hz, 2H), 2.05 - 1.87 (m, 2H), 1.83 - 1.69 (m, 2H). ¹³C NMR (101 MHz, DMSO-*d₆*) δ 167.7, 154.1, 151.5, 138.3, 134.2, 129.6, 126.8, 125.5, 125.3, 122.6, 122.5, 118.1, 115.3, 89.0 (d, J = 169.4 Hz), 44.5 (d, J = 6.9 Hz), 30.9 (d, J = 19.1 Hz).

### 2-(4-(4-fluoropiperidin-1-yl)styryl)-3-methylbenzo[d]thiazol-3-ium iodide (18b)

The synthesis was carried out according to general procedure B (143 mg, 87%). R*_{f}*: 0.19 (DCM/MeOH 5%). ¹H NMR (400 MHz, DMSO-*d₆*) δ 8.33 (dd, J = 8.1, 1.2 Hz, 1H), 8.13 (d, J = 8.4 Hz, 1H), 8.07 (d, J = 15.5 Hz, 1H), 7.92 (d, J = 8.7 Hz, 2H), 7.80 (td, J = 8.1, 7.2, 1.3 Hz, 1H), 7.75 - 7.64 (m, 2H), 7.11 (d, J = 8.7 Hz, 2H), 4.93 (dtt, J = 48.9, 7.1, 3.5 Hz, 1H), 4.26 (s, 3H), 3.76 - 3.58 (m, 2H), 3.55 - 3.44 (m, 2H), 1.98 (dddd, J = 24.8, 16.1, 7.5, 3.6 Hz, 2H), 1.78 (dtt, J = 14.1, 7.2, 3.8 Hz, 2H). ¹³C NMR (101 MHz, DMSO-*d₆*) δ 172.1, 153.5, 150.0, 142.4, 133.2, 129.4, 128.1, 127.5, 124.4, 123.3, 116.6, 114.3, 108.1, 88.7 (d, J = 169.5 Hz), 43.6 (d, J = 6.6 Hz), 36.2, 31.0 (d, J = 19.4 Hz). ¹⁹F NMR (376 MHz, DMSO-*d₆*) δ -177.5.

### 4-(2-(benzo[d]thiazol-2-yl)vinyl)-N,N-dimethylaniline (19a)

The synthesis was carried out according to general procedure A (58 mg, 34%). R*_{f}*: 0.40 (Hept/EtOAc 3:1). ¹H NMR (600 MHz, CDCl₃) δ 7.94 (dt, J = 8.0, 0.9 Hz, 1H), 7.82 (dt, J = 7.7, 0.9 Hz, 1H), 7.48 (dt, J = 8.8, 2.8 Hz, 2H), 7.45 (d, J = 16.1 Hz, 1H), 7.43 (ddd, J = 8.3, 7.2, 1.2 Hz, 1H), 7.32 (ddd, J = 8.2, 7.2, 1.2 Hz, 1H), 7.21 (d, J = 16.1 Hz, 1H), 6.72 (dt, J = 8.9, 2.9 Hz, 2H), 3.03 (s, 6H). ¹³C NMR (151 MHz, CDCl₃) δ 168.4, 154.2, 151.4, 138.5, 134.3, 129.1, 126.2, 124.9, 123.5, 122.6, 121.5, 117.4, 112.2, 40.4.

### 2-(4-(dimethylamino)styryl)-3-methylbenzo[d]thiazol-3-ium iodide (19b)

The synthesis was carried out according to general procedure B (125 mg, 90%). R*_{f}*: 0.14 (DCM/MeOH 5%). ¹H NMR (400 MHz, DMSO-*d₆*) δ 8.30 (dd, J = 8.1, 1.2 Hz, 1H), 8.15 - 7.99 (m, 2H), 7.91 (d, J = 8.9 Hz, 2H), 7.78 (ddd, J = 8.5, 7.2, 1.2 Hz, 1H), 7.72 - 7.65 (m, 1H), 7.62 (d, J = 15.3 Hz, 1H), 6.84 (d, J = 8.9 Hz, 2H), 4.23 (s, 3H), 3.10 (s, 6H). ¹³C NMR (101 MHz, DMSO-*d₆*) δ 171.3, 153.5, 150.1, 141.9, 132.8, 128.8, 127.4, 126.8, 123.8, 121.4, 115.9, 111.9, 106.2, 39.8, 35.5.

### 2-(4-(piperidin-1-yl)styryl)benzo[d]thiazole (20)

The synthesis was carried out according to general procedure A (1.14 g, 53%). R*_{f}*: 0.38 (Hept/EtOAc 3:1). ¹H NMR (400 MHz, CDCl₃) δ 7.95 (d, J = 8.1 Hz, 1H), 7.83 (d, J = 7.9 Hz, 1H), 7.53 - 7.39 (m, 4H), 7.33 (t, J = 7.6 Hz, 1H), 7.23 (d, J = 16.2 Hz, 1H), 6.91 (d, J = 8.5 Hz, 2H), 3.28 (t, J = 5.6 Hz, 4H), 1.70 (p, J = 5.6 Hz, 4H), 1.63 (q, J = 6.5, 5.6 Hz, 2H). ¹³C NMR (101 MHz, CDCl₃) δ 168.1, 154.2, 152.6, 138.1, 134.3, 128.9, 126.3, 125.4, 125.0, 122.7, 121.5, 118.4, 115.4, 49.5, 25.7, 24.5.

### 3-methyl-2-(4-(piperidin-1-yl)styryl)benzo[d]thiazol-3-ium iodide (RB1)

The synthesis was carried out according to general procedure B (322 mg, 81%). R*_{f}*: 0.17 (DCM/MeOH 5%). ¹H NMR (400 MHz, DMSO-*d₆*) δ 8.31 (dd, J = 8.2, 1.2 Hz, 1H), 8.11 (d, J = 8.4 Hz, 1H), 8.05 (d, J = 16.1 Hz, 1H), 7.90 (d, J = 9.0 Hz, 2H), 7.79 (ddd, J = 8.5, 7.3, 1.3 Hz, 1H), 7.73 - 7.62 (m, 2H), 7.05 (d, J = 8.7 Hz, 2H), 4.24 (s, 3H), 3.50 (t, J = 5.1 Hz, 4H), 1.69 - 1.51 (m, 6H). ¹³C NMR (101 MHz, DMSO-*d₆*) δ 171.9, 154.1, 150.2, 142.4, 133.4, 129.4, 128.0, 127.4, 124.3, 122.7, 116.5, 113.9, 107.4, 48.0, 36.1, 25.6, 24.4.

### 3-methyl-2-(4-(4-methylpiperazin-1-yl)styryl)benzo[d]thiazol-3-ium iodide (RB2)

The synthesis was carried out according to general procedure B (159 mg, 97%). R*_{f}*: 0.19 (DCM/MeOH 5%). ¹H NMR (400 MHz, DMSO-*d₆*) δ 8.35 (d, J = 7.9 Hz, 1H), 8.15 (d, J = 8.4 Hz, 1H), 8.10 (d, J = 15.5 Hz, 1H), 7.95 (d, J = 8.5 Hz, 2H), 7.82 (t, J = 7.9 Hz, 1H), 7.75 (d, J = 15.5 Hz, 1H), 7.72 (t, J = 7.7 Hz, 1H), 7.11 (d, J = 8.5 Hz, 2H), 4.28 (s, 3H), 3.56 (br s, 4H), 2.85 (br s, 4H), 2.53 (s, 3H). ¹³C NMR (101 MHz, DMSO-*d₆*) δ 171.7, 153.0, 149.3, 141.9, 132.4, 129.0, 127.7, 127.1, 123.9, 123.7, 116.2, 114.0, 108.4, 53.3, 45.2, 44.0, 35.9.

### 6-fluoro-2-(4-(4-(piperidin-1-yl)phenyl)buta-1,3-dien-1-yl)benzo[d]thiazole (43)

The synthesis was carried out according to general procedure A (37 mg, 28%). R*_{f}*: 0.47 (PE/EtOAc4:1). ¹H NMR (600 MHz, CDCl₃) δ 7.90 (dd, J = 8.9, 4.8 Hz, 1H), 7.52 (dd, J = 7.1, 1.6 Hz, 1H), 7.40 (d, J = 8.3 Hz, 2H), 7.33 - 7.27 (m, 1H), 7.19 (t, J = 8.7 Hz, 1H), 6.92 (d, J = 8.3 Hz, 2H), 6.88 - 6.80 (m, 3H), 3.27 (t, J = 5.4 Hz, 4H), 1.81 1.68 (m, 4H), 1.64 (p, J = 5.7 Hz, 2H). ¹³C NMR (151 MHz, CDCl₃) δ 167.3, 160.5 (d, J = 245.6 Hz), 152.1, 150.8 (d, J = 1.5 Hz), 139.3, 138.4, 135.4 (d, J = 11.2 Hz), 128.4, 126.9, 124.0, 123.5 (d, J = 9.3 Hz), 123.1, 115.7, 114.8 (d, J = 24.8 Hz), 107.8 (d, J = 27.0 Hz), 49.8, 25.6, 24.4.

### 6-fluoro-2-(4-(4-(pyrrolidin-1-yl)phenyl)buta-1,3-dien-1-yl)benzo[d]thiazole (44)

The synthesis was carried out according to general procedure A (24 mg, 36%). R*_{f}*: 0.51 (PE/EtOAc4:1). ¹H NMR (600 MHz, CDCl₃) δ 7.92 - 7.77 (m, 1H), 7.46 (d, J = 8.3 Hz, 1H), 7.34 (d, J = 8.1 Hz, 2H), 7.25 - 7.20 (m, 1H), 7.13 (t, J = 9.1 Hz, 1H), 6.93 - 6.68 (m, 3H), 6.52 (d, J = 8.8 Hz, 2H), 3.31 (s, 4H), 2.00 (s, 4H). ¹³C NMR (151 MHz, CDCl₃) δ 167.7, 162.1 (d, J = 239.1 Hz), 150.5, 148.3, 140.1, 139.4, 137.2, 128.8, 127.8, 123.3 (d, J = 8.4 Hz), 122.4, 121.9, 114.8 (d, J = 24.0 Hz), 112.1, 107.8 (d, J = 26.8 Hz), 47.9, 25.6.

### 4-(4-(2-(6-fluorobenzo[d]thiazol-2-yl)vinyl)phenyl)morpholine (45 - MFSB)

The synthesis was carried out according to general procedure A (21 mg, 69%). R*_{f}*: 0.18 (PE/EtOAc4:1). ¹H NMR (600 MHz, CDCl₃) δ 7.90 (dd, J = 9.0, 4.8 Hz, 1H), 7.58 - 7.47 (m, 3H), 7.43 (d, J = 16.0 Hz, 1H), 7.24 (d, J = 16.0 Hz, 1H), 7.19 (td, J = 8.9, 2.6 Hz, 1H), 6.96 (d, J = 8.2 Hz, 2H), 3.90 (t, J = 4.6 Hz, 4H), 3.27 (t, J = 4.2 Hz, 4H). ¹³C NMR (151 MHz, CDCl₃) δ 167.6, 160.6 (d, J = 245.5 Hz), 151.8, 150.4, 138.0, 135.2, 129.0, 127.0, 123.5 (d, J = 9.0 Hz), 118.9, 115.4, 115.0 (d, J = 25.1 Hz), 107.9 (d, J = 27.1 Hz), 66.7, 48.7.

### 6-bromo-2-(4-(piperidin-1-yl)styryl)benzo[d]thiazole (46)

The synthesis was carried out according to general procedure A (2.80 g, 80%). R*_{f}*: 0.51 (PE/EtOAc4:1). ¹H NMR (600 MHz, CDCl₃) δ 7.94 (s, 1H), 7.78 (d, J = 8.6 Hz, 1H), 7.52 (d, J = 8.6 Hz, 1H), 7.46 (d, J = 8.3 Hz, 2H), 7.43 (d, J = 16.0 Hz, 1H), 7.18 (d, J = 16.0 Hz, 1H), 6.92 (br s, 2H), 3.29 (t, J = 5.4 Hz, 4H), 1.70 (br s, 4H), 1.63 (q, J = 5.7 Hz, 2H). ¹³C NMR (151 MHz, CDCl₃) δ 168.6, 153.0, 152.5, 138.7, 136.0, 129.7, 129.0, 125.3, 124.0, 123.7, 118.4, 117.9, 115.5, 49.6, 25.5, 24.4.

### 4-(4-(2-(6-bromobenzo[d]thiazol-2-yl)vinyl)phenyl)morpholine (47)

The synthesis was carried out according to general procedure A (639 mg, 73%). R*_{f}*: 0.15 (PE/EtOAc4:1). ¹H NMR (600 MHz, CDCl₃) δ 7.97 (d, J = 1.9 Hz, 1H), 7.82 (d, J = 8.6 Hz, 1H), 7.55 (dd, J = 8.6, 1.9 Hz, 1H), 7.53 (dt, J = 8.7, 2.0 Hz, 2H), 7.48 (d, J = 16.1 Hz, 1H), 7.25 (d, J = 16.1 Hz, 1H), 7.00 (d, J = 8.4 Hz, 2H), 3.92 (t, J = 4.8 Hz, 4H), 3.30 - 3.26 (m, 4H). ¹³C NMR (151 MHz, CDCl₃) δ 168.3, 151.9, 151.0, 138.7, 135.6, 130.1, 129.3, 126.2, 124.2, 123.6, 118.9, 118.7, 116.0, 66.4, 49.2.

### 2-(4-(piperidin-1-yl)styryl)-6-(4,4,5,5-tetramethyl-1,3,2-dioxaborolan-2-yl)benzo[d]thiazole (48)

Pd(dppf)Cb (303 mg, 0.40 mmol) was added to a solution of 46 (1.60 g, 4.01 mmol), potassium acetate (786 mg, 8.01 mmol) and bis(pinacolato)diboron (1.53 g, 6.01 mmol) in dry DMF (34.0 mL). The mixture was heated at 100°C for 45 min. It was poured into water and extracted with EtOAc. The organic phase was dried over MgSO₄, evaporated under reduced pressure, and purified by flash chromatography (PE/EtOAc 5% to 20% B) to afford the product as a yellow solid (1.66 g, 93%). R*_{f}*: 0.35 (PE/EtOAc 6:1). ¹H NMR (600 MHz, CDCl₃) δ 8.31 (d, J = 1.1 Hz, 1H), 7.93 (d, J = 8.1 Hz, 1H), 7.87 (dd, J = 8.1, 1.1 Hz, 1H), 7.49 - 7.44 (m, 3H), 7.23 (d, J = 16.1 Hz, 1H), 6.90 (d, J = 8.4 Hz, 2H), 3.29 - 3.23 (m, 4H), 1.68 (p, J = 5.5 Hz, 4H), 1.60 (q, J = 7.0, 6.4 Hz, 2H), 1.37 (s, 12H). ¹³C NMR (151 MHz, CDCl₃) δ 169.6, 156.1, 152.4, 138.5, 133.8, 132.3, 129.0, 129.0, 128.4, 125.4, 121.8, 118.4, 115.4, 84.1, 49.5, 25.5, 25.0, 24.3.

### 4-(4-(2-(6-(4,4,5,5-tetramethyl-1,3,2-dioxaborolan-2-yl)benzo[d]thiazol-2-yl)vinyl)phenyl)morpholine (49)

The synthesis was carried out following the same procedure as 48 (124 mg, 18%). R*_{f}*: 0.17 (PE/EtOAc 3:1). ¹H NMR (600 MHz, CDCl₃) δ 8.31 (d, J = 1.1 Hz, 1H), 7.94 (d, J = 8.1 Hz, 1H), 7.87 (dd, J = 8.1, 1.1 Hz, 1H), 7.49 (d, J = 8.7 Hz, 2H), 7.48 (d, J = 16.1 Hz, 1H), 7.26 (d, J = 16.1 Hz, 1H), 6.89 (dt, J = 8.8, 2.9 Hz, 2H), 3.87 - 3.84 (m, 4H), 3.25 - 3.21 (m, 4H), 1.36 (s, 12H). ¹³C NMR (151 MHz, CDCl₃) δ 169.5, 155.6, 152.0, 138.5, 133.6, 132.4, 130.7, 129.0, 128.5, 126.6, 121.8, 118.8, 115.1, 84.2, 66.7, 48.3, 25.0.

### Manual synthesis of [¹⁸F]PFSB

[¹⁸F]Fluoride was produced by a PETtrace 890 cyclotron (GE Healthcare, Uppsala, Sweden) and delivered as a target wash in H₂O. A Sep-Pak QMA carb light cartridge was conditioned with a sequence of: 10 mL of KOTf aq (90 mg/mL), 10 mL of air, 10 mL of H₂O, 10 mL of air. [¹⁸F]Fluoride was trapped into the QMA cartridge, dried with argon (10 mL passed through the cartridge) and eluted with a solution of TBAOTf in MeOH (10 mg/1 mL) to afford [¹⁸F]TBAF. The resulting solution was aliquoted in 4 reaction vials and MeOH was evaporated at 90°C.

A stock solution of Cu(OTf)₂ in DMA (0.1 mg/µL) was prepared. To prepare each reaction mixture, 25.5 µL of the stock solution were diluted in the chosen amount of DMA (510 µL for a and b, 499 µL for c and d, Table 4) and *n*-BuOH (60.0 µL) and pyridine (4.90 µL, 60 µmol or 16.0 µL, 200 µmol; Table 4) were added. The solution was added to the precursor 48 (9.00 mg, 20.2 µmol or 4.50 mg, 10.1 µmol; Table 4). The mixture was sonicated, added to the correspondent reaction vial and heated at 120°C for 20 min. The reaction was quenched with 1 mL HCl 0.1 M and neutralized with 1 mL NaOH 0.1 M. 500 µL MeCN were added to each mixture to avoid product precipitation. Reaction performance was evaluated by radioTLC (PE/EtOAc 2:1) and radioHPLC. The evaluation allowed optimization of the reaction conditions for the automated synthesis.

### Automated synthesis of [¹⁸F]PFSB and [¹⁸F]MFSB

A Sep-Pak Plus Light QMA carb cartridge was conditioned with a sequence of: 10 mL of KOTf aq (90 mg/mL), 10 mL of air, 10 mL of H₂O, 10 mL of air. A Sep-Pak Plus Light Alumin N cartridge was prepared with 5 mL of H₂O. A Sep-Pak Plus tC18 and a Sep-Pak Light C18 were conditioned with 10 mL of EtOH and 10 mL of H₂O each. To prepare the reaction mixture, Cu(OTf)₂ (2.40 mg, 6.72 µmol) was dissolved in 535.7 µL of DMA. n-BuOH (60.0 µL) and pyridine (4.30 µL, 53.8 µmol) were added. The solution was added to the PFSB precursor 48 (4.00 mg, 8.96 µmol) or the MFSB precursor 49 (4.00 mg, 8.92 µmol) and sonicated.

[¹⁸F]Fluoride was produced by a PETtrace 890 cyclotron (GE Healthcare, Uppsala, Sweden) and delivered into an FxNPro module (GE Heatlhcare, Münster, Germany). It was trapped into the QMA cartridge, eluted into the reactor with a solution of TBAOTf in MeOH (10 mg/1 mL) and the solvent evaporated at 90°C. The reaction mixture was added and the reactor was heated at 120°C for 20 min. The resulting mixture was diluted with 10 mL of MeCN/ammonium formate buffer (25 mM, pH 8) 1:1 v/v and trapped on a overlap of Alox and tC18 cartridges. The product was eluted with MeCN (3.4 mL for [¹⁸F]PFSB; 2.8 mL for [¹⁸F]MFSB) into tube 2, which was equipped with 25 mM ammonium formate (1.6 mL for [¹⁸F]PFSB; 2.2 mL for [¹⁸F]MFSB). The mixture was injected into the HPLC loop. Semi-preparative HPLC conditions for purification: Luna 5 µm C8 (2) 100 Å 250 x 10 mm; 68% MeCN in 25 mM ammonium formate at pH 8 (retention time ≈ 17 min) for [¹⁸F]PFSB; 55% MeCN in 25 mM ammonium formate at pH 8 (retention time ≈ 12 min) for [¹⁸F]MFSB; 6 mL/min.

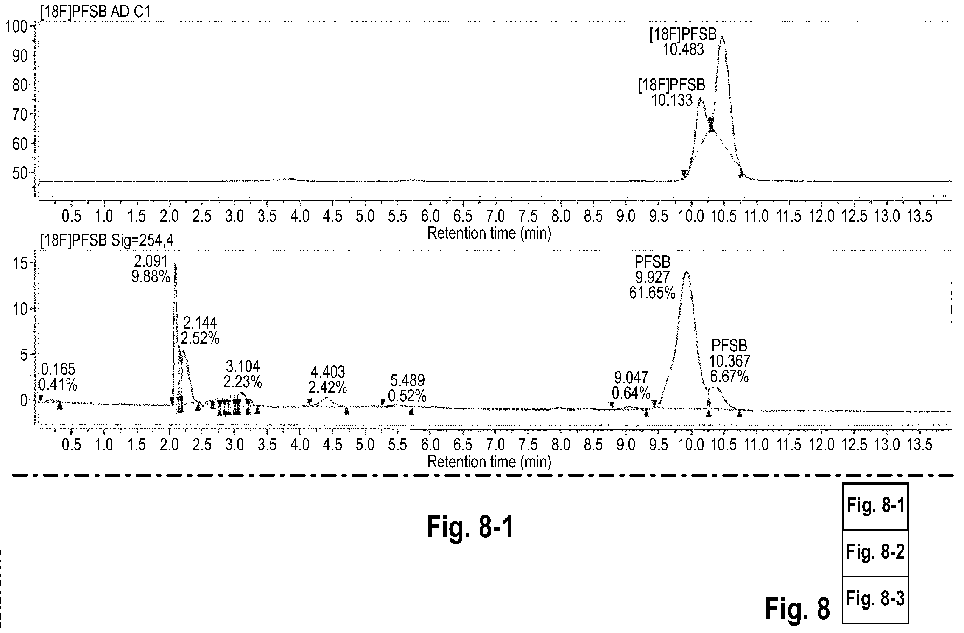

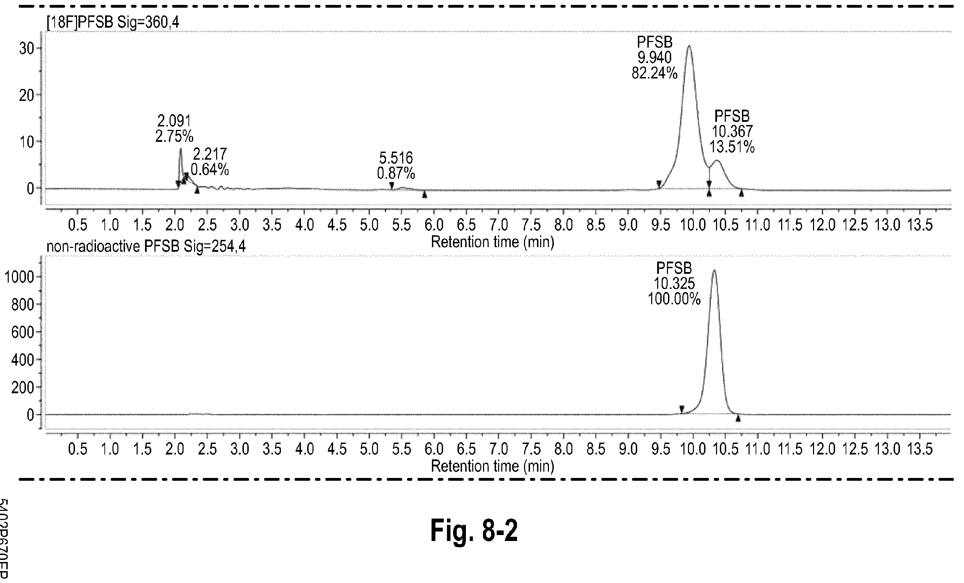

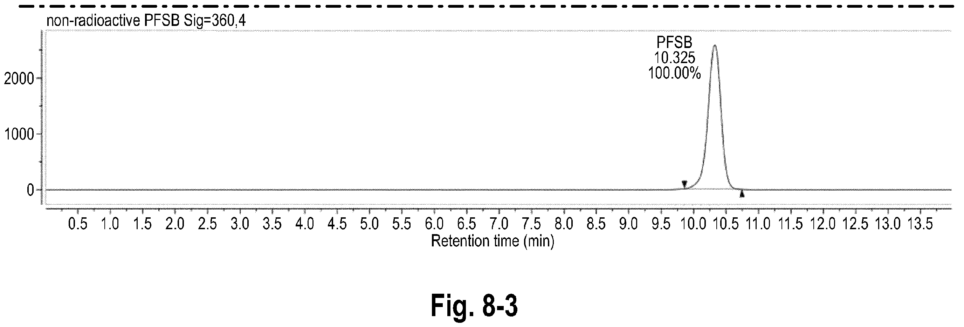

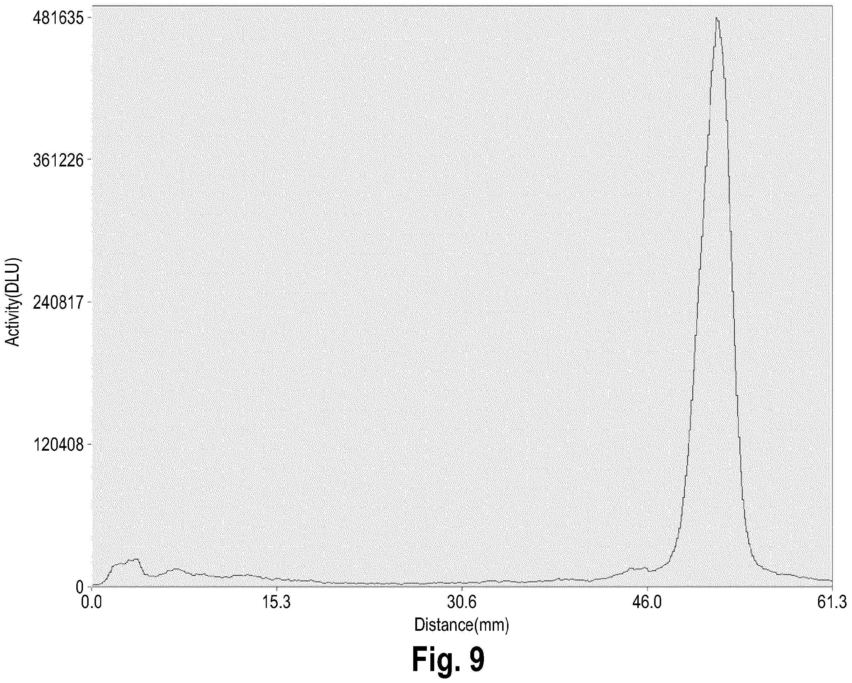

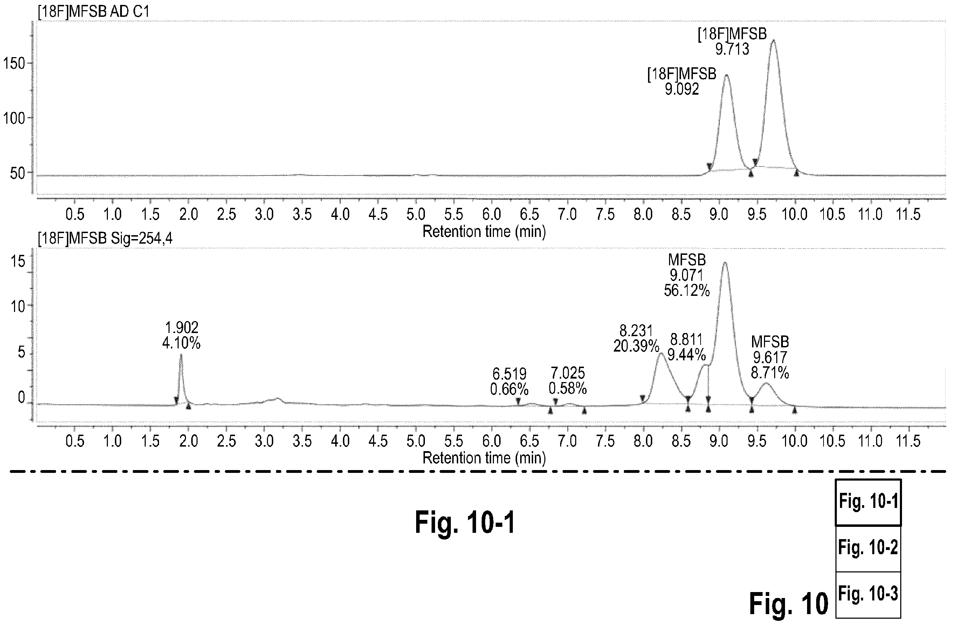

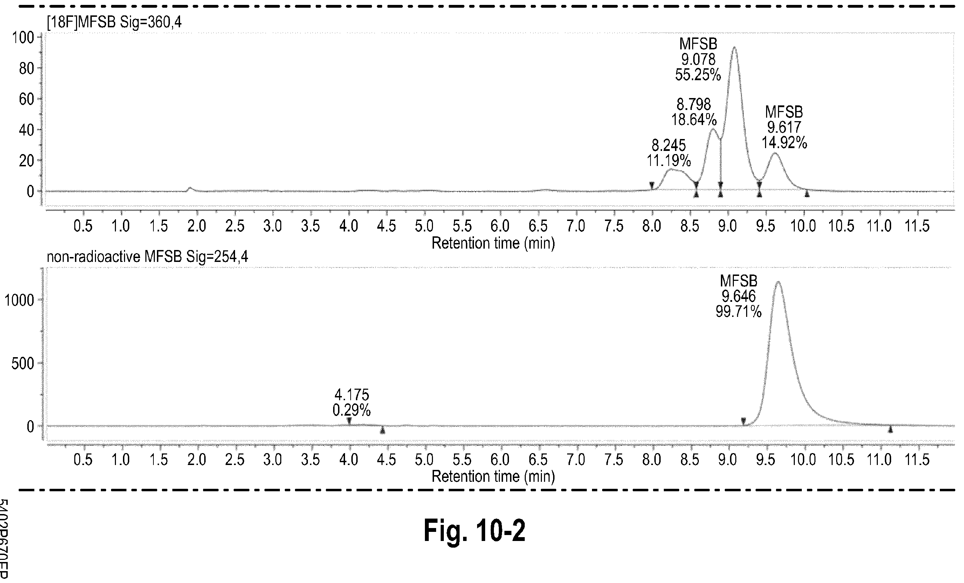

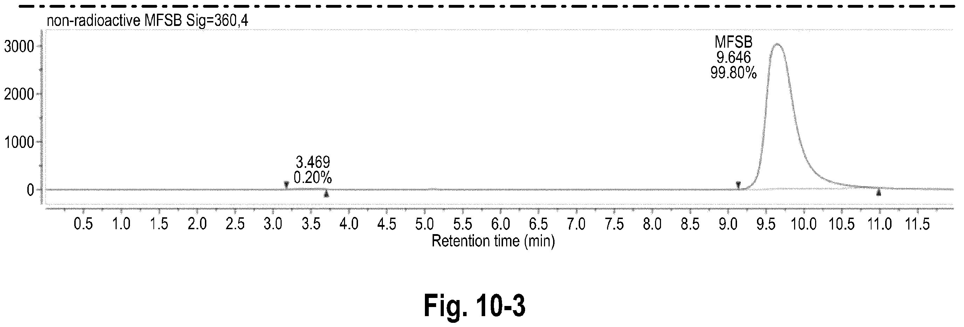

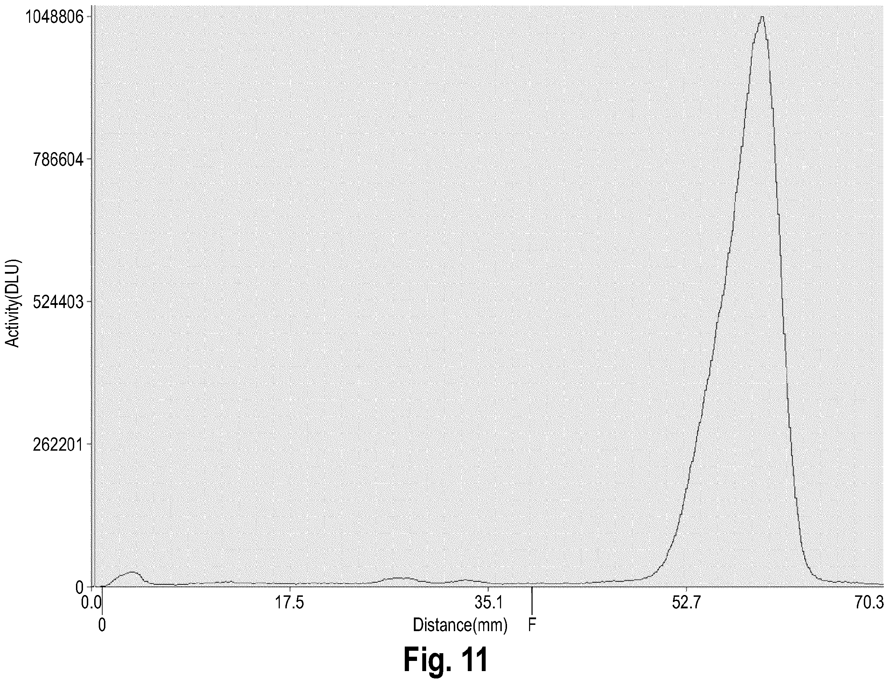

The product peak was cut, diluted with water (55 mL), and trapped into a C18 cartridge. It was washed with water (5 mL), eluted with EtOH (0.5 mL), formulated with PBS (4.5 mL) and transferred into the product vial. Quality control (QC) was performed (analytical HPLC conditions and chromatograms are reported in Fig. 8 to Fig 11).

### Tritiation of Pittsburgh Compound-B [³H]PiB and [³H]MODAG-001

PiB and MODAG-001 (4-(3-(2-bromopyridin-4-yl)-1H-pyrazol-5-yl)-N,N-dimethylaniline) were tritiated by RC Tritec AG (Teufen, Switzerland), dissolved in EtOH, and stored at -80°C until use. Radiochemical purities of > 99% were achieved for both radioligands and the molar activities (Aₘ) of [³H]PiB and [³H]MODAG-001 were 21.7 Ci/mmol and 78.9 Ci/mmol, respectively.

### 1.1.4. General procedures

### 1.1.4.1. General procedure S1

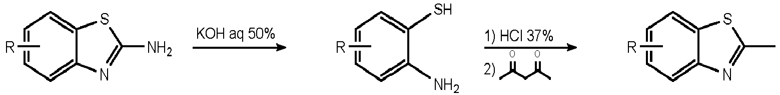

A suspension of the selected 2-aminobenzothiazole (7.36 mmol) in KOH aq 50% (28.0 mL) was refluxed for 18h to 42h. The mixture was acidified by addition of HCl 37% (20.0 mL). Color changed and precipitate formed. 2,4-pentanedione (11.04 mmol) was added and the reaction was stirred at room temperature for 90 min. The mixture was extracted with EtOAc. The organic phase was dried over MgSO₄, evaporated under reduced pressure, and purified by flash chromatography (Hept/EtOAc).

### 1.1.4.2. General procedure S2

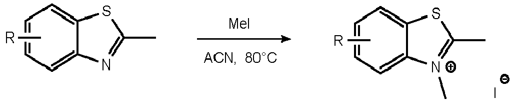

To a solution of the selected 2-methylbenzothiazole (8.24 mmol) in MeCN (25.0 mL) was added methyl iodide (16.5 mmol). The mixture was stirred overnight at 80°C. The precipitate was filtered under vacuum, washed with EtOAc and/or triturated in Et₂O.

### 1.1.4.3. General procedure S3

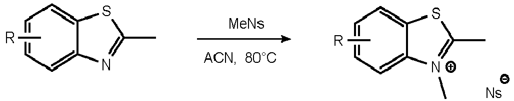

To a solution of the selected 2-methylbenzothiazole (0.63 mmol) in MeCN (2.00 mL) was added methyl nosylate (1.26 mmol). The mixture was stirred overnight at 80°C. The precipitate was filtered under vacuum, washed with EtOAc and/or triturated in Et₂O.

### 1.1.4.4. General procedure S4

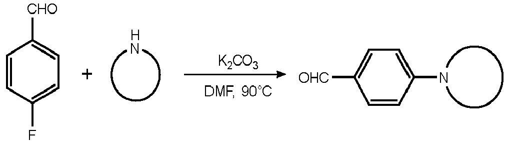

A mixture of 4-fluorobenzaldehyde (46.6 mmol) and the selected secondary amine (39.6 mmol) was diluted with DMF (25.0 mL). K₂CO₃ (58.3 mmol) was added, and the reaction was stirred overnight at 90°C. The mixture was diluted with water and extracted with EtOAc. The organic phase was dried over MgSO₄, evaporated under reduced pressure, and purified by flash chromatography (Hept/EtOAc).

### 1.1.5. Synthesis of the 2-methylbenzothiazole derivative, 2,3-dimethylbenzothiazolium salt derivative, 4-aminobenzaldehyde derivative or 4-aminocinnamaldehyde derivative to be used in General procedures A or B

### 2,4-dimethylbenzo[d]thiazole (21a)

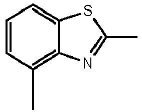

The synthesis was carried out according to general procedure S1, additionally adding ethylene glycol (0.5 mL/mmol) to the mixture (393 mg, 33%). R*_{f}*: 0.41 (Hept/EtOAc 4:1). ¹H NMR (400 MHz, DMSO-*d₆*) δ 7.82 (pd, J = 3.6, 0.6 Hz, 1H), 7.28 (s, 1H), 7.27 - 7.25 (m, 1H), 2.80 (s, 3H), 2.63 (s, 3H). ¹³C NMR (101 MHz, DMSO-*d₆*) δ 165.6, 152.2, 134.9, 131.4, 126.4, 124.6, 119.2, 19.8, 18.1.

### 2,3,4-trimethylbenzo[d]thiazol-3-ium 4-nitrobenzenesulfonate (21b)

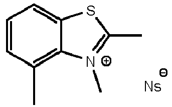

The synthesis was carried out according to general procedure S3 (248 mg, 67%). R*_{f}*: 0.29 (DCM/MeOH 9:1). ¹H NMR (400 MHz, DMSO-*d₆*) δ 8.30 - 8.22 (m, 1H), 8.19 (d, J = 8.4 Hz, 2H), 7.82 (d, J = 8.3 Hz, 2H), 7.64 (d, J = 2.1 Hz, 1H), 7.62 (s, 1H), 4.36 (s, 3H), 3.13 (s, 3H), 2.93 (s, 3H). ¹³C NMR (151 MHz, DMSO-*d₆*) δ 176.6, 154.2, 147.3, 140.6, 132.8, 129.5, 128.3, 127.7, 126.9, 123.4, 122.4, 39.9, 20.4, 17.6.

### 4-methoxy-2-methylbenzo[d]thiazole (22a)

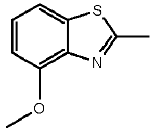

The synthesis was carried out according to general procedure S1 (223 mg, 20%). R*_{f}*: 0.09 (Hept/EtOAc 4:1). ¹H NMR (400 MHz, DMSO-*d₆*) δ 7.55 (dd, *J*= 8.0, 1.0 Hz, 1H), 7.32 (t, *J* = 8.0 Hz, 1H), 7.01 (dd, *J* = 8.0, 1.0 Hz, 1H), 3.93 (s, 3H), 2.77 (s, 3H). ¹³C NMR (101 MHz, DMSO-*d*₆) δ 164.7, 152.6, 142.8, 136.7, 125.7, 113.6, 107.4, 55.7, 19.7.

### 4-methoxy-2,3-dimethylbenzo[d]thiazol-3-ium iodide (22b)

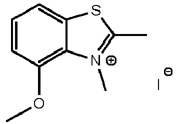

The synthesis was carried out according to general procedure S2 (35 mg, 20%). R*_{f}*: 0.34 (DCM/MeOH 9:1). ¹H NMR (600 MHz, DMSO-*d*₆) δ 7.93 (dd, *J* = 8.2, 0.9 Hz, 1H), 7.70 (t, *J* = 8.2 Hz, 1H), 7.45 (dd, *J* = 8.2, 0.9 Hz, 1H), 4.35 (s, 3H), 4.05 (s, 3H), 3.09 (s, 3H). ¹³C NMR (151 MHz, DMSO-*d*₆) δ 176.4, 150.4, 131.5, 131.1, 129.5, 116.4, 112.1, 57.5, 40.6, 17.6.

### 4-fluoro-2-methylbenzo[d]thiazole (23a)

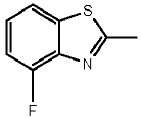

The synthesis was carried out according to general procedure S1 (431 mg, 36%). R*_{f}*: 0.35 (Hept/EtOAc4:1). ¹H NMR (400 MHz, DMSO-*d*₆) δ 7.86 (dd, *J* = 8.1, 1.1 Hz, 1H), 7.41 (td, *J* = 8.1, 4.9 Hz, 1H), 7.31 (ddd, *J=* 11.0, 8.1, 1.1 Hz, 1H), 2.82 (s, 3H). ¹³C NMR (101 MHz, DMSO-*d*₆) δ 168.2, 154.4 (d, J = 252.9 Hz), 141.2 (d, J = 13.3 Hz), 138.1 (d, J = 3.8 Hz), 125.7 (d, J = 7.1 Hz), 118.1 (d, J = 4.1 Hz), 111.7 (d, J = 17.9 Hz), 19.7. ¹⁹F NMR (376 MHz, DMSO-*d*₆) δ -123.34.

### 4-fluoro-2,3-dimethylbenzo[d]thiazol-3-ium 4-nitrobenzenesulfonate (23b)

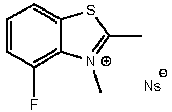

The synthesis was carried out according to general procedure S3 (188 mg, 78%). R*_{f}*: 0.32 (DCM/MeOH 9:1). ¹H NMR (400 MHz, DMSO-*d*₆) δ 8.28 - 8.20 (m, 1H), 8.18 (d, *J* = 8.4 Hz, 2H), 7.86 - 7.75 (m, 4H), 4.29 (d, *J* = 2.7 Hz, 3H), 3.16 (s, 3H). ¹³C NMR (101 MHz, DMSO-*d*₆) δ 178.6, 154.3, 150.8 (d, J = 253.2 Hz), 147.2, 131.3, 130.3 (d, J = 10.7 Hz), 129.1 (d, J = 7.5 Hz), 126.9, 123.3, 120.8 (d, J = 4.6 Hz), 116.0 (d, J = 19.1 Hz), 39.2, 17.0. ¹⁹F NMR (376 MHz, DMSO-*d*₆) δ -125.33.

### 2,3,5-trimethylbenzo[d]thiazol-3-ium iodide (24b)

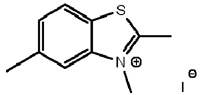

The synthesis was carried out according to general procedure S2 from the commercially available **24a** (460 mg, 61%). R*_{f}*: 0.32 (DCM/MeOH 9:1). ¹H NMR (400 MHz, DMSO-*d*₆) δ 8.29 (d, *J* = 8.4 Hz, 1H), 8.14 (d, *J=* 1.5 Hz, 1H), 7.64 (dd, J = 8.4, 1.5 Hz, 1H), 4.17 (s, 3H), 3.15 (s, 3H), 2.57 (s, 3H). ¹³C NMR (101 MHz, DMSO-*d*₆) δ 176.8, 141.8, 139.8, 129.5, 125.8, 123.9, 116.4, 36.0, 21.1, 17.0.

### 5-methoxy-2-methylbenzo[d]thiazole (25a)

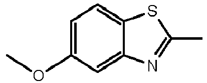

To a solution of 2-methyl-5-benzothiazolol (500 mg, 3.03 mmol) in DMF (8.50 mL) was added K₂CO₃ (836 mg, 6.05 mmol). The mixture was stirred at room temperature for 10 min. Methyl iodide (283 µL, 4.54 mmol) was added dropwise and the reaction was stirred at room temperature for 1h. The mixture was diluted with water and extracted with EtOAc. The organic phase was dried over MgSO₄, evaporated under reduced pressure, and used without any further purification (477 mg, 88%). R*_{f}*: 0.16 (Hept/EtOAc 4:1). ¹H NMR (400 MHz, DMSO-*d*₆) δ 7.87 (d, *J* = 8.8 Hz, 1H), 7.45 (d, *J* = 2.5 Hz, 1H), 7.02 (dd, *J* = 8.8, 2.5 Hz, 1H), 3.83 (s, 3H), 2.76 (s, 3H). ¹³C NMR (101 MHz, DMSO-*d*₆) δ 168.0, 158.4, 154.3, 126.8, 122.2, 114.1, 105.1, 55.4, 19.8.

### 5-methoxy-2,3-dimethylbenzo[d]thiazol-3-ium iodide (25b)

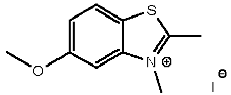

The synthesis was carried out according to general procedure S2 (204 mg, 54%). R*_{f}*: 0.40 (DCM/MeOH 9:1). ¹H NMR (400 MHz, DMSO-*d*₆) δ 8.29 (d, *J* = 9.0 Hz, 1H), 7.78 (d, *J* = 2.4 Hz, 1H), 7.42 (dd, *J* = 9.0, 2.4 Hz, 1H), 4.17 (s, 3H), 3.97 (s, 3H), 3.13 (s, 3H). ¹³C NMR (101 MHz, DMSO-*d*₆) δ 177.0, 160.6, 143.1, 125.1, 120.4, 117.7, 100.0, 56.4, 36.2, 17.1.

### 5-fluoro-2,3-dimethylbenzo[d]thiazol-3-ium iodide (26b)

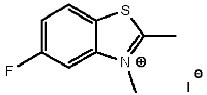

The synthesis was carried out according to general procedure S2 from commercially available **26a** (168 mg, 23%). R*_{f}*: 0.32 (DCM/MeOH 9:1). ¹H NMR (400 MHz, DMSO-*d*₆) δ 8.48 (dd, *J* = 9.1, 5.1 Hz, 1H), 8.34 (dd, *J* = 9.5, 2.5 Hz, 1H), 7.74 (td, *J* = 9.0, 2.4 Hz, 1H), 4.17 (s, 3H), 3.17 (s, 3H). ¹³C NMR (101 MHz, DMSO-*d*₆) δ 179.5, 162.3 (d, J = 246.7 Hz), 142.7 (d, J = 12.8 Hz), 126.5 (d, J = 10.1 Hz), 124.7 (d, J = 2.1 Hz), 116.8 (d, J = 25.0 Hz), 104.2 (d, J = 28.8 Hz), 36.4, 17.3. ¹⁹F NMR (376 MHz, DMSO-*d*₆) δ -110.17.

### 2,6-dimethylbenzo[d]thiazole (27a)

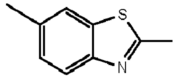

The synthesis was carried out according to general procedure S1 (1.50 g, 60%). R*_{f}*: 0.37 (Hept/EtOAc 3:1). ¹H NMR (400 MHz, DMSO-*d*₆) δ 7.80 (p, *J* = 0.8 Hz, 1H), 7.77 (d, *J* = 8.3 Hz, 1H), 7.27 (dd, *J* = 8.2, 1.6 Hz, 1H), 2.76 (s, 3H), 2.42 (s, 3H). ¹³C NMR (101 MHz, DMSO-*d*₆) δ 165.6, 151.1, 135.3, 134.2, 127.3, 121.5, 121.4, 20.9, 19.6.

### 2,3,6-trimethylbenzo[d]thiazol-3-ium iodide (27b)

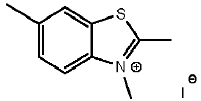

The synthesis was carried out according to general procedure S2 (378 mg, 50%). R*_{f}*: 0.28 (DCM/MeOH 9:1). ¹H NMR (400 MHz, DMSO-*d*₆) δ 8.21 (t, *J* = 1.2 Hz, 1H), 8.18 (d, *J* = 8.7 Hz, 1H), 7.72 (dd, *J* = 8.7, 1.7 Hz, 1H), 4.17 (s, 3H), 3.14 (s, 3H), 2.53 (s, 3H). ¹³C NMR (101 MHz, DMSO-*d*₆) δ 176.0, 139.8, 138.4, 130.5, 128.7, 123.7, 116.3, 36.1, 21.0, 17.0.

### 6-methoxy-2-methylbenzo[d]thiazole (28a)

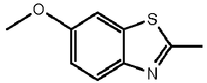

The synthesis was carried out according to general procedure S1 (1.34 g, 61%). R*_{f}*: 0.29 (Hept/EtOAc 3:1). ¹H NMR (400 MHz, DMSO-*d*₆) δ 7.78 (d, *J* = 8.9 Hz, 1H), 7.59 (d, *J* = 2.6 Hz, 1H), 7.05 (dd, *J* = 8.9, 2.6 Hz, 1H), 3.81 (s, 3H), 2.73 (s, 3H). ¹³C NMR (101 MHz, DMSO-*d*₆) δ 164.0, 156.9, 147.3, 136.5, 122.3, 114.9, 104.7, 55.6, 19.5.

### 6-methoxy-2,3-dimethylbenzo[d]thiazol-3-ium iodide (28b)

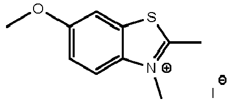

The synthesis was carried out according to general procedure S2 (410 mg, 57%). R*_{f}*: 0.15 (DCM/MeOH 9:1). ¹H NMR (400 MHz, DMSO-*d*₆) δ 8.18 (d, *J* = 9.3 Hz, 1H), 8.00 (d, *J* = 2.6 Hz, 1H), 7.47 (dd, *J* = 9.3, 2.6 Hz, 1H), 4.16 (s, 3H), 3.90 (s, 3H), 3.11 (s, 3H). ¹³C NMR (101 MHz, DMSO-*d*₆) δ 174.3, 159.0, 135.8, 130.4, 118.4, 117.6, 106.6, 56.2, 36.2, 16.9.

### 6-fluoro-2-methylbenzo[d]thiazole (29a)

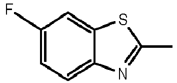

2-amino-5-fluorobenzenethiol (300 mg, 2.09 mmol) and 2,4-pentanedione (323 µL, 3.14 mmol) were dissolved in MeCN (1 mL) and p-toluenesulfonic acid (18.0 mg, 0.10 mmol) was added. The mixture was stirred overnight at room temperature. It was diluted with water and extracted with EtOAc. The organic phase was dried over MgSO₄, evaporated under reduced pressure, and purified by flash chromatography (Hept/EtOAc 0% B to 20% B) to afford the product (257 mg, 73%). R*_{f}*: 0.29 (Hept/EtOAc 5:1). ¹H NMR (400 MHz, DMSO-*d*₆) δ 7.94 (dd, *J* = 8.2, 2.1 Hz, 1H), 7.91 (dd, *J* = 8.3, 4.3 Hz, 1H), 7.33 (td, *J* = 9.1, 2.7 Hz, 1H), 2.78 (s, 3H). ¹³C NMR (101 MHz, DMSO-*d*₆) δ 167.1 (d, J = 3.2 Hz), 159.4 (d, J = 241.7 Hz), 149.7, 136.4 (d, J = 11.7 Hz), 123.0 (d, J = 9.5 Hz), 114.3 (d, J = 24.8 Hz), 108.3 (d, J = 27.1 Hz), 19.7. ¹⁹F NMR (376 MHz, DMSO-*d*₆) δ -117.24.

### 6-fluoro-2,3-dimethylbenzo[d]thiazol-3-ium iodide (29b)

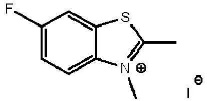

The synthesis was carried out according to general procedure S2 (73.6 mg, 24%). R*_{f}*: 0.17 (DCM/MeOH 9:1). ¹H NMR (600 MHz, DMSO-*d*₆) δ 8.36 (dd, *J* = 9.1, 4.3 Hz, 1H), 8.32 (dd, *J=* 8.3, 2.7 Hz, 1H), 7.84 (td, *J* = 9.1, 2.7 Hz, 1H), 4.19 (s, 3H), 3.15 (s, 3H). ¹³C NMR (151 MHz, DMSO-*d*₆) δ 177.8 (d, J = 2.5 Hz), 160.7 (d, J = 247.2 Hz), 138.5, 130.3 (d, J = 12.2 Hz), 118.8 (d, J = 9.6 Hz), 117.9 (d, J = 25.7 Hz), 110.9 (d, J = 28.6 Hz), 36.5, 17.2. ¹⁹F NMR (376 MHz, DMSO-*d*₆) δ -111.66.

### 1-methyl-3-nitro-2-thiocyanatobenzene (30.1)

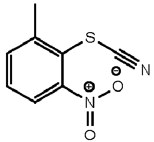

To a suspension of 2-methyl-6-nitroaniline (1.20 g, 7.89 mmol) in water (27.0 mL) cooled to 0°C, HCl 37% (12.0 mL) was added. Sodium nitrite (653 mg, 9.46 mmol) was dissolved in water (5.00 mL) and added dropwise. After stirring for 5 min at 0°C, a solution of potassium thiocyanate (2.30 g, 23.7 mmol) and iron (III) chloride (640 mg, 3.94 mmol) in water (9.00 mL) was added dropwise. The mixture was stirred at room temperature for 5h. It was diluted with water and extracted with EtOAc. The organic phase was dried over MgSO₄, evaporated, and used without any further purification (quant.). R*_{f}*: 0.54 (Hept/EtOAc 1:1). ¹H NMR (400 MHz, DMSO-*d*₆) δ 7.96 (d, *J* = 7.0 Hz, 1H), 7.82 (d, *J* = 6.9 Hz, 1H), 7.73 (t, *J* = 7.3 Hz, 1H), 2.66 (s, 3H). ¹³C NMR (101 MHz, DMSO-*d*₆) δ 152.9, 144.5, 135.4, 132.1, 123.1, 117.0, 110.5, 21.1.

### 2,7-dimethylbenzo[d]thiazole (30a)

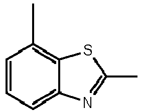

To a solution of 30.1 (2.55 g, 13.1 mmol) in EtOH/H₂O 1:1 v/v (45 mL, 45 mL), Na₂S (2.05 g, 26.3 mmol) was added in portions under argon atmosphere. The mixture was heated at 65°C for 3h and then extracted with EtOAc. The organic phase was dried over MgSO₄, evaporated under reduced pressure, and purified by flash chromatography (DCM/MeOH 0% to 2% B). The collected residue was dissolved in MeCN (1 mL) and 2,4-pentanedione (0.06 mL, 0.60 mmol) and p-toluenesulfonic acid (3.00 mg, 0.02 mmol) were added. The mixture was stirred at room temperature for 3h. It was diluted with water and extracted with EtOAc. The organic phase was dried over MgSO₄, evaporated, and purified by flash chromatography (PE/EtOAc 0% to 20% B) to afford the product (17.0 mg, 1%). R*_{f}*: 0.26 (PE/EtOAc 4:1). ¹H NMR (400 MHz, CDCl₃) δ 7.79 (d, *J* = 8.1 Hz, 1H), 7.36 (t, *J* = 7.7 Hz, 1H), 7.15 (dt, *J* = 7.4, 1.0 Hz, 1H), 2.85 (s, 3H), 2.54 (s, 3H). ¹³C NMR (151 MHz, CDCl₃) δ 166.7, 153.2, 136.2, 131.7, 126.3, 125.1, 119.9, 21.6, 20.3.

### 2,3,7-trimethylbenzo[d]thiazol-3-ium iodide (30b)

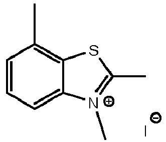

The synthesis was carried out according to general procedure S2 (12.0 mg, 40%). R*_{f}*: 0.35 (DCM/MeOH 9:1). ¹H NMR (600 MHz, DMSO-*d₆*) δ 8.15 (d, *J* = 8.5 Hz, 1H), 7.83 (dd, *J* = 8.5, 7.5 Hz, 1H), 7.66 (dt, *J* = 7.5, 1.0 Hz, 1H), 4.21 (s, 3H), 3.20 (s, 3H), 2.66 (s, 3H). ¹³C NMR (151 MHz, DMSO-*d₆*) δ 176.2, 147.0, 136.7, 133.5, 129.7, 128.5, 114.5, 36.6, 19.3, 17.2.

### 2-methoxy-6-nitroaniline (31.1)

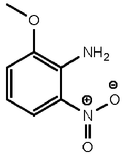

To a solution of 2-amino-3-nitrophenol (4.18 g, 27.1 mmol) in MeCN (47.0 mL) was added K₂CO₃ (3.75 g, 27.1 mmol). Methyl iodide (1.86 mL, 29.8 mmol) was added dropwise, and the mixture was stirred at room temperature for 22h. It was diluted with water and extracted with EtOAc. The organic phase was dried over MgSO₄, evaporated under reduced pressure, and purified by flash chromatography (Hept/EtOAc 0% to 20% B) to afford the product as an orange solid (2.63 g, 58%). R*_{f}*: 0.39 (Hept/EtOAc 2:1). ¹H NMR (400 MHz, CDCl₃) δ 7.73 (dd, J = 8.9, 1.3 Hz, 1H), 6.88 (dd, J = 7.8, 1.3 Hz, 1H), 6.61 (dd, J = 8.9, 7.8 Hz, 1H), 6.42 (s, 2H), 3.92 (s, 3H). ¹³C NMR (101 MHz, CDCl₃) δ 148.3, 137.2, 131.8, 117.5, 114.7, 113.5, 56.4.

### 1-methoxy-3-nitro-2-thiocyanatobenzene (31.2)

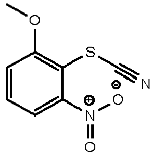

The synthesis was carried out following the same procedure as **30.1** (quant.). R*_{f}*: 0.21 (PE/EtOAc 3:1). ¹H NMR (600 MHz, DMSO-*d₆*) δ 7.82 - 7.70 (m, 2H), 7.64 (dd, J = 8.2, 4.2 Hz, 1H), 4.07 (s, 3H). ¹³C NMR (151 MHz, DMSO-*d₆*) δ 158.8, 132.9, 124.5, 117.7, 117.3, 109.9, 106.6, 57.5.

### 7-methoxy-2-methylbenzo[d]thiazole (31a)

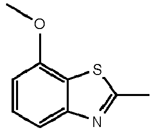

The synthesis was carried out following the same procedure as **30a** (49.0 mg, 2%). R*_{f}*: 0.27 (PE/EtOAc 4:1). ¹H NMR (600 MHz, DMSO-*d₆*) δ 6.79 (t, J = 8.0 Hz, 1H), 6.26 (dd, J = 8.3, 0.9 Hz, 1H), 6.21 (dd, J = 7.8, 0.9 Hz, 1H), 3.70 (s, 3H), 2.11 (s, 3H). ¹³C NMR (151 MHz, DMSO-*d₆*) δ 176.9, 153.8, 142.9, 131.4, 117.0, 109.1, 109.1, 57.0, 36.9, 17.6.

### 7-methoxy-2,3-dimethylbenzo[d]thiazol-3-ium iodide (31b)

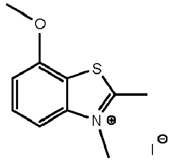

The synthesis was carried out according to general procedure S2 (39.0 mg, 52%). R*_{f}*: 38 (DCM/MeOH 9:1). ¹H NMR (600 MHz, DMSO-*d₆*) δ 7.88 (s, 2H), 7.41 (s, 1H), 4.20 (s, 3H), 4.07 (s, 3H), 3.20 (s, 3H). ¹³C NMR (151 MHz, DMSO-*d₆*) δ 176.9, 153.8, 142.9, 131.4, 117.0, 109.1, 109.1, 57.0, 36.9, 17.6.

### N-(2-bromo-3-fluorophenyl)acetamide (32.1)

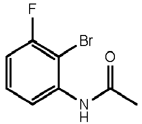

To a solution of 2-bromo-3-fluoroaniline (1.55 g, 8.16 mmol) in acetic anhydride (38.5 mL) was added pyridine (6.5 mL, 81.6 mmol). The mixture was stirred at room temperature for 3h. It was poured into water and extracted with EtOAc. The organic phase was dried over MgSO₄, evaporated and used without any further purification (1.75 g, 93%). R*_{f}*: 0.18 (PE/EtOAc4:1). ¹H NMR (600 MHz, DMSO-*d₆*) δ 9.58 (s, 1H), 7.47 (dt, J = 8.1, 1.3 Hz, 1H), 7.39 (td, J = 8.2, 6.2 Hz, 1H), 7.18 (td, J = 8.5, 1.5 Hz, 1H), 2.10 (s, 3H). ¹³C NMR (151 MHz, DMSO-*d₆*) δ 168.7, 158.8 (d, J = 243.7 Hz), 138.5, 128.7 (d, J = 9.2 Hz), 122.4, 112.7 (d, J = 22.2 Hz), 105.0 (d, J = 24.1 Hz), 23.3.

### N-(2-bromo-3-fluorophenyl)ethanethioamide (32.2)

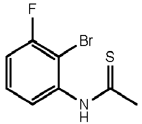

To a solution of 32.1 (1.40 g, 6.03 mmol) in THF (12 mL), Lawesson's reagent (1.71 g, 4.22 mmol) was added under argon atmosphere. The mixture was stirred overnight at room temperature and the precipitate was filtered. The filtrate was collected and further purified by flash chromatography (PE/EtOAc 5% to 80% B) to afford the product as an orange oil (1.04 g, 69%). R*_{f}*: 0.72 (PE/EtOAc 1:1). ¹H NMR (600 MHz, DMSO-*d₆*) δ 11.54 (s, 1H), 7.47 (td, J = 8.2, 6.1 Hz, 1H), 7.35 (td, J = 8.5, 1.4 Hz, 1H), 7.25 (dt, J = 8.0, 1.4 Hz, 1H), 2.62 (s, 3H). ¹³C NMR (151 MHz, DMSO-*d₆*) δ 202.4, 159.1 (d, J = 244.9 Hz), 140.5 (d, J = 1.9 Hz), 129.1 (d, J = 9.3 Hz), 125.7 (d, J = 3.1 Hz), 115.3 (d, J = 22.4 Hz), 108.5 (d, J = 21.4 Hz), 33.2.

### 7-fluoro-2-methylbenzo[d]thiazole (32a)

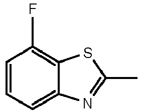

To a solution of 32.2 (810 mg, 3.26 mmol) in 1,4-dioxane (12 mL), Cs₂CO₃ (1.60 g, 4.90 mmol) and Pd(PPh₃)₄ (189 mg, 0.16 mmol) were added under argon atmosphere. The mixture was stirred overnight at 80°C. It was diluted with water and extracted with EtOAc. The organic phase was dried over MgSO₄, evaporated under reduced pressure, and purified by flash chromatography (PE/EtOAc 1% to 15% B) to afford the product (314 mg, 58%). R*_{f}*: 0.53 (PE/EtOAc 3:1). ¹H NMR (600 MHz, DMSO-*d₆*) δ 7.78 (d, J = 8.1 Hz, 1H), 7.51 (td, J = 8.1, 5.7 Hz, 1H), 7.29 (ddd, J = 9.4, 8.2, 1.1 Hz, 1H), 2.83 (s, 3H).¹³C NMR (151 MHz, DMSO-*d₆*) δ 168.0, 156.2 (d, J = 246.6 Hz), 155.9 (d, J = 2.6 Hz), 127.4 (d, J = 7.6 Hz), 121.9 (d, J = 16.7 Hz), 118.3 (d, J = 3.4 Hz), 110.4 (d, J = 18.7 Hz), 19.7.

### 7-fluoro-2,3-dimethylbenzo[d]thiazol-3-ium iodide (32b)

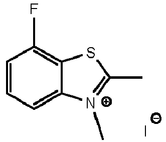

The synthesis was carried out according to general procedure S2 (47.0 mg, 25%). R*_{f}*: 0.19 (DCM/MeOH 9:1). ¹H NMR (600 MHz, DMSO-*d₆*) δ 8.21 (d, J = 8.5 Hz, 1H), 7.97 (q, J = 7.5 Hz, 1H), 7.78 (t, J = 8.9 Hz, 1H), 4.24 (s, 3H), 3.23 (s, 3H). ¹³C NMR (151 MHz, DMSO-*d₆*) δ 178.4, 155.8 (d, J = 250.7 Hz), 143.9 (d, J = 5.6 Hz), 131.5 (d, J = 8.1 Hz), 116.4 (d, J = 23.4 Hz), 114.1 (d, J = 17.8 Hz), 113.6 (d, J = 3.5 Hz), 37.1, 17.6.

### 2-methylbenzo[d]thiazole (33a)

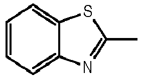

p-Toluenesulfonic acid (402 mg, 2.34 mmol) was added to a mixture of 2-aminobenzenethiol (5.00 mL, 46.7 mmol) and 2,4-pentanedione (7.20 mL, 70.1 mmol) and stirred overnight. The mixture was diluted with water and extracted with EtOAc. The organic phase was dried over MgSO₄, evaporated under reduced pressure, and purified by flash chromatography (Hept/EtOAc 0% to 20% B) to afford the product as an orange oil (5.74 g, 82%). R*_{f}*: 0.23 (Hept/EtOAc 5:1). ¹H NMR (400 MHz, DMSO-*d₆*) δ 8.02 (dt, *J* = 8.0, 1.0 Hz, 1H), 7.91 (dt, *J* = 8.2, 0.9 Hz, 1H), 7.47 (ddd, *J* = 8.2, 7.2, 1.4 Hz, 1H), 7.38 (ddd, *J* = 8.3, 7.3, 1.3 Hz, 1H), 2.79 (s, 3H). ¹³C NMR (101 MHz, DMSO-*d₆*) δ 166.8, 153.0, 135.2, 125.9, 124.7, 121.9, 19.7.

### 2,3-dimethylbenzo[d]thiazol-3-ium iodide (33b)

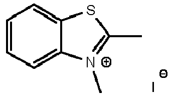

The synthesis was carried out according to general procedure S2 to afford a pink solid (1.21 g, 50%). R*_{f}*: 0.18 (DCM/MeOH 9:1). ¹H NMR (400 MHz, DMSO-*d*₆) δ 8.44 (dd, *J* = 8.1, 1.2 Hz, 1H), 8.29 (d, *J=* 8.4 Hz, 1H), 7.90 (ddd, *J* = 8.5, 7.3, 1.3 Hz, 1H), 7.81 (ddd, *J* = 8.3, 7.2, 1.1 Hz, 1H), 4.21 (s, 3H), 3.18 (s, 3H). ¹³C NMR (101 MHz, DMSO-*d₆*) δ 177.2, 141.6, 129.2, 128.7, 128.0, 124.5, 116.7, 36.2, 17.1.

### 4-(piperidin-1-yl)benzaldehyde (34)

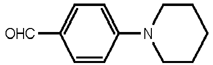

The synthesis was carried out according to general procedure S4 to afford a white solid (6.45 g, 86%). R*_{f}*: 0.18 (Hept/EtOAc 5:1). ¹H NMR (400 MHz, DMSO-*d*₆) δ 9.68 (s, 1H), 7.67 (dt, J = 9.8, 2.9 Hz, 2H), 7.00 (dt, J = 9.0, 2.7 Hz, 2H), 3.41 (dd, J = 6.6, 3.9 Hz, 4H), 1.64-1.52 (m, 6H). ¹³C NMR (101 MHz, DMSO-*d*₆) δ 189.9, 154.6, 131.6, 125.4, 113.0, 47.6, 24.8, 23.9.

### 4-morpholinobenzaldehyde (35)

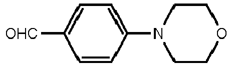

The synthesis was carried out according to general procedure S4 (1.77 g, 58%). R*_{f}*: 0.19 (Hept/EtOAc 3:1). ¹H NMR (600 MHz, DMSO-*d₆*) δ 9.74 (s, 1H), 7.73 (dt, J = 9.0, 2.8 Hz, 2H), 7.06 (dt, J = 8.9, 2.6 Hz, 2H), 3.78 - 3.69 (m, 4H), 3.38 - 3.32 (m, 4H). ¹³C NMR (151 MHz, DMSO-*d₆*) δ 190.4, 154.9, 131.4, 126.7, 113.2, 65.8, 46.6.

### 4-thiomorpholinobenzaldehyde (36)

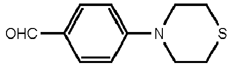

The synthesis was carried out according to general procedure S4 (73.5 mg, 11%). R*_{f}*: 0.22 (Hept/EtOAc 5:1). ¹H NMR (600 MHz, DMSO-*d₆*) δ 9.70 (s, 1H), 7.71 (dd, J = 9.0, 2.8 Hz, 2H), 7.13 - 6.89 (m, 2H), 3.93 - 3.69 (m, 4H), 2.75 - 2.55 (m, 4H). ¹³C NMR (151 MHz, DMSO-*d₆*) δ 190.1, 153.3, 131.7, 125.8, 113.4, 49.4, 24.8.

### 4-(pyrrolidin-1-yl)benzaldehyde (37)

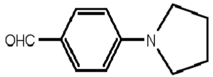

The synthesis was carried out according to general procedure S4 (381 mg, 46%). R*_{f}*: 0.37 (Hept/EtOAc 3:1). ¹H NMR (400 MHz, DMSO-*d₆*) δ 9.64 (s, 1H), 7.67 (dt, J = 8.8, 2.7 Hz, 2H), 6.63 (dt, J = 8.8, 2.6 Hz, 2H), 3.38 - 3.30 (m, 4H), 1.98 - 1.93 (m, 4H).¹³C 1NMR (101 MHz, DMSO-*d₆*) δ 189.6, 151.6, 131.7, 124.2, 111.2, 47.3, 24.9.

### 4-(4-fluoropiperidin-1-yl)benzaldehyde (38)

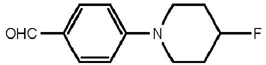

The synthesis was carried out according to general procedure S4 (293 mg, 59%). R*_{f}*: 0.21 (Hept/EtOAc 3:1). ¹H NMR (400 MHz, DMSO-*d₆*) δ 9.71 (s, 1H), 7.70 (dt, J = 8.9, 2.5 Hz, 2H), 7.07 (dt, J = 8.9, 2.5 Hz, 2H), 4.90 (dtt, J = 48.9, 7.1, 3.6 Hz, 1H), 3.71 - 3.55 (m, 2H), 3.41 (ddd, J = 13.5, 7.4, 3.9 Hz, 2H), 1.94 (dddd, J = 24.9, 12.0, 8.1, 3.8 Hz, 2H), 1.84 - 1.62 (m, 2H). ¹³C NMR (101 MHz, DMSO-d6) δ 190.6, 154.5, 132.1, 126.5, 113.8, 88.8 (d, J = 169.5 Hz), 43.7 (d, J = 6.6 Hz), 30.8 (d, J = 19.3 Hz). ¹⁹F NMR (376 MHz, DMSO-*d₆*) δ -177.3.

### 4-(dimethylamino)benzaldehyde (39)

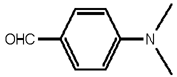

The synthesis was carried out according to general procedure S4 (346 mg, 41%). R*_{f}*: 0.33 (Hept/EtOAc 3:1). ¹H NMR (400 MHz, DMSO-*d₆*) δ 9.67 (s, 1H), 7.68 (dt, J = 9.1, 2.7 Hz, 2H), 6.79 (dt, J = 8.9, 2.8 Hz, 2H), 3.04 (s, 6H). ¹³C NMR (101 MHz, DMSO-*d₆*) δ 190.3, 154.7, 132.0, 125.0, 111.5, 40.1.

### 4-(4-methylpiperazin-1-yl)benzaldehyde (40)

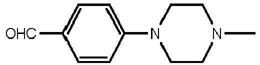

The synthesis was carried out according to general procedure S4 (408 mg, 50%). R*_{f}*: 0.08 (DCM/MeOH 2%). ¹H NMR (400 MHz, DMSO-*d₆*) δ 9.71 (s, 1H), 7.70 (dt, J = 9.0, 2.8 Hz, 2H), 7.04 (dt, J = 9.0, 2.5 Hz, 2H), 3.37 (dd, J = 6.4, 3.8 Hz, 4H), 2.42 (dd, J = 6.6, 3.6 Hz, 4H), 2.21 (s, 3H). ¹³C NMR (101 MHz, DMSO-*d₆*) δ 190.2, 154.7, 131.4, 126.2, 113.3, 54.2, 46.3, 45.7.

### 2-(4-bromophenyl)benzo[d]thiazole (41)

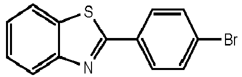

A solution of 2-aminobenzenethiol (750 mg, 5.99 mmol) and 4-bromobenzaldehyde (1.11 g, 5.99 mmol) in DMSO (6.00 mL) was heated at 140°C for 75 min. The mixture was diluted with water and extracted with EtOAc. The organic phase was dried over MgSO₄, evaporated under reduced pressure, and recrystallized from Et₂O to afford the product as a white solid (569 mg, 33%). R*_{f}*: 0.70 (PE/EtOAc 5:1). ¹H NMR (600 MHz, DMSO-*d₆*) δ 8.16 (dt, J = 8.2, 0.8 Hz, 1H), 8.07 (dt, J = 8.0, 0.8 Hz, 1H), 8.03 (dt, J = 8.5, 2.5 Hz, 2H), 7.77 (dt, J = 8.5, 2.5 Hz, 2H), 7.56 (ddd, J = 8.2, 7.2, 1.3 Hz, 1H), 7.48 (ddd, J = 8.2, 7.2, 1.2 Hz, 1H). ¹³C NMR (151 MHz, DMSO-*d₆*) δ 166.1, 153.5, 134.5, 132.4, 132.0, 129.0, 126.8, 125.8, 124.9, 123.0, 122.4.

### 3-(4-(piperidin-1-yl)phenyl)acrylaldehyde (42)

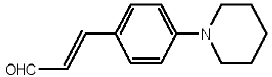

A solution of 34 (800 mg, 4.23 mmol) in H₂SO₄ 96% (3 mL) was cooled to 0°C and acetaldehyde (0.71 mL, 12.7 mmol) was added dropwise. The mixture was stirred for 1h at 0°C. It was poured into water, neutralized with NaOH aq 18M and extracted with EtOAc. The organic phase was dried over MgSO₄, evaporated under reduced pressure, and purified by flash chromatography (PE/EtOAc 5% to 10% B) to afford the product (390 mg, 43%). R*_{f}*: 0.51 (PE/EtOAc 3:1). ¹H NMR (600 MHz, DMSO-*d₆*) δ 9.54 (d, J = 7.9 Hz, 1H), 7.59 - 7.52 (m, 3H), 6.95 (dt, J = 8.9, 3.1 Hz, 2H), 6.60 (dd, J = 15.6, 7.9 Hz, 1H), 3.34 (d, J = 5.4 Hz, 4H), 1.60 - 1.56 (m, 6H). ¹³C NMR (151 MHz, DMSO-d6) δ 193.7, 153.9, 152.9, 130.6, 124.0, 122.7, 114.1, 47.9, 24.9, 24.0.

### 3-(4-(pyrrolidin-1-yl)phenyl)acrylaldehyde (50)

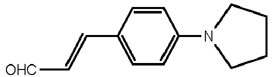

The synthesis was carried out following the same procedure as **42** (513 mg, 64%). R*_{f}*: 0.32 (PE/EtOAc 4:1). ¹H NMR (600 MHz, DMSO-*d₆*) δ 9.51 (d, J = 8.0 Hz, 1H), 7.58 - 7.51 (m, 3H), 6.59 - 6.53 (m, 3H), 3.32 - 3.28 (m, 4H), 2.00 - 1.92 (m, 4H). ¹³C NMR (151 MHz, DMSO-*d₆*) δ 193.3, 154.5, 149.7, 130.8, 122.7, 120.8, 111.7, 47.3, 24.9.

### 1.1.6. Biological evaluation

### 1.1.6.1. Calculation of BBB score and CNS MPO

All required properties (cLogP, cLogD, TPSA, molecular weight, pKa) were calculated via Chemicalize (ChemAxon, Budapest, Hungary) and entered into the calculation Excel tables provided by Gupta, M. et al., The Blood-Brain Barrier (BBB) Score; Journal of Medicinal Chemistry 2019, 62 (21), 9824-9836 and Wager, T. T. et al., Moving beyond Rules: The Development of a Central Nervous System Multiparameter Optimization (CNS MPO) Approach To Enable Alignment of Druglike Properties; ACS Chemical Neuroscience 2010, 1 (6), 435-449. All values are reported in Table 5.

### 1.1.6.2. Preparation of αSYN and Aβ₁₋₄₂ fibrils

The method for Aβ₁₋₄₂ fibrils generation was adapted from Bagchi, D. P. et al., Binding of the radioligand SIL23 to alpha-synuclein fibrils in Parkinson disease brain tissue establishes feasibility and screening approaches for developing a Parkinson disease imaging agent; PLoS One 2013, 8 (2), e55031, and described in Kuebler, L. et al., [11C]MODAG-001-towards a PET tracer targeting α-synuclein aggregates; European Journal of Nuclear Medicine and Molecular Imaging 2020, 48, 1759-1772. Synthetic lyophilized human Aβ₁₋₄₂ peptide (5 mg) with >90 % purity (EMC Microcollections, Tuebingen, Germany) was dissolved in DMSO (221.5 µL), followed by the addition of deionized water (4.1 mL) and 1 M Tris-HCl (111 µL, pH 7.6) to reach a final monomeric concentration of 250 µM. Aggregation was induced by incubation in Eppendorf Thermomixer at 37°C with shaking at 800 rpm for 72 hours. The resulted fibrils were sonicated for 3 min in a water bath (Elmasonic S 60 H, Elma Schmidbauer GmbH, Singen, Germany). The final products were aliquoted, frozen on dry ice, and stored at -80°C until use.

### 1.1.6.3. Fibril binding assays

The K*_{d}* values of [³H]PiB and [³H]MODAG-001 were determined via saturation binding assays on human recombinant αSYN (180 nM for [³H]PiB, 50 nM for [³H]MODAG-001) and synthetic human Aβ₁₋₄₂ fibrils (2 µM for [³H]PiB, 1 µM for [³H]MODAG-001) diluted in phosphate-buffered saline (PBS; Gibco DPBS, no calcium, no magnesium, Thermo Fisher Scientific, Waltham, MA, USA). The fibrils were incubated in 96-well micro test low-binding plates (Ratiolab GmbH, Dreieich, Germany) with increasing concentrations of [³H]PiB (up to 56 nM) and [³H]MODAG-001 (up to 36 nM) in 30 mM Tris-HCl, 0.1% bovine serum albumin, 0.05% Tween20 in a total volume of 200 µL/well. The tracers were incubated with the correspondent non-radioactive compound (1.5 µM PiB or 0.5 µM MODAG-001). The stock solution of PiB and MODAG-001 were prepared by dissolving the compounds in DMSO to 1 mM, which yielded DMSO concentration of ≤0.15% in the final assay.

The binding affinity (K*ᵢ* values) of the newly developed 2-styrylbenzothiazoles were determined via competition binding assays against [³H]PiB and [³H]MODAG-001. The test compounds were dissolved in DMSO to a stock concentration of 1 mM, which resulted in ≤1% DMSO concentration in the final assay. Increasing concentrations of the test compounds (0.6 nM - 10 µM) competed against 6 nM [³H]PiB and 1 nM [³H]MODAG-001. Concentrations of αSYN and Aβ₁₋₄₂ fibrils were as described above.

Plates were incubated on a shaker (MaxQ 6000, Thermo Fisher Scientific Inc., Marietta, OH, USA) at 50 rpm for 2 hours at 37°C, covered by removable sealing tapes (PerkinElmer, Waltham, MA, USA). Vacuum filtration and read-out were performed as disclosed in Kuebler, L. et al., [11C]MODAG-001-towards a PET tracer targeting α-synuclein aggregates; European Journal of Nuclear Medicine and Molecular Imaging 2020, 48, 1759-1772. Radioactivity was plotted against increasing concentrations of [³H]PiB, [³H]MODAG-001, or the non-radioactive test compounds. Data points were fitted using non-linear regression analysis in GraphPad Prism (GraphPad Software, Inc., Version 8.4.0, La Jolla, USA).

### 1.1.6.4. Autoradiography and immunohistochemistry

Post-mortem human brain slices (10 µm thickness) were obtained from and the subject cases were analyzed by the Neurobiobank München (NBM, Munich, Germany), where tissues were collected on the basis of written informed consent according to the guidelines of the ethics committee of the Ludwig-Maximilians-University Munich, Germany (# 345-13). The use of brain tissue samples in this study was approved by the ethics committee of the Faculty of Medicine at the University of Tuebingen (Ethics approval number: 813/2018BO2). Table 1 summarizes the information of the subjects from which samples were obtained and used in the experiment.

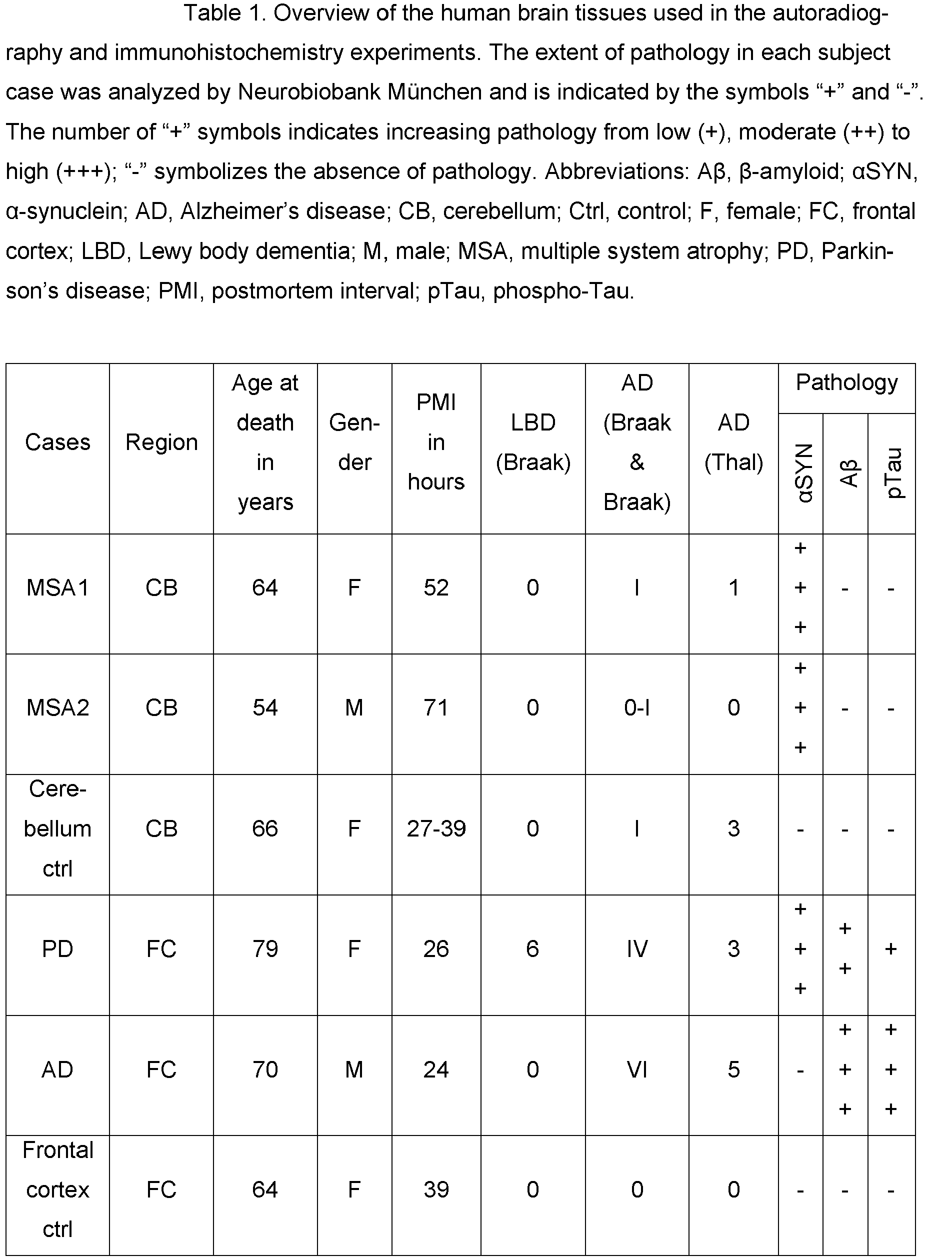

For autoradiography, brain slices were thawed for one hour before preincubation in BSA buffer for 25 minutes at room temperature. To determine total binding (TB), brain slices were incubated with the tracer (10 nM [¹⁸F]PFSB, 10 nM [¹⁸F]MFSB). Incubation of consecutive slices with the correspondent non-radioactive compounds (10 µM PFSB, 10 µM MFSB) was performed to determine non-specific binding (NSB). The non-radioactive compounds were prepared by dissolving in DMSO to 10 mM, which yielded DMSO concentration of ≤0.1% in the final solution. Incubation was carried out for one hour at room temperature with subsequent washing in cold BSA buffer (three times for ten minutes) followed by three dipping in cold deionized water. After drying under UV lamps, brain slices were exposed to a storage phosphor screen (Molecular Dynamics, Caesarea, Israel) for 18 hours, which was then scanned in a phosphor imager (STORM 840, Molecular dynamics, Sunnyvale, CA, USA).

Quantitative data analysis was performed by drawing four regions of interest (ROIs) in relevant areas of the slice and one ROI next to it for background subtraction (imaged 1.8.0_172, National Institute of Health, Bethesda, Maryland, USA) (Schneider, C. A. et al., NIH Image to imaged: 25 years of image analysis; Nat Methods 2012, 9 (7), 671-5). Specific binding (SB) is obtained by subtracting NSB from TB. The SB disease/SB control ratio was calculated by dividing by the SB in diseased tissues with the respective SB in control tissues.

After autoradiography procedures, brain slices were stored at -20°C. IHC was performed on the same tissue slices which were used for TB in autoradiography. After thawing, the slices were post-fixed in 4.5% paraformaldehyde (PFA, SAV Liquid Production GmbH, Flintsbach am Inn, Germany) for 20 min at room temperature. Following washing steps in PBS (2x 5 min), antigen retrieval was performed. For αSYN pSer129 staining, sodium citrate buffer (10 mM, pH 6, Sigma Aldrich Chemie GmbH, Darmstadt, Germany) was boiled and the brain slices were incubated at room temperature in the boiled buffer for 30 minutes; brain sections to be stained for Aβ were incubated in 97% formic acid for 10 min at room temperature. After washing, quenching was performed for 20 minutes (1 mL quenching solution = 890 µL tris-buffered saline (TBS), 100 µL methanol, and 10 µL 30% hydrogen peroxide). Brain slices were subsequently washed and equilibrated in TBS (2x 5 min) and TBS supplemented with 0.1% Triton-X and 1% BSA (in the following referred to as TBS-X) (1x 5 min). Blocking in TBS supplemented with 0.3% Triton-X and 10% normal goat serum for 60 min at room temperature was performed. Incubation with primary antibody was carried out overnight at 4°C with either mouse anti-phosphorylated αSYN pSer129 monoclonal antibody (1:5000 in TBS-X, clone pSyn#64; 015-25191, FUJIFILM Wako Chemicals Europe GmbH, Neuss, Germany) or mouse anti-β-amyloid 17-24 antibody (1:6000 in TBS-X, clone 4G8, 800708, BioLegend, Amsterdam, The Netherlands).

On the second day, the brain slices were washed with TBS-X (3x 10 min) and incubated with secondary antibody (EnVision+/HRP Dual Link Rabbit/Mouse, K406189-2, Agilent, Waldbronn, Germany) for 30 min at room temperature. After washing in TBS-X (2x 10 min) and TBS (1x 10 min), samples were incubated with 3,3'-diaminobenzidine (1:50, Agilent, Waldbronn, Germany) for 10 min. After washing with distilled water (2x 5 min), the brain slices were incubated in hematoxylin (Merck kGaA, Darmstadt, Germany) for 45 s and then rinsed with running tap water for 10 min. To dehydrate and clear the tissues, samples were washed in 70% EtOH (1 min), 95% EtOH (2x 1 min), 100% EtOH (2x 1 min) and xylene (2x 2 min). The slides were mounted with Eukitt quick-hardening mounting medium (Fluka Analytical, Munich, Germany). The stained tissues were scanned with NanoZoomer 2.0 HT (Hamamatsu Photonics K.K., Hamamatsu, Japan) at 40x magnification.

### 1.1.6.5. In vivo PET/MR imaging

The animal experiments were performed in compliance to the European directives on the protection and use of laboratory animals (Council Directive 2010/63/UE) and the German animal welfare act with approval from the local authorities (Regierungspräsidium Tuebingen, R3/19G). Three healthy wild-type female C57BU6J mice (20.3 ± 0.9 g; 9 weeks old) purchased from Charles River Laboratories (Sulzbach, Germany) were maintained in a vivarium on a 12:12 hour light dark cycle and were kept at a temperature of 22°C with 40-60% humidity and free access to a standard diet and tap water.

The mice (n = 3) were anaesthetized with 1.5% isoflurane evaporated in 100% oxygen at a flow rate of 0.8 L/min. Body temperature of 37°C was maintained using a feedback temperature control unit. PET imaging studies were performed on Inveon dedicated microPET system (Inveon D-PET, Siemens, Knoxville, TN, USA). Five seconds after the start of PET acquisition, mice were injected intravenously with 9.9 ± 0.6 MBq of [¹⁸F]MFSB (Aₘ = 46.2 ± 2.5 GBq/µmol at time of injection). The one-hour dynamic acquisitions were divided into 39 time frames (12×5 s, 6×10 s, 6×30 s, 5×60 s and 10×300 s). A 13-minute transmission measurement with a cobalt-57 point source was performed for attenuation correction. Subsequently, an anatomical magnetic resonance (MR) scan with a 7 Tesla MR scanner (ClinScan, Bruker BioSpin MRI GmbH, Ettlingen, Germany) using a rat whole-body volume coil and the Paravision software (v6.0.1, Bruker, Ettlingen, Germany) using a T2-weighted Turbo-RARE MRI sequence.

The PET image was reconstructed via the OSEM3D/SP-MAP reconstruction algorithm and coregistered to the whole-body MRI scan. Volumes of interest (VOls) were hand-drawn for the relevant organs based on the MR anatomy in PMOD (PMOD Technologies, Faellanden, Switzerland, Version 4.2) and VOls at different regions in the mouse brain were extracted using the atlas provided by PMOD to calculate the respective time-activity curves (TACs). Standardized uptake values (SUVs) were calculated as a ratio of the detected activity with injected activity and weight (SUV = (TAC (kBq/cc) / inj. activity (kBq)) * weight (g)).

### 2. Results

### 2.1. Development and in vitro evaluation of a 2-styrylbenzothiazole-based library

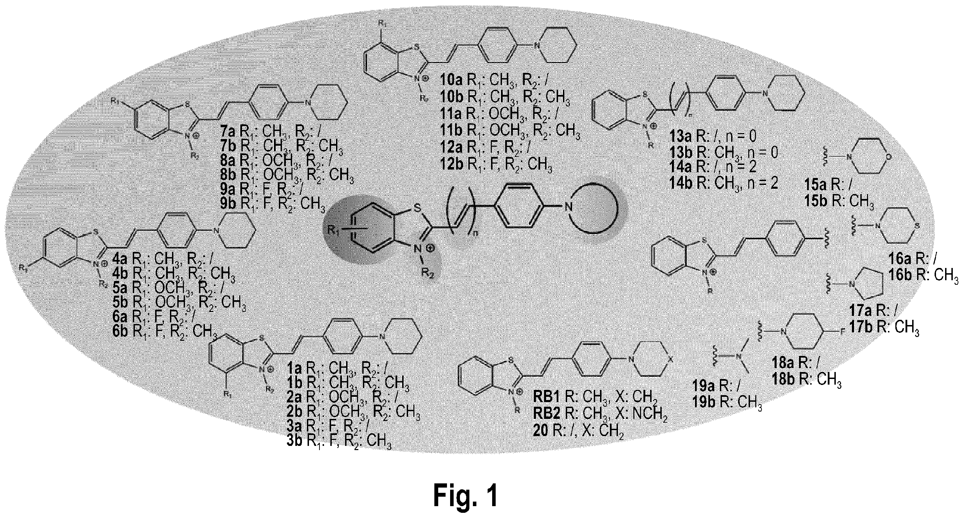

A library of 2-styrylbenzothiazoles was designed and developed in order to produce six classes of compounds (Figure 1) and investigate the result of structural modification on the affinity to αSYN fibrils and to end up with a lead compound. All compounds are fluorescent. Methyl-, methoxy- and fluoro-substitutions were applied at different positions on the benzothiazole ring (compounds 1a-12b). The length of the conjugated system was modified by directly connecting the phenyl-moiety with the benzothiazole or including two additional carbon atoms (compounds 13a-14b). The impact of anilinic nitrogen substitution on binding affinity was investigated, too (compounds 15a-19b). Furthermore, the effect of removal of the N-methylation was investigated by synthesizing non-N-methylated analogs for each structural modification.

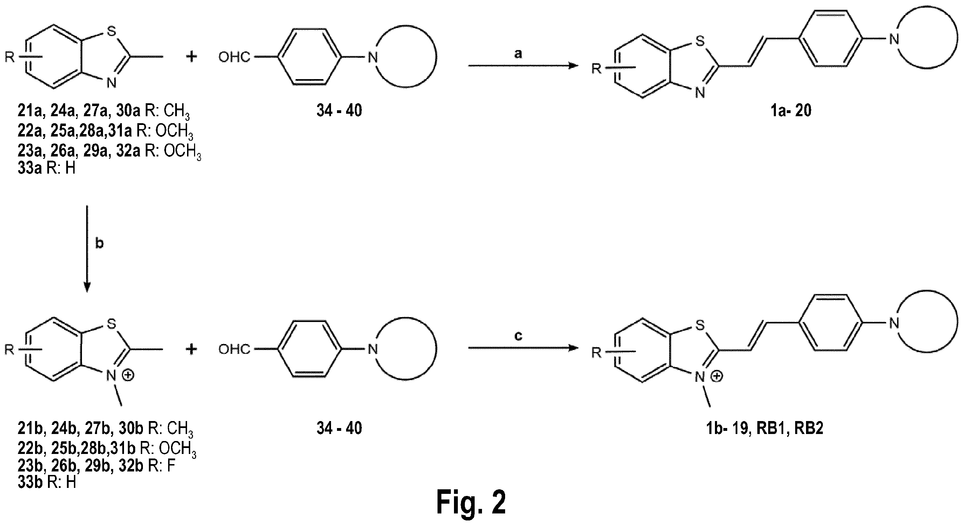

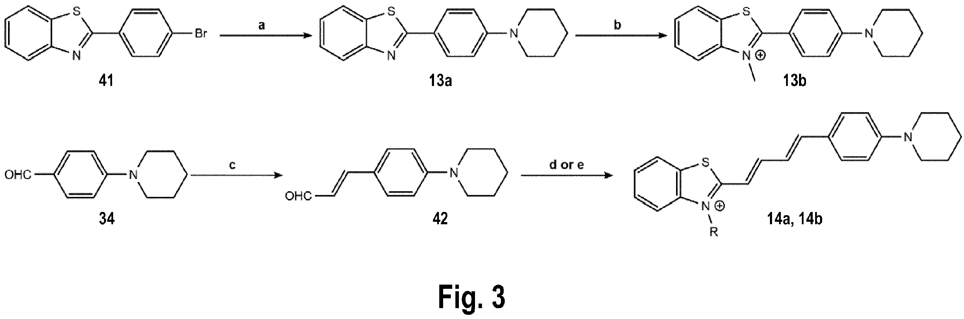

The diversely substituted benzothiazoles (compounds 21a-33a) were produced via dedicated synthetic pathways depending on the availability of starting materials. All benzothiazoles were N-methylated by reacting them with methyl iodide or methyl nosylate, depending on their reactivity (compounds 21b-33b, Figure 2). Various 4-amino-benzaldehydes (compounds 34-40) were synthesized by base-activated substitution of 4-fluorobenzaldehyde with different amines. The benzothiazole- and the benzaldehyde-moieties were combined through condensation reactions to produce RB1, RB2, and 35 other analogs (Figure 2). Pd(PPh₃)₄ catalyzed amination of 2-(4-bromophenyl)benzothiazole (41) and subsequent N-methylation afforded the ThT-analogs 13a and 13b. 4-piperidinecinnamaldehyde (42) was synthesized from the correspondent benzaldehyde by reacting with acetaldehyde under strongly acid conditions and it was coupled with 2-methylbenzothiazoles to produce 1,3-butadiene derivatives (compounds 14a and 14b, Figure 3).

The full library was screened for its binding affinity to αSYN fibrils via [³H]PiB competition assays (Table 2, 3) and showed significant fluctuations depending on structural modification. Affinity was considerably improved in some analogs, up to Kᵢ = 14.7 ± 5.1 nM for the 6-methoxy derivative 8b.

**Table 2. Binding affinity (mean Kᵢ ± SEM, two data points) of non-methylated compounds determined by [³H]PiB competition assays and calculated values for BBB score and CNS MPO. (for 9a, 14a, 15a, 17a and 18a are based on three data points for 12a is based on a single data point)**

| 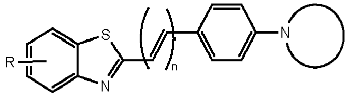 | | | | | | |
|---|---|---|---|---|---|---|
| # | R₁ | n | *N*-substitution | K*ᵢ*(nM) | BBB score | CNS MPO |
| 1a | 4-CH₃ | 1 | C₅H₁₀N | 134.8 ± 63.5 | 4.79 | 3.0 |
| 2a | 4-OCH₃ | 1 | C₅H₁₀N | > 400 | 4.90 | 3.3 |
| 3a | 4-F | 1 | C₅H₁₀N | 148.6 ± 38.0 | 4.78 | 3.0 |
| 4a | 5-CH₃ | 1 | C₅H₁₀N | 160.7 ± 72.7 | 4.79 | 3.0 |
| 5a | 5-OCH₃ | 1 | C₅H₁₀N | 158.1 ± 29.7 | 4.90 | 3.3 |
| 6a | 5-F | 1 | C₅H₁₀N | 146.8 ± 22.3 | 4.78 | 3.0 |
| 7a | 6-CH₃ | 1 | C₅H₁₀N | 223.9 ± 6.1 | 4.79 | 3.0 |
| 8a | 6-OCH₃ | 1 | C₅H₁₀N | 106.5 ± 3.6 | 4.90 | 3.3 |
| 9a (PFSB) | 6-F | 1 | C₅H₁₀N | 25.4 ± 2.3 | 4.78 | 3.0 |
| 10a | 7-CH₃ | 1 | C₅H₁₀N | > 400 | 4.79 | 3.0 |
| 11a | 7-OCH₃ | 1 | C₅H₁₀N | >400 | 4.90 | 3.3 |
| 12a | 7-F | 1 | C₅H₁₀N | 283.3 | 4.78 | 3.0 |
| 13a | H | 0 | C₅H₁₀N | 73.3 ± 61.2 | 4.77 | 3.0 |
| 14a | H | 2 | C₅H₁₀N | 81.9 ± 15.6 | 4.76 | 3.0 |
| 15a | H | 1 | C₄H₈NO | 92.0 ± 30.3 | 4.76 | 3.5 |
| 16a | H | 1 | C₄H₈NS | 138.9 ± 80.2 | 4.62 | 3.0 |
| 17a | H | 1 | C₄H₈N | 73.4 ± 19.0 | 4.81 | 3.0 |
| 18a | H | 1 | C₅H₉FN | 99.8 ± 32.6 | 4.68 | 3.0 |
| 19a | H | 1 | N(CH₃)₂ | > 400 | 4.84 | 3.1 |
| 20 | H | 1 | C₅H₁₀N | 102.0 ± 68.5 | 4.83 | 3.0 |

**Table 3. Binding affinity (mean Kᵢ ± SEM, two data points) of methylated compounds determined by [³H]PiB competition assays.**

| 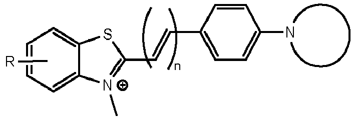 | | | | |
|---|---|---|---|---|
| # | R | n | *N*-substitution | K*ᵢ*(nM) |
| RB1 | H | 1 | C₅H₁₀N | > 400 |
| RB2 | H | 1 | C₅H₁₁N₂ | > 400 |
| 1b | 4-CH₃ | 1 | C₅H₁₀N | 60.0 ± 23.9 |
| 2b | 4-OCH₃ | 1 | C₅H₁₀N | 52.6 ± 18.3 |
| 3b | 4-F | 1 | C₅H₁₀N | > 400 |
| 4b | 5-CH₃ | 1 | C₅H₁₀N | 72.2 ± 12.4 |
| 5b | 5-OCH₃ | 1 | C₅H₁₀N | 95.2 ± 67.7 |
| 6b | 5-F | 1 | C₅H₁₀N | 351.7 ± 83.5 |
| 7b | 6-CH₃ | 1 | C₅H₁₀N | 20.3 ± 3.5 |
| 8b | 6-OCH₃ | 1 | C₅H₁₀N | 14.7 ± 5.1 |
| 9b | 6-F | 1 | C₅H₁₀N | 189.5 ± 145.9 |
| 10b | 7-CH₃ | 1 | C₅H₁₀N | 147.6 ± 83.7 |
| 11b | 7-OCH₃ | 1 | C₅H₁₀N | 205.5 ± 28.2 |
| 12b | 7-F | 1 | C₅H₁₀N | > 400 |
| 13b | H | 0 | C₅H₁₀N | > 400 |
| 14b | H | 2 | C₅H₁₀N | 19.9 ± 3.1 |
| 15b | H | 1 | C₄H₈NO | > 400 |
| 16b | H | 1 | C₄H₈NS | > 400 |
| 17b | H | 1 | C₄H₈N | 101.7 ± 43.0 |
| 18b | H | 1 | C₅H₉FN | > 400 |
| 19b | H | 1 | N(CH₃)₂ | >400 |

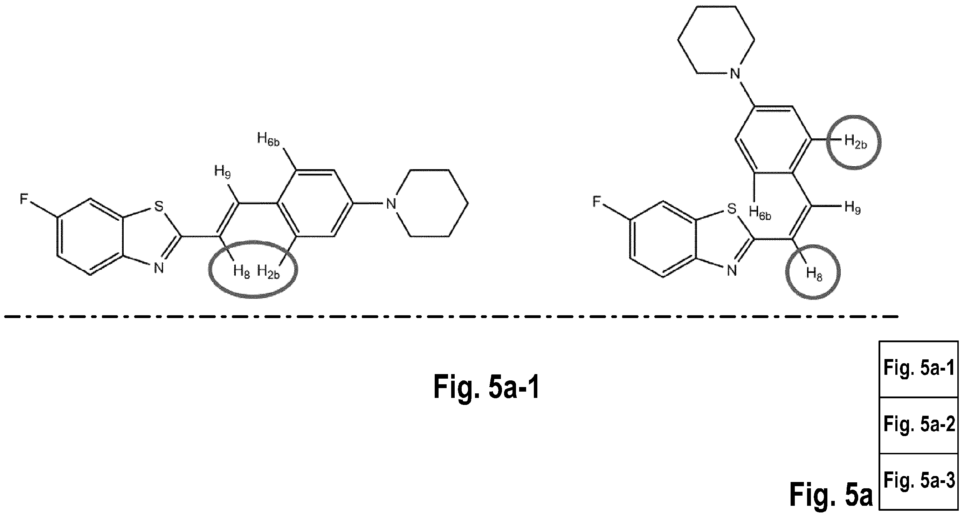

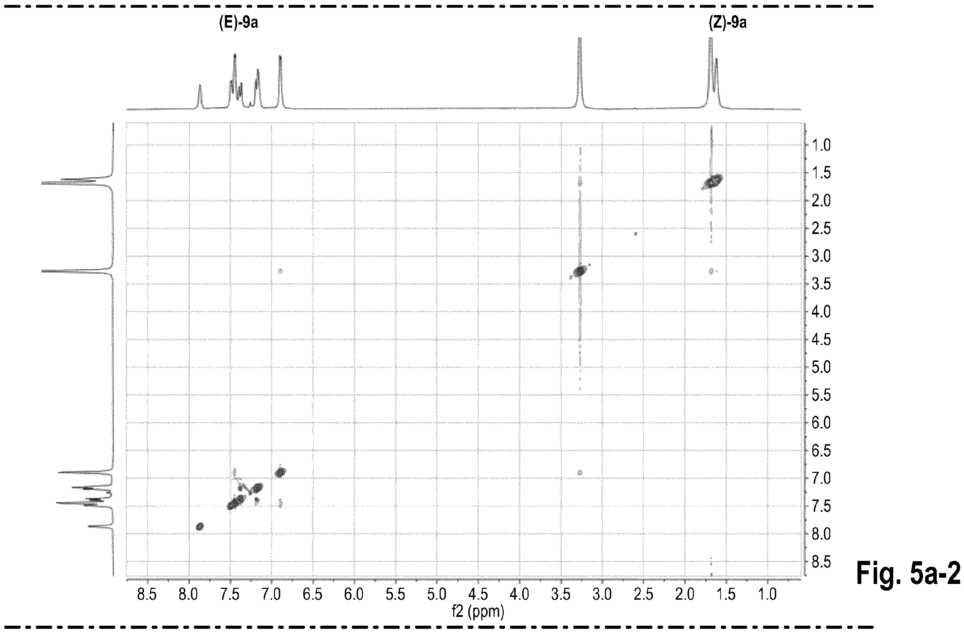

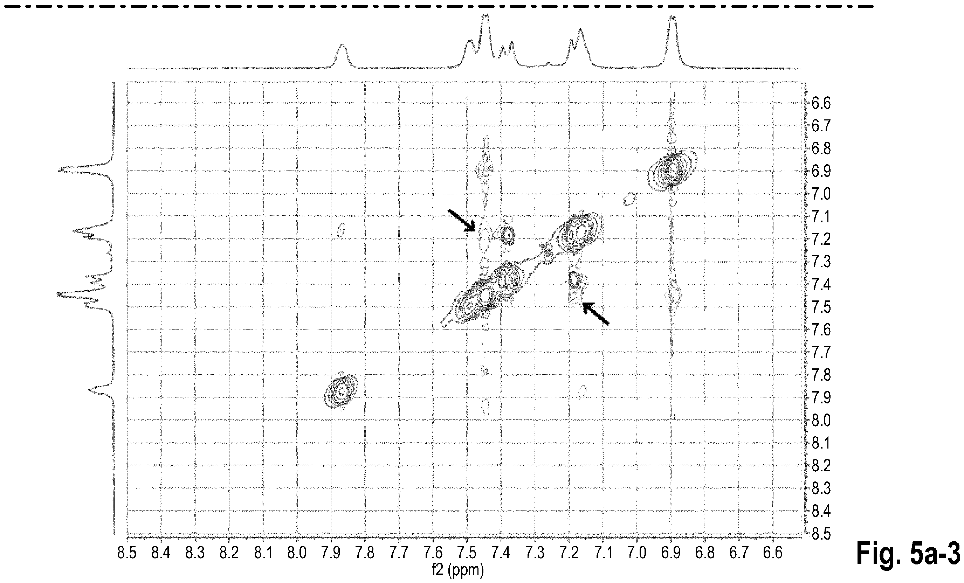

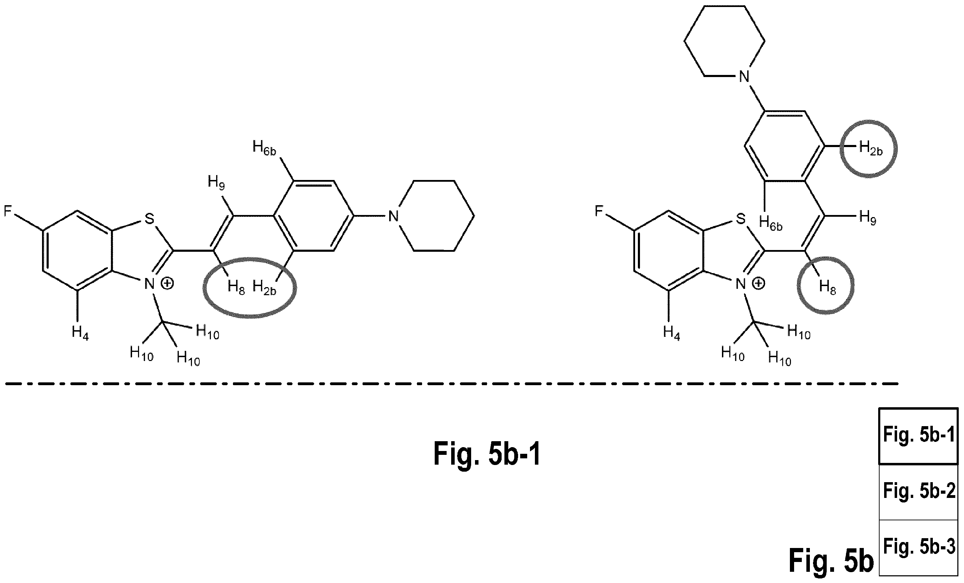

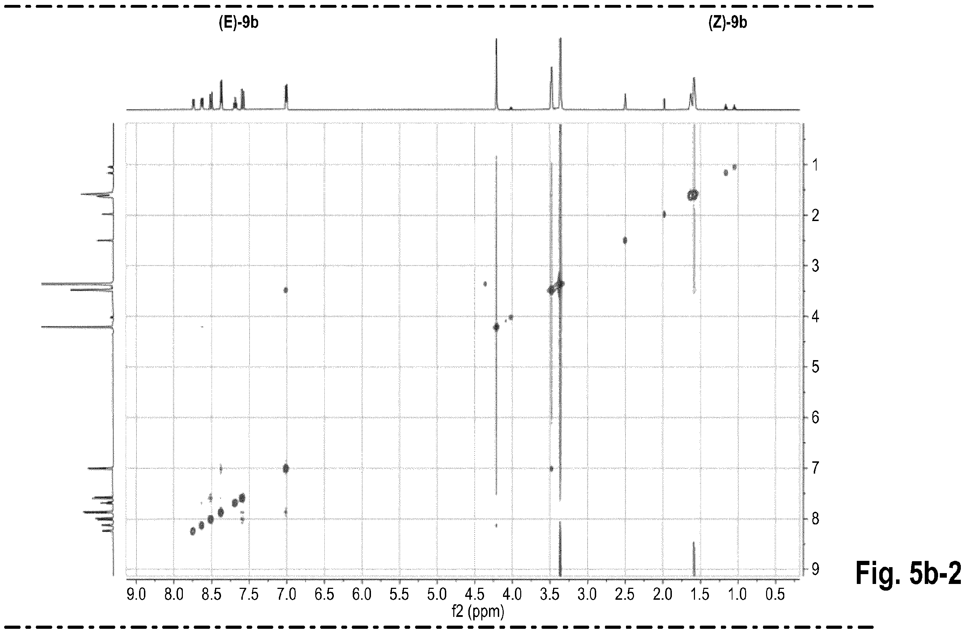

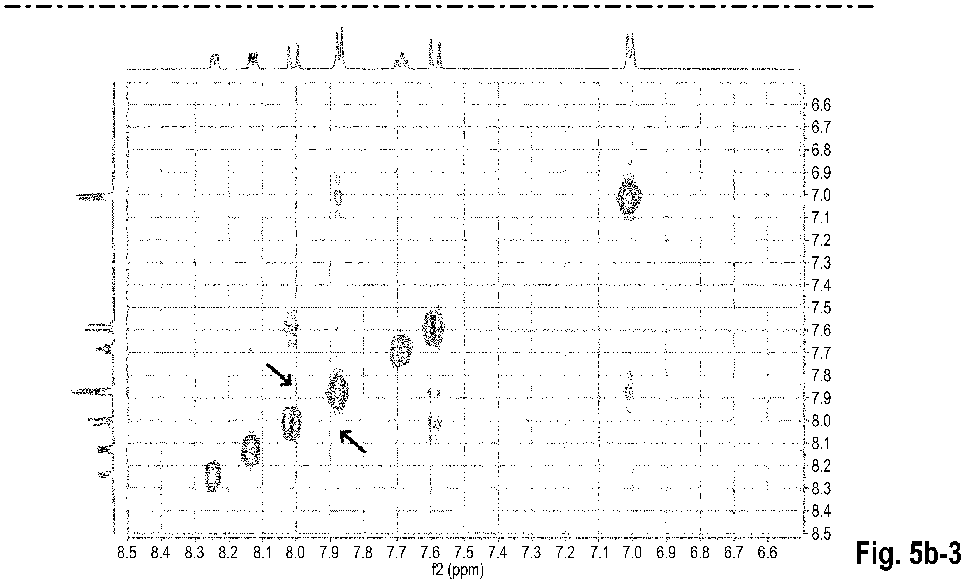

The different structural modifications displayed different impact on neutral and cationic compounds, as can be seen in table 2 and table 3. The different impact of structural modifications on neutral and cationic compounds could be potentially related to the configuration of their double bond. Non-ionic derivatives proved to be a mixture of E and Z isomers, while N-methylated analogs were preferentially in the Z configuration (illustrated by ¹H NOESY NMR, Figure 5).

To cross-match the binding properties with the compounds predicted ability to reach the brain, calculations of BBB score and CNS MPO (Central Nervous System Multiparameter Optimization) were carried out. The calculated values for all non-ionic compounds were in the interval 4.62 - 4.90 for BBB score (range: 0 to 6) and 3.0 - 3.5 for CNS MPO (range: 0 to 5). As both parameters did not significantly differ among the analogs, these predictions were not considered in the selection of a lead compound.

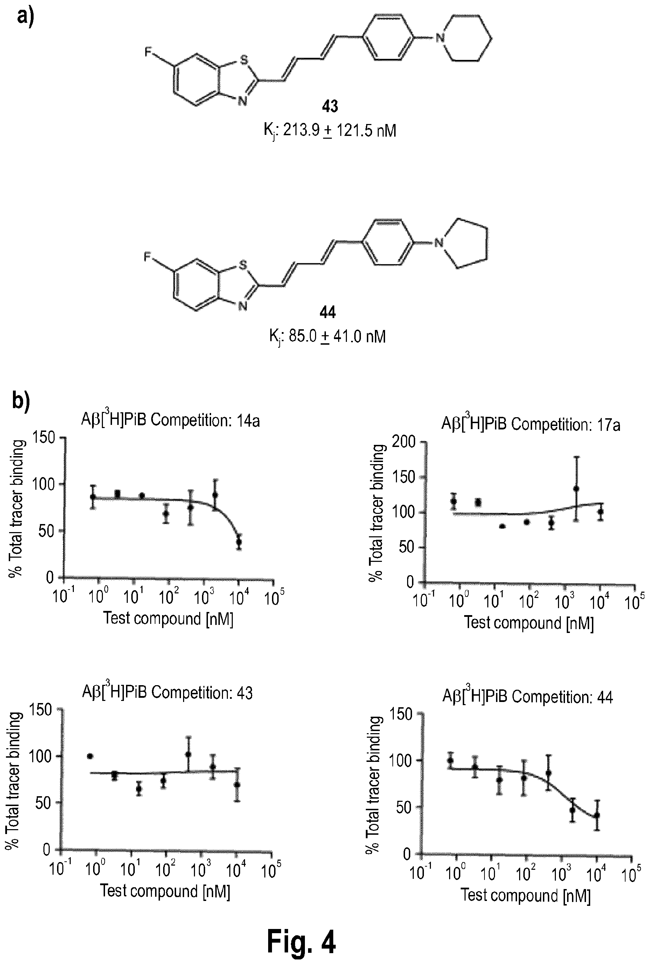

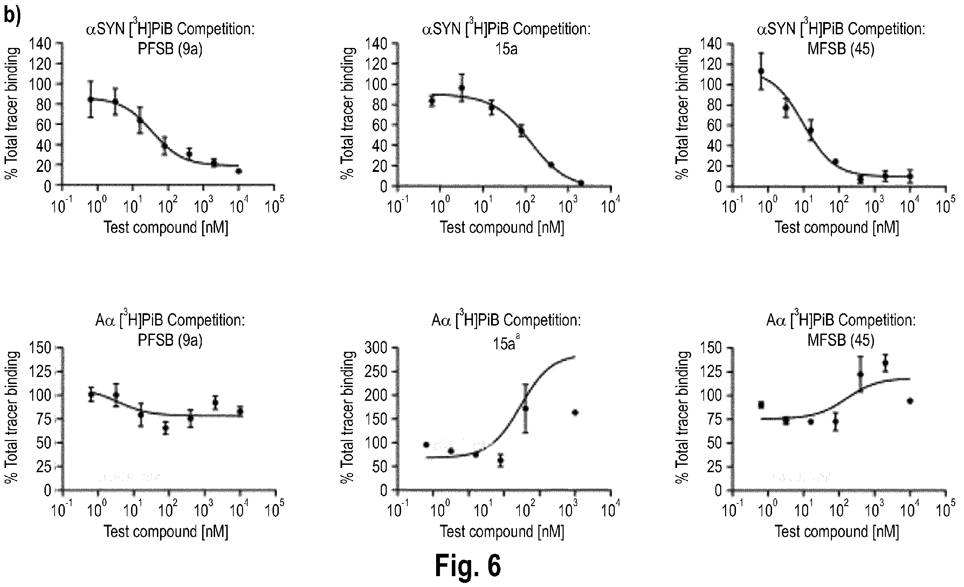

Based on their affinity to αSYN, the most promising compounds were selected for further evaluation and their selectivity was assessed in [³H]PiB competition binding assays to Aβ fibrils. No significant competition with the tracer was detected for any of the tested analogs, suggesting a high selectivity for αSYN over Aβ characterizes the 2-styrylbenzothiazoles in this library (Figure 4b, 6b).

### 2.2. Optimization by combination of promising features

Based on the observed results, some structural features were recognized to favor the binding to αSYN fibrils. With the purpose of further optimization, these components were combined to develop compounds 43 and 44: 6-fluorobenzothiazole was selected due to lower K*ᵢ* values and opportunity for fluorine-18 labeling, a longer π-system (n = 2) was adopted, and N-pyrrolidine substitution was included (Figure 4a). Although the binding to Aβ remained low (Figure 4b), competition binding assays against [³H]PiB showed a decrease in affinity to αSYN fibrils (Kᵢ₋₄₃: 213.9 ± 121.5 nM, K_{*i*-44}: 85.0 ± 41.0 nM), deeming the optimization unsuccessful.

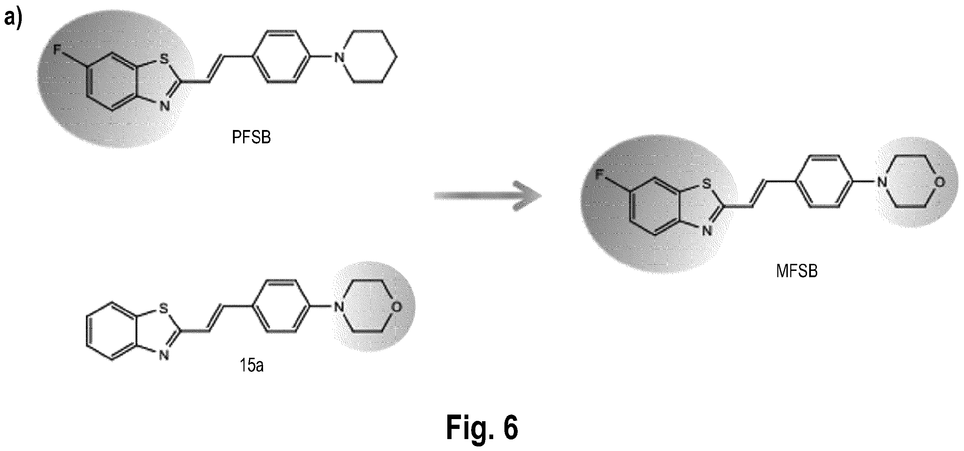

In addition to this attempt to enhance binding affinity, the combination of favorable moieties focused on the improvement of pharmacokinetics. As the cLogP of most compounds within the library was ≥ 5.5 (Table 5), it raised a concern regarding lipophilicity. Thus, a more hydrophilic derivative was designed. The *N*-morpholine compound 15a shows comparable affinity to its *N*-piperidine analog 20 as well as a significantly lower cLogP (4.57, Table 5) and the highest CNS MPO among the evaluated compounds (3.5, Table 5). Thus, as compound 9a (PFSB) was selected from the first set of analogs for radiolabeling and further evaluation, the *N*-morpholine moiety was chosen to be combined with a 6-fluorobenzothiazole to produce MFSB (45, Figure 6a). [³H]PiB competition binding assays proved affinity was not only comparable to PFSB but improved by a 2.5 factor (K*ᵢ*: 10.3 ± 4.7 nM). The selectivity towards αSYN over Aβ remained unchanged (Figure 6b).

**Table 5. Chemical properties required for the calculation of BBB score and CNS MPO for all non-ionic compounds.**

| # | cLogP | TPSA (Å²) | MW (g/mol) | pKa | aromatic rings | heavy atoms | Hydrogen bond acceptors | Hydrogen bond donors | BBB scor e | CNS MP O |
|---|---|---|---|---|---|---|---|---|---|---|
| 1a | 6.15 | 16.13 | 334.48 | 5.30 | 3 | 24 | 2 | 0 | 4.79 | 3.0 |
| 2a | 5.48 | 25.36 | 350.48 | 5.30 | 3 | 25 | 3 | 0 | 4.90 | 3.3 |
| 3a | 5.78 | 16.13 | 338.44 | 5.30 | 3 | 24 | 2 | 0 | 4.78 | 3.0 |
| 4a | 6.15 | 16.13 | 334.48 | 5.31 | 3 | 24 | 2 | 0 | 4.79 | 3.0 |
| 5a | 5.48 | 25.36 | 350.48 | 5.31 | 3 | 25 | 3 | 0 | 4.90 | 3.3 |
| 6a | 5.78 | 16.13 | 338.44 | 5.30 | 3 | 24 | 2 | 0 | 4.78 | 3.0 |
| 7a | 6.15 | 16.13 | 334.48 | 5.30 | 3 | 24 | 2 | 0 | 4.79 | 3.0 |
| 8a | 5.48 | 25.36 | 350.48 | 5.30 | 3 | 25 | 3 | 0 | 4.90 | 3.3 |
| 9a | 5.78 | 16.13 | 338.44 | 5.30 | 3 | 24 | 2 | 0 | 4.78 | 3.0 |
| 10a | 6.15 | 16.13 | 334.48 | 5.31 | 3 | 24 | 2 | 0 | 4.79 | 3.0 |
| 11a | 5.48 | 25.36 | 350.48 | 5.31 | 3 | 25 | 3 | 0 | 4.90 | 3.3 |
| 12a | 5.78 | 16.13 | 338.44 | 5.30 | 3 | 24 | 2 | 0 | 4.78 | 3.0 |
| 13a | 5.10 | 16.13 | 294.42 | 4.36 | 3 | 21 | 2 | 0 | 4.77 | 3.0 |
| 14a | 6.16 | 16.13 | 346.49 | 5.37 | 3 | 25 | 2 | 0 | 4.76 | 3.0 |
| 15a | 4.57 | 25.36 | 322.42 | 2.20 | 3 | 23 | 3 | 0 | 4.76 | 3.5 |
| 16a | 5.15 | 16.13 | 338.49 | 3.50 | 3 | 23 | 2 | 0 | 4.62 | 3.0 |
| 17a | 5.19 | 16.13 | 306.43 | 4.89 | 3 | 22 | 2 | 0 | 4.81 | 3.0 |
| 18a | 5.00 | 16.13 | 338.44 | 4.44 | 3 | 24 | 2 | 0 | 4.68 | 3.0 |
| 19a | 4.79 | 16.13 | 280.39 | 4.75 | 3 | 20 | 2 | 0 | 4.84 | 3.1 |
| 20 | 5.64 | 16.13 | 320.45 | 5.30 | 3 | 23 | 2 | 0 | 4.83 | 3.0 |
| 43 | 6.30 | 16.13 | 364.48 | 5.37 | 3 | 26 | 2 | 0 | 4.71 | 3.0 |
| 44 | 5.86 | 16.13 | 350.46 | 4.96 | 3 | 25 | 2 | 0 | 4.70 | 3.0 |
| 45 | 4.71 | 25.36 | 340.42 | 2.26 | 3 | 24 | 3 | 0 | 4.73 | 3.4 |

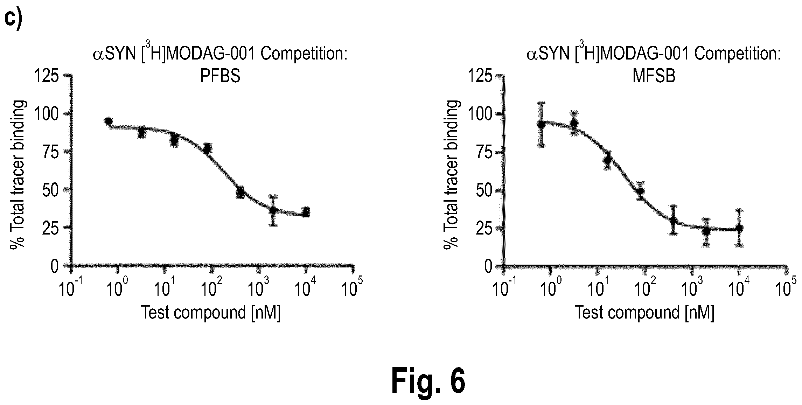

Due to scaffold similarity, all fibril binding assays adopted [³H]PiB as a radiolabeled competitor, with the aim of evaluating the impact of structural modification across the library on the binding to αSYN. To assess their affinity in a less relative manner, PFSB and MFSB were also evaluated in competition with [³H]MODAG-001, the current standard in αSYN preclinical imaging, due to its low K*_{d}* value of 0.6 ± 0.1 nM. Both compounds demonstrated moderate to good competition, with MFSB exhibiting a 4.6-fold lower K*ᵢ* than PFSB (K_{i-PFSB}: 142.1 ± 16.9 nM, K_{*i*-MFSB}: 30.8 ± 10.5 nM, Figure 6c).

### 2.3. Radiolabeling of [¹⁸F]PFSB and [¹⁸F]MFSB

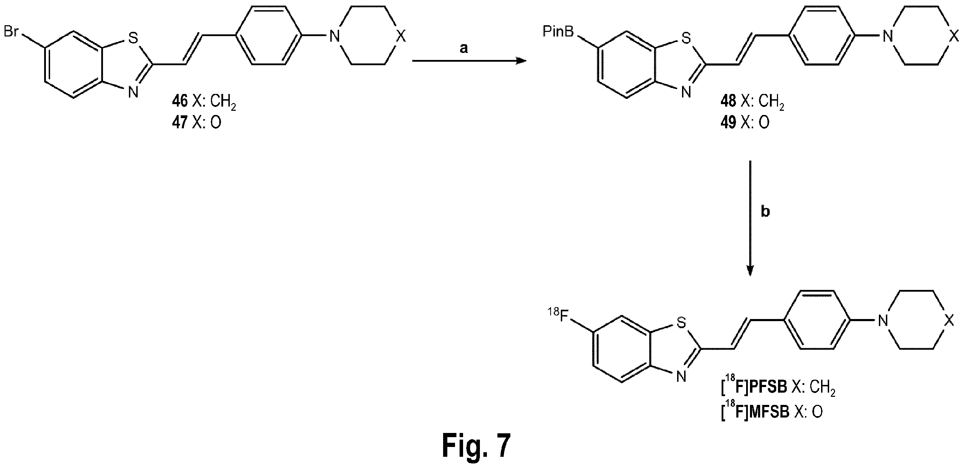

From the first set of compounds, PFSB was selected for radiolabeling and further evaluation. A bromo-substituted analog (46) was synthesized according to the general procedure (Figure 2) and reacted in a Pd(dppf)Cl₂-catalyzed borylation to generate a pinacol boronate precursor (48) for fluorine-18 (¹⁸F) labeling (Figure 7). A copper-mediated radiofluorination (CMRF) was established by optimizing the amount of precursor and pyridine (Table 4). As precursor-load did not seem to affect the reaction efficiency, a lower amount was chosen on behalf of poor solubility. Increased pyridine concentration negatively impacted radiochemical conversion (RCC). According to these results, entry (b) was selected as a starting point for automation. [¹⁸F]PFSB was produced with radiochemical yield (RCY) of 5.8% ± 1.3 and molar activity (Aₘ) of 36.5 ± 8.5 GBq/µmol.

The described procedure was equally applied for precursor synthesis (49) and radiolabeling of the morpholine analog [¹⁸F]MFSB (Figure 7) to afford the tracer with RCY of 11.6% ± 2.9 and Aₘ of 41.2 ± 12.0 GBq/µmol. Analytical results from both tracers are reported in Figures 8 to 11.

**Table 4. Optimization of CMRF of [¹⁸F]PFSB: variation of the amount of precursor and pyridine and correspondent RCC. The amount Cu(OTf)₂, n-BuOH and DMA remained constant in all experiments.**

| # | Precursor 48 (µmol) | Pyridine (µmol) | RCC (%) |
|---|---|---|---|
| a | 20 | 60 | 7.6 |
| b | 10 | 60 | 7.0 |
| c | 20 | 200 | 2.4 |
| d | 10 | 200 | 4.0 |

### 2.4. In vitro autoradiography and in vivo PET/MR imaging

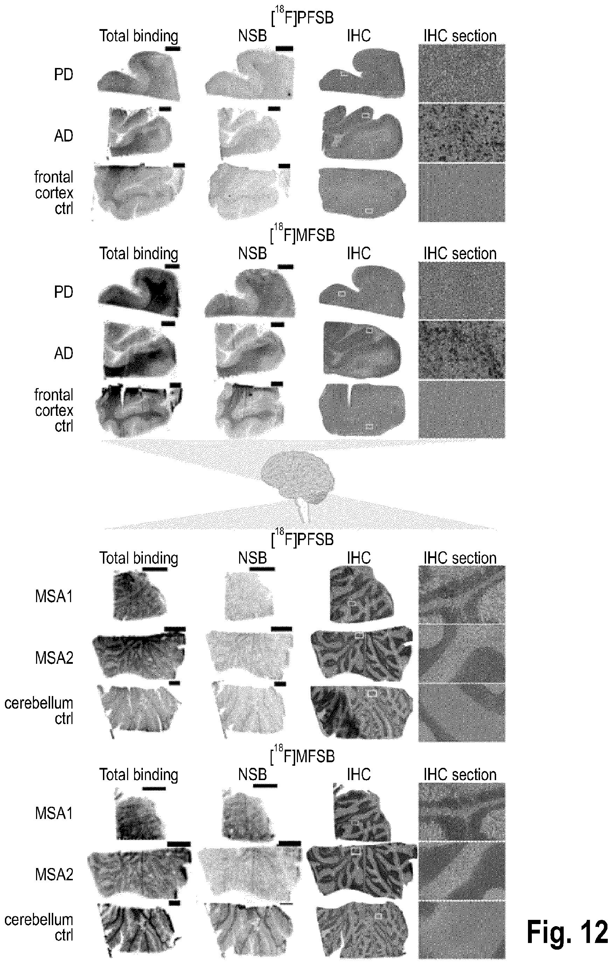

To validate the results from fibril binding assays, in vitro autoradiography of [¹⁸F]PFSB on human brain slices was performed. The experiment corroborated the binding profile previously observed: the tracer displayed affinity to αSYN pathology and selectivity towards αSYN over Aβ (Figure 12). Brain slices from MSA patients exhibited a particularly high specific binding (SB), with a ratio of 3.94 ± 0.30 compared to the control investigating the same brain area (Table 6). Instead, an Alzheimer's disease (AD) sample showed no increased binding in the regions where immunohistochemistry (IHC) detected the presence of Aβ plaques (AD/frontal cortex SB ratio: 1.06) (Figure 12). The autoradiography of [¹⁸F]MFSB validated the selective binding to αSYN of the 2-styrylbenzothiazoles in the library as the AD/frontal cortex SB ratio in the grey matter area remained favorable (Figure 12).

**Table 6. Quantitative analysis of in vitro autoradiography of [¹⁸F]PFSB and [¹⁸F]MFSB. The accurate quantification of the disease/control SB ratio for MSA sections in the experiment with [¹⁸F]MFSB was hampered by high NSB.**

| **SB disease/SB control** | **[¹⁸F]PFSB** | **[¹⁸F]MFSB** |
|---|---|---|
| MSA1/cerebellum ctrl | 3.73 | 1.20 |
| MSA2/cerebellum ctrl | 4.15 | 1.27 |
| PD/frontal cortex ctrl | 1.51 | 1.18 |
| AD/frontal cortex ctrl | 1.03 | 1.03 |

### 2.5. BBB passage and in vivo detection

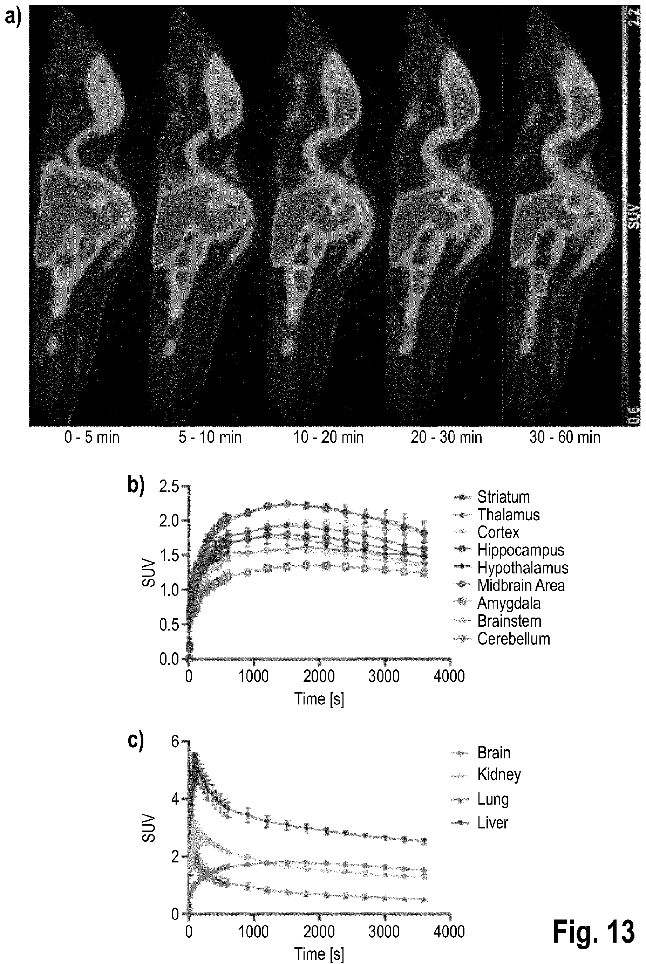

To assess its pharmacokinetic profile, [¹⁸F]MFSB was injected to three healthy wild-type mice and its distribution evaluated over 60 min via dynamic PET/MR. The experiment proved crossing of the BBB was successful, and beginning of clearance was observed (Figure 13). High uptake was detected in lungs and liver in the first 2 min (SUV ≈ 5), followed by fast to moderate clearance. Significant kidney uptake was also observed, showing renal excretion starting in the first few minutes (Figure 13). This shows that [¹⁸F]MFSB can pass the BBB and that in vivo detection of the αSYN binding compound is possible. Thus, α-synuclein binding compounds being 2-styrylbenzothiazole derivatives are promising diagnostic compounds with regard to at least neurogenerative diseases.

### 3. Conclusion

The inventors provide high-affinity and high-selectivity α-synuclein binding compounds suitable for diagnosis and research of synucleinopathies.

## Claims

1. An α-synuclein binding compound having the structure 1, 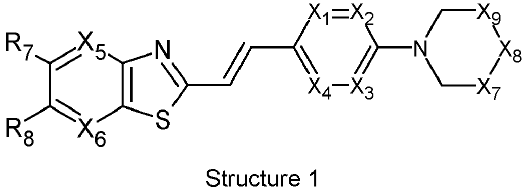
- wherein X₁-X₆ are independently selected from the group consisting of: C-Rₓ, N, wherein the x in each Rₓ is equal to the index of the X to which the Rₓ is bound, with the provisor that within the group consisting of: X₁, X₂ and within the group consisting of: X₃, X₄, no more than one X is N; and
- wherein R₇, R₈ and Rₓ are independently selected from the group consisting of: H, F, Cl, Br, OH, C₁-C₃ alkyl ether-group, fluorinated C₁-C₃ alkyl-group, fluorinated C₁-C₃ alkyl ether-group and are preferably selected from the group consisting of: H, O-CH₃, F; and
- wherein X₇-X₉ are independently selected from the group consisting of: CH₂, O, NH, S, and are preferably selected from the group consisting of: CH₂, O, with the provisor that no heteroatom is directly bound to a heteroatom.

2. The α-synuclein binding compound of claim 1, wherein X₁ to X₆ are C-Rₓ.

3. The α-synuclein binding compound of claim 2, wherein R₁ to R₄ are H.

4. The α-synuclein binding compound of any of claims 2 to 3, wherein R₅, R₆ and R₇ are H.

5. The α-synuclein binding compound of any of claims 2 to 4, wherein R₈ is F.

6. The α-synuclein binding compound of any previous claim, with the provisor that no more than one of X₇ to X₉ is not CH₂.

7. The α-synuclein binding compound of any previous claim, having the structure 2 or the structure 3.

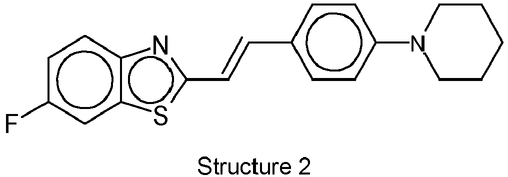

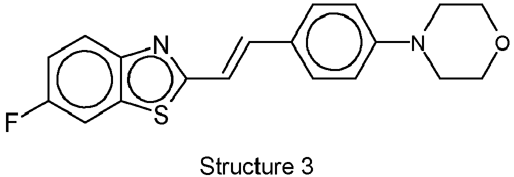

8. The α-synuclein binding compound of any previous claim, wherein the compound is fluorescent.

9. The α-synuclein binding compound of any previous claim, wherein at least one atom of the compound is a radionuclide.

10. The α-synuclein binding compound of claim 9, wherein at least one C-atom is a ¹¹C radionuclide.

11. The α-synuclein binding compound of any of claims 9 or 10, wherein at least one F atom is a ¹⁸F radionuclide.

12. The α-synuclein binding compound of any previous claim for use in diagnosis of a disease in a living being, wherein the disease is preferably a neurodegenerative disease, more preferably a neurodegenerative disease associated with unphysio-logical α-synuclein presence in a tissue of a mammal.

13. The α-synuclein binding compound of any previous claim for use of claim 12, wherein the diagnosis is a diagnosis of a synucleinopathy, wherein the synucleinopathy is preferably selected from the group consisting of: Parkinson's disease, multiple system atrophy, and dementia with Lewy bodies.

14. A diagnostic composition containing the α-synuclein binding compound of any preceding claim and a pharmaceutically acceptable carrier.

15. The diagnostic composition of claim 14, wherein the diagnostic composition is configured for administration to a living being, wherein the living being is preferably a mammal.
